# EUROPEAN PATENT SPECIFICATION

(11) **EP 1 227 859 B1**
(45) Date of publication and mention of the grant of the patent: **09.08.2006**
(21) Application number: 00980231.5
(22) Date of filing: 24.10.2000
(51) Int. Cl.: A61P 27/06, A61K 31/4439, A61K 31/444, A61K 31/00

(54) **NEUROPHILIN LIGANDS FOR TREATING OCULAR CONDITIONS**
VERWENDUNG VON NEUROPHILIN LIGANDEN ZUR BEHANDLUNG VON AUGENERKRANKUNGEN
LIGANDS DE LA NEUROPHILINE POUR TRAITEMENT DE TROUBLES OCULAIRES

(30) Priority: 12.11.1999 US 165137 P
(43) Date of publication of application: 07.08.2002
(73) Proprietor: Alcon, Inc., 6331 Hünenberg (CH)
(72) Inventor: PANG, Iok-hou, Grand Prairie, TX 75052 (US); HELLBERG, Mark, A., Arlington, TX 76017 (US); NAMIL, Abdelmoula, Arlington, TX 76001 (US)
(74) Representative: Wain, Christopher Paul
(86) International application number: PCT/US2000/029320
(87) International publication number: WO 2001/035948

(56) References cited:
- WO-A-00/09108
- WO-A-96/40633
- WO-A-99/42104
- US-A- 5 840 736
- US-A- 5 922 773
- PERRY HENRY D ET AL: "Topical cyclosporine A in the management of postkeratoplasty glaucoma and corticosteroid-induced ocular hypertension (CIOH) and the penetration of topical 0.5 percent cyclosporine A into the cornea and anterior chamber." CLAO JOURNAL, vol. 24, no. 3, July 1998 (1998-07), pages 159-165, XP001009670 ISSN: 0733-8902
- SALAS-PRATO M ET AL: "Inhibition by rapamycin of PDGF- and bFGF-induced human tenon fibroblast proliferation in vitro" JOURNAL OF GLAUCOMA,US,NEW YORK, NY, vol. 5, no. 1, February 1996 (1996-02), pages 54-59, XP002107527 ISSN: 1057-0829
- HAMILTON G S ET AL: "Immunophilins: Beyond immunosuppression." JOURNAL OF MEDICINAL CHEMISTRY, vol. 41, no. 26, 17 December 1998 (1998-12-17), pages 5119-5143, XP001002810 ISSN: 0022-2623
- ROBERT BERKOW: "The Merck Manual of Diagnosis and Therapy - Fifteenth Edition" , MERCK SHARP & DOHME RESEARCH LABORATORIES , RAHWAY N.J. XP002170643 page 2234 -page 2240
- LAM T T ET AL: "Neuroprotective effect of cyclosporine a in a rat model of chronic elevated intraocular pressure." IOVS, vol. 41, no. 4, 15 March 2000 (2000-03-15), page S829 XP001009674 Annual Meeting of the Association in Vision and Opthalmology.;Fort Lauderlade, Florida, USA; April 30-May 05, 2000
- MATSUBARA M ET AL: "Tacrolimus reduces secondary degeneration of the retinal ganglion cell in an ocular hypertensive model and an optic nerve crush model in the rat." IOVS, vol. 41, no. 4, 15 March 2000 (2000-03-15), page S829 XP001009675 Annual Meeting of the Association in Vision and Opthalmology.;Fort Lauderlade, Florida, USA; April 30-May 05, 2000

## Description

The present invention is directed to the use of neurophilin ligands for preventing or reducing the rate of visual field loss and treating the ocular hypertension associated with glaucoma.

### Background of the Invention

The glaucomas are a heterogeneous group of optic neuropathies characterized by the cupping of the optic nerve head, thinning of the retinal nerve fiber layer, and specific changes in visual fields. Elevated intraocular pressure (IOP) is a very important risk factor for the development of the most common forms of glaucoma (Sommer A., et al., "Relationship between intraocular pressure and primary open angle glaucoma among white and black Americans," Arch. Ophthalmol., 109:1090-1095 (1991)). Elevated IOP is believed to be caused by the increased deposition of extracellular matrix material by the trabecular meshwork cells which line the outflow pathway in the trabecular meshwork or by a decrease in the synthesis, release, and activation of matrix metalloproteinases by the trabecular meshwork cells or both. The result is that the trabecular meshwork becomes clogged and unable to perform one of its most critical functions, serving as a gateway for aqueous humor flow from the anterior chamber of the eye to the Schlemm's canal. When the aqueous humor flow out of the eye via the trabecular meshwork is diminished, IOP rises.

Due to the association between elevated IOP and glaucomatous visual field loss, glaucoma has been traditionally treated by lowering IOP medically (Sugrue, M.F, "New approaches to antiglaucoma therapy", *J Med. Chem.,* 40;2793-2809(1997)), and/or by laser trabeculectomy, and/or surgically (Quigley, H.A. "Open-angle glaucoma", *New England J Med.* 328:1097-1106(1993).

Laser trabeculectomy is often effectively used to decrease elevated IOP, however, the effects of laser trabeculectomy are seldom permanent. Laser treatment of the trabecular meshwork results in a dramatic increase in cell division in a population of cells believed to serve as trabecular meshwork stem cells. This leads to a repopulation of the trabecular meshwork (Acott, T.S, et al. "Trabecular repopulation by anterior trabecular meshwork cells after laser trabeculectomy", *Am. J of Ophthalmol,* 107:1-3 (1989)). Laser treatment also induces an increase in the expression of matrix metalloproteinases in the trabecular meshwork (Parshley D.E. et al., "Laser trabeculectomy induces stromelysin expression by trabecular juxtacanalicular cells" *Invest. Ophthamol. Vis.* Sci.37:795-804 (1996), Bradley, J.D. et al., "Effects of matrix metalloproteinase activity on outflow in perfused human organ culture", *Invest. Ophthalmol. Vis. Sci.,* 39:2649-2658 (1998)). This increase is believed to increase the rate of degradation of extracellular matrix resulting in a decrease in outflow resistance.

Elevated IOP does not always result in the occurrence of visual field loss, and visual field loss may occur at levels of IOP which are considered within the normal range. Thus, factors other than IOP may play a role in determining the occurrence of visual field loss. Degeneration of the retinal ganglion cells may be related to ischemia or a cascade of events that may have been initiated by the effects of IOP on the optic nerve that once initiated proceeds even if IOP is normalized. Various methods have been directed at treating retinal ganglion cell degeneration including the use of: polyamine antagonists (Kapin, M.A. USP 5,710,165:1998), noncompetitive inhibitors of the NMDA receptor-channel complex (Lipton, S.A., USP 5,922,773:1999), sodium channel blockers (Adorante, J.S., WO98/43612), 2-imidolin-2-yl(amino)quinoxalines (Wheeler, L.A. et al., USP 5,856,329), and EP₂ receptor agonists (Woodward, D.F. USP 5,877,211).

Immunophilins are a series of chaperone proteins which mediate the activity of immunosuppressant drugs such as FK506, rapamycin, and cyclosporin A (Pratt W.B., et al, "Steroid receptor interactions with heat shock proteins and immunophilin chaperones," *Endoc Rev,* 18:306-26(1997)). Immunophilins are enriched in neurons throughout the central and peripheral nervous system, indicating that the immunophilins may play a role in neural function. The finding that FK-506 dose-dependently accelerates functional recovery from nerve injury initiated a search to determine the mechanism of this function. The finding that non-immunosuppressant analogs of FK-506 can also facilitate neurogeneration suggested that ligands of non-immunosuppressant immunophilins (referred to herein as neurophilin ligands) may have therapeutic utility in a variety of neurodegenerative disorders such as Parkinson's disease, Alzheimer's disease, diabetic and peripheral neuropathies, and spinal cord injury (Hamilton, G.S.; Steiner, **J.P.,** "Immunophilins: Beyond Immunosuppression," J. *Med. Chem.,* 41:5119-5143).

The neurotrophic properties of immunophilin ligands (such as FK-506) were believed to depend on their interaction with the 12-kDa FK506 binding protein (FKBP-12). More recent studies have suggested that the protective effects of these compounds may be mediated by interaction with the immunophilin FKBP-52 (also know as FKBP-59 or heat shock protein 56) and possibly other related immunophilins (Gold G.G. et al., "Immunophilin FK506-binding protein 52 (not FK506-binding protein 12) mediates the neurotrophic action of FK506", *J. Pharmacol. Exp. Ther.,* 289:1202-1210). The use of pipecolic acid derivatives having affinity for FKBP-type immunophilins to stimulate or promote growth or regeneration including neurological disorders of the eye has been disclosed (Steiner, J.P. et al. WO 96/40140). The use of N-glyoxyl-prolyl ester compounds having an affinity for FKBP-type immunophilin for treatment of neurological disorders of the eye has also been disclosed (Hamilton, G.S., et al., WO 96/40633).

### Summary of the Invention

The present invention is directed to the use of neurophilin ligands for the manufacture of a medicament to treat glaucoma, lower and control IOP, and prevent the visual field loss associated with glaucoma in mammals.

According to the present invention there is provided the use of a neurophilin ligand which does not exhibit immunosuppressant activity for the manufacture of a medicament for the treatment of a mammal suffering from glaucoma; or for lowering IP in a mammal suffering from elevated IPO; or for preventing visual field loss associated with glaucoma; or for lowering IOP and providing neuroprotection in a mammal suffering from elevated IOP; wherein the neurophilin ligand is as claimed in claim 1.

### Detailed Description of Preferred Embodiments

The neurophilin ligands are a class of compounds that effectively treat glaucoma by exerting a protective effect on the retinal ganglion cells and the cells of the optic nerve head and by decreasing IOP by rejuvenating the trabecular meshwork cells. Although the exact mechanism or mechanisms which underlie the protective effect of the neurophilin ligands is not completely understood, these compounds are effective in inhibiting neuronal degeneration and in promoting neuro-regeneration in animal neurotoxicity models.

In addition to reducing the rate of retinal ganglion cell loss and thereby slowing the progressive visual field loss associated with glaucoma, the neurophilin ligands are believed to reduce elevated IOP by stimulating trabecular meshwork cell function. The neurophilin ligands have been shown to stimulate neurite outgrowth in PC12 cells, which, like the trabecular meshwork cells, are derived from the neural crest stem cells. It is believed that neurophilin ligands will stimulate both the proliferation and the activation of the trabecular meshwork cells resulting in the repopulation of the trabecular meshwork and an increase in matrix metalloproteinase production or activation which results in the degradation of the extracellular debris occluding the outflow pathway.

According to both aspects, the invention preferably will be used to treat patients which have primary open angle glaucoma, chronic closed angle glaucoma, pseudoexfoliation glaucoma, normal tension glaucoma, or other sub-types of glaucoma or ocular hypertension. Administration of the drug is achieved through routes including, but not limited to, topical ocular, periocular injection, intravitreal injection, or intravitreal implant at a dose ranging from about 0.001 to about 2 mg/eye/day; systemic, including oral, transdermal, intravenous, transnasal, buccal, or subcutaneous at concentrations of about 0.01 to about 10 mg/kg/day; or using an ocular implant, such as, an intravitreal implant comprising about 0.2 to 100 mg.

The neurophilin ligands of the present invention can be used alone (including a combination of more than one neurophilin ligand) and in combination with other agents for treating glaucoma, such as, IOP, lowering drugs (ex., prostaglandins, beta blockers, carbonic anhydrase inhibitors, muscarinics, sympathomimetics, alpha agonists, and serotonergics) and/or neuroprotectants (ex., calcium or sodium channel blockers, glutamate antagonists, including NMDA antagonists, anti-apoptotic agents, adenosine reuptake inhibitors, nitric oxide synthase inhibitors, vasodilators, neurotrophic factor enhancers, neurotrophic factors (such as ciliary neurotrophic factor (CNTF), and basic fibroblast growth factor (bFGF, etc.)).

The preferred neurophilin ligands are those that have neurotrophic activity, but have little or no immunosuppressant activity. For example, one skilled in the art is referred to the following patents and patent applications for their teaching of neurotrophic compounds which are lacking immunosuppressive activity: WO 99/14998 (Amgen, Method for Preventing and Treating hearing Loss using Sensorineurotrophic Compounds) and the references disclosed therein, including, a series of pipecolic acid derivatives that act as neurophilin ligands (US 5,696,135); a series of proline derivatives that act as neurophilin ligands (US 5,614,547); a series of N-sulfonyl pipecolyl and prolyl derivatives that act as neurophilin ligands (US 5,721,256); a series of heterocyclic thiesters and ketones that act as neurophilin ligands (US 5,786,378); a series of N-glyoxyl-prolyl ester that act as neurophilin ligands (US 5,795,908); a series of pipecolic acid derivatives that act as neurophilin ligands (US 5,798,355); and a series of heterocyclic ester and amide derivatives that act as neurophilin ligands (US 5,801,187).

Further compounds which can be used according to the present invention are disclosed in US 5,840,736 (the use of a neurophilin ligand in the presence of a neurotrophic factor for stimulating neurite outgrowth); US 5,654,332 (the use of a neurophilin ligand in the presence of a nerve growth factor for stimulating neurite outgrowth); US 5,811,434 (the use of a neurophilin ligand in the presence of a nerve growth factor for stimulating neurite outgrowth); US 5,780,484 (the use of a piperidine derivative as a neurophilin ligand in the presence of a nerve growth factor for stimulating neurite outgrowth); US 5846979 (the use of N-oxide of heterocyclic esters, amides, thiesters, and ketones as neurophilin ligands).

Christner, C. et al. disclose a series of cycloheximide derivatives as neurophilin ligands with neuroregenerative properties ("Synthesis and Cytotoxic Evaluation of Cycloheximide Derivatives as Poterntial Inhibitors of FKBP12 with Neuroregenerative Properties", *J. Medicinal Chem.* 42:3615-22, 1999) which can be used according to the methods of this invention.

One class of preferred compounds is disclosed in the above mentioned patent US 5,840,736. Especially preferred is (S)-N-benzyl-3-(4-chlorophenyl)-2-(methyl-(2-oxo-2(3,4,5-trimethoxyphenyl)acetyl)amino)-N-(3-(pyridinyl-4-yl-) 1-(2-(pyrodinyl-4-yl)-ethyl)propyl)propionamide (Timcodar).

Another class of preferred compounds is disclosed in the above mentioned patent US 5,614,547. Especially preferred is 1-(3,3-dimethyl-1,2-dioxopentyl)-(L)-proline 3-(3-pyridinyl)propyl ester (GPI-1046).

## Claims

1. The use of a neurophilin ligand which does not exhibit immunosuppressant activity, for the manufacture of a medicament for treatment of a mammal suffering from glaucoma, wherein the neurophilin ligand is
(a) a pipecolic acid derivative which is (E)-3-(3,4-dichlorophenyl)-2-propenyl 1-(3,3-dimethyl-2-oxopentanoyl)-2-piperidinecarboxylate; (E)-3-(3,4,5-trimethoxyphenyl)-2-propenyl 1-(3,3-dimethyl-2-oxopentanoyl)-2-piperidinecarboxylate; (E)-3-phenyl-2-propenyl 1-(3,3-dimethyl-2-oxopentanoyl)-2-piperidinecarboxylate: (E)-3-((3-(2,5-dimethoxy)-phenylpropyl)phenyl)-2-propenyl 1-(3,3-dimethyl-2-oxopentanoyl)-2-piperidinecarboxylate; (E)-3-(1,3-benzodioxol-5-yl)-2-propenyl 1-(3,3-dimethyl-2-oxopentanoyl)-2-piperidinecarboxylate; 4-(4-methoxyphenyl)butyl 1-(2-oxo-2-phenylacetyl)-2-piperidinecarboxylate; 3-phenylpropyl 1-(2-oxo-2-phenylacetyl)-2-piperidinecarboxylate; 3-(3-pyridyl)propyl 1-(2-oxo-2-phenylacetyl)-2-piperidinecarboxylate; 3-(3-pyridyl)propyl 1-(3,3-dimethyl-2-oxopentanoyl)-2-piperidinecarboxylate; 4-phenyl-1-(3-phenylpropyl)butyl 1-(3,3-dimethyl-2-oxopentanoyl)-2-piperidinecarboxylate; 4-(4-methoxyphenyl)butyl1-(3,3-dimethyl-2-oxopentanoyl)-2-piperidinecarboxylate; 1- (4-methoxyphenethyl)-4-phenylbutyl 1-(3,3-dimethyl-2-oxopentanoyl)-2-piperidinecarboxylate; 3-(2,5-dimethoxyphenyl)propyl 1-(3,3-dimethyl-2-oxopentanoyl)-2-piperidinecarboxylate; 3-(1,3-benzodioxol-5-yl)propyl 1-(3,3-dimethyl-2-oxopentanoyl)-2-piperidinecarboxylate; 1-phenethyl-3-phenylpropyl 1-(3,3-dimethyl-2-oxopentanoyl)-2-piperidinecarboxylate; 4-(4-methoxyphenyl)butyl 1-(2-cyclohexyl-2-oxoacetyl)-2-piperidinecarboxylate; 3-cyclohexylpropyl 1-(2-cyclohexyl-2-oxoacetyl1-2-piperidinecarboxylate; 3-phenylpropyl 1-(2-cyclohexyl-2-oxoacetyl)-2-piperidinecarboxylate; 3-cyclohexylpropyl 1-(3,3-dimethyl-2-oxobutanoyl)-2-piperidinecarboxylate; 3-phenylpropyl 1-(3,3-dimethyl-2-oxobutanoyl)-2-piperidinecarboxylate; 4-(4-methoxyphenyl)butyl 1-(3,3-dimethyl-2-oxobutanoyl)-2-piperidinecarboxylate; or 4-phenyl-1-(3-phenylpropyl)butyl1-(3,3-dimethyl-2-oxobutanoyl)-2-piperidinecarboxylate,
(b) a proline derivative which is
(i) a compound of the formula: 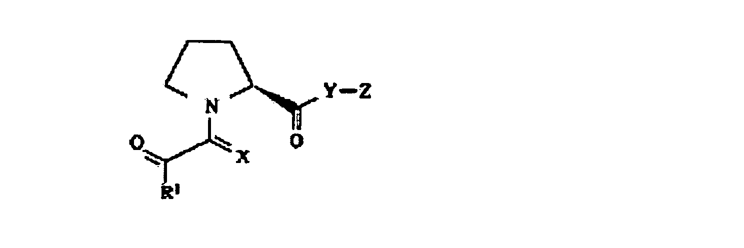 where R₁ is a C₁ -C₉ straight or branched chain alkyl or alkenyl group optionally substituted with C₃ -C₈ cycloalkyl, C₃ or C₅ cycloalkyl, C₅ -C₇ cycloalkenyl, or Ar₁, where said alkyl, alkenyl, cycloalkyl or cycloalkenyl groups may be optionally substituted with C₁ -C₄ alkyl, C₁ -C₄ alkenyl, or hydroxy, and where Ar₁ is selected from the group consisting of 1-napthyt, 2-napthyl, 2-indolyl, 3indolyl, 2-furyl, 3-furyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁ -C₆ straight or branched alkyl or alkenyl, C₁ -C₄ alkoxy or C₁ -C₄ alkenyloxy, phenoxy, benzyloxy, and amino;
X is selected from the group consisting of oxygen and sulfur;
Y is oxygen or NR₂, where R₂ is hydrogen or C₁-C₆ alkyl; and
Z is a C₂-C₆ straight or branched chain alkyl or alkenyl, wherein the alkyl chain is substituted in one or more positions with Ar₁ as defined above, C₃-C₈ cycloalkyl, cycloalkyl connected by a C₁-C₆ straight or unbranched alkyl or alkenyl chain, or Ar₂
where Ar₂ is selected from the group consisting of 2-indolyl, 3-indolyl, 2-furyl, 3-furyl, 2-thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁ -C₆ straight or branched alkyl or alkenyl, C₁ -C₄ alkoxy or C₁ -C₄ alkenyloxy, phenoxy, benzyloxy, and amino;
Z may also be the fragment: 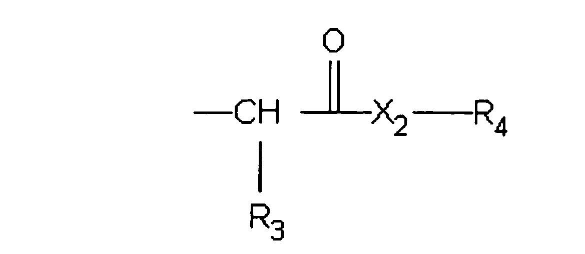 where R₃ is selected from the group consisting of straight or branched alkyl C₁ -C₈ optionally substituted with C₃ -C₈ cycloalkyl, or Ar₁ as defined above;
X₂ is O or NR₅, where R₅ is selected from the group consisting of hydrogen, C₁ -C₆ straight or branched alkyl and alkenyl;
R₄ is selected from the group consisting of phenyl, benzyl, C₁-C₅ straight or branched alkyl or alkenyl, and C₁-C₅ straight or branched alkyl or alkenyl substituted with phenyl; or pharmaceutically acceptable salts or hydrates thereof; including the compounds 3-phenyl-1-propyl (2S)-1-(3,3-dimethyl-1,2-dioxopentyl)-2-pyrrolidinecarboxylate,
3-phenyl-1-prop-2-(E)-enyl (2S)-1-(3,3-dimethyl-1,2-dioxopentyl)-2-pyrrolidinecarboxylate,
3-(3,4,5-trimethoxyphenyl)-1-propyl (2S)-1-(3,3-dimethyl-1,2-dioxopentyl)-2-pyrrolidinecarboxylate,
3-(3,4,5-trimethoxyphenyl)-1-prop-2-(E)-enyl (2S)-1-(3,3-dimethyl-1,2-dioxopentyl)-2-pyrrolidinecarboxylate,
3-(4,5-dichlorophenyl)-1-propyl (2S)-1-(3,3-dimethyl-1,2-dioxopentyl)-2-pyrrolidinecarboxylate,
3-(4,5-dichlorophenyl)-1-prop-2-(E)-enyl (2S)-1-(3,3-dimethyl-1,2-dioxopentyl)-2-pyrrolidinecarboxylate,
3-(4,5-methylenedioxyphenyl)-1-propyl (2S)-1-(3,3-dimethyl-1,2-dioxopentyl)-2-pyrrolidinecarboxylate,
3-(4,5-methylenedioxyphenyl)-1-prop-2-(E)-enyl (2S)-1-(3,3-dimethyl-1,2-dioxopentyl)-2-pyrrolidinecarboxylate,
3-cyclohexyl-1-propyl (2S) -1-(3,3-dimethyl-1,2-dioxopentyl)-2-pyrrolidinecarboxylate,
3-cyclohexyl-1-prop-2-(E)-enyl (2S)-1-(3,3-dimethyl-1,2-dioxopentyl)-2-pyrrolidinecarboxylate,
(1R)-1,3-diphenyl-1-propyl (2S)-1-(3,3-dimethyl-1,2-dioxopentyl)-2-pyrrolidinecarboxylate,
(1R)-1,3-diphenyl-1-prop-2-(E)-enyl (2S)-1-(3,3-dimethyl-1,2-dioxopentyl)-2-pyrrolidinecarboxylate,
(1R)-1-cyclohexyl-3-phenyl-1-propyl (2S)-1-(3,3-dimethyl-1,2-dioxopentyl)-2-pyrrolidinecarboxylate,
(1R)-1-cyclohexyl-3-phenyl-1-prop-2-(E)-enyl (2S)-1-(3,3-dimethyl-1,2-dioxopentyl)-2-pyrrolidinecarboxylate,
(1R)-1-(4,5-dichlorophenyl)-3-phenyl-1-propyl (2S)-1-(3,3-dimethyl-1,2-dioxopentyl) -2-pyrrolidinecarboxylate,
3-phenyl-1-propyl (2S)-1-(1,2-dioxo-2-cyclohexyl)ethyl-2-pyrrolidinecarboxylate,
3-phenyl-1-propyl (2S)-1-(1,2-dioxo-4-cyclohexyl)butyl-2-pyrrolidinecarboxylate,
3-phenyl-1-propyl (2S)-1-(1,2-dioxo-2-[2-furanyl])ethyl-2-pyrrolidinecarboxylate,
3-phenyl-1-propyl (2S)-1-(1,2-dioxo-2-[2-thienyl])ethyl-2-pyrrolidinecarboxylate,
3-phenyl-1-propyl (2S)-1-(1,2-dioxo-2-[2-thiazolyl])ethyl-2-pyrrolidinecarboxylate
3-phenyl-1-propyl (2S)-1-(1,2-dioxo-2-phenyl)ethyl -2-pyrrolidinecarboxylate,
1,7-diphenyl-4-heptyl (2S)-1-(3,3-dimethyl-1,2-dioxopentyl)-2-pyrrolidinecarboxylate, 3-Phenyl-1-propyl (2S)-1-(3,3-dimethyl-1,2-dioxo-4-hydroxybutyl)-2-pyrrolidinecarboxylate,
3-phenyl-1-propyl (2S)-1-(3,3-dimethyl-1,2-dioxopentyl)-2-pyrrolidinecarboxamide,
[1-(3,3-dimethyl-1,2-dioxopentyl)-L-proline]-L-phenylalanine ethyl ester,
1-[1-(3,3-dimethyl-1,2-dioxopentyl)-L-proline]-L-leucine ethyl ester,
1-[1-(3,3 -dimethyl-1,2-dioxopentyl)-L-proline]-L-phenylglycine ethyl ester,
1-[1-(3,3-dimethyl-1,2-dioxopentyl)-L-proline]-L-phenylalanine phenyl ester,
1-[1-(3,3-dimethy)-1,2-dioxopentyl)-L-proiine]-L-phenylalanine benzyl ester, and
1-[1-(3,3-dimethy)-1,2-dioxopentyl)-L-proline]-L-isoleucine ethyl ester; or
(ii) a compound of the formula: 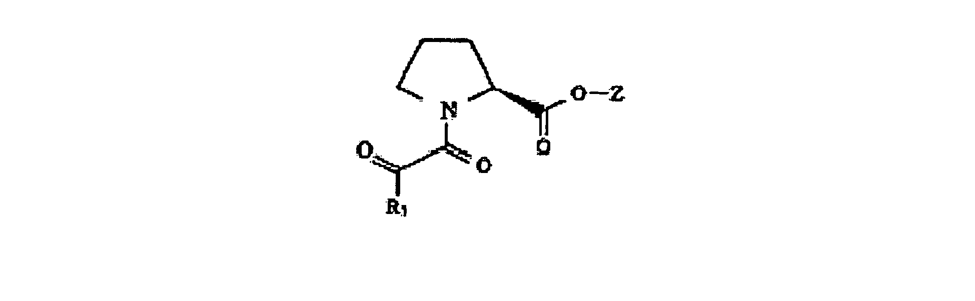 where R₁ is a C₁ -C₉ straight or branched chain alkyl or alkenyl group-optionally substituted with C₃ -C₈ cycloalkyl, C₃ or C₅ cycloalkyl, C₅ -C₇ cycloalkenyl, or Ar₁, where said alkyl, alkenyl, cycloalkyl or cycloalkenyl groups may be optionally substituted with C₁-C₄ alkyl, C₁-C₄ alkenyl, or hydroxy, and where Ar₁ is selected from the group consisting of 1-napthyl, 2-napthyl, 2-indolyl, 3-indolyl, 2-furyl, 3-furyl, 2-thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁-C₆ straight or branched alkyl or alkenyl, C₁-C₄ alkoxy or C₁-C₄ alkenyloxy, phenoxy, benzyloxy, and amino; is a C₂-C₆ straight or branched chain alkyl or alkenyl, wherein the alkyl chain is substituted in one or more positions with Ar₁ as defined above, C₁-C₈ cycloalkyl, cycloalkyl connected by a C₁-C₆ straight or unbranched alkyl or alkenyl chain, or Ar₂ where Ar₂ is selected from the group consisting of 2-indolyl, 3-indolyl, 2-furyl, 3-furyl, 2-thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁-C₆ straight or branched alkyl or alkenyl, C₁-C₄ alkoxy or C₁-C₄ alkenyloxy, phenoxy, benzyloxy, and amino; or pharmaceutically acceptable salts or hydrates thereof; or
(iii) a compound of the formula: 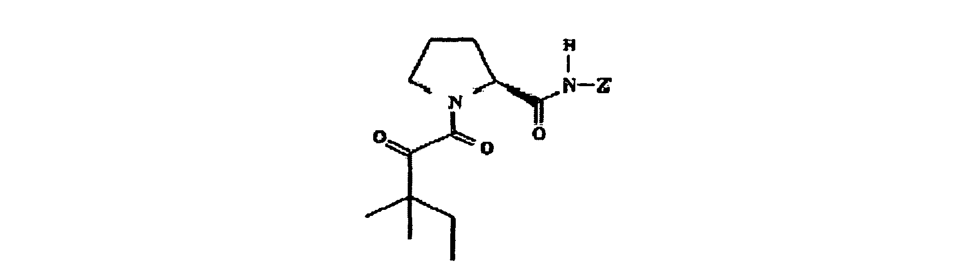 where Z' is the fragment: 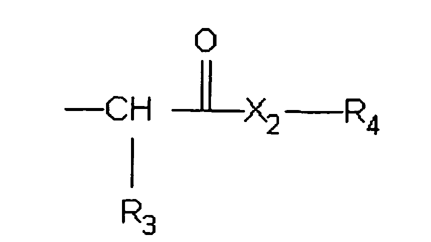 where R₃ is selected from the group consisting of straight or branched alkyl C₁ -C₈ optionally substituted with C₃ -C₈ cycloalkyl, or Ar₁ as defined above, and unsubstituted Ar₁ :
X₂ is O or NR₅, where R₅ is selected from the group consisting of hydrogen, C₁ -C₆ straight or branched alkyl and alkenyl;
R₄ is selected from the group consisting of phenyl, benzyl, C₁ -C₅ straight or branched alkyl or alkenyl, and C₁-C₅ straight or branched alkyl or alkenyl substituted with phenyl; or pharmaceutically acceptable salts or hydrates thereof,
(c) a N-sulfonyl pipecolyl or prolyl derivative which is 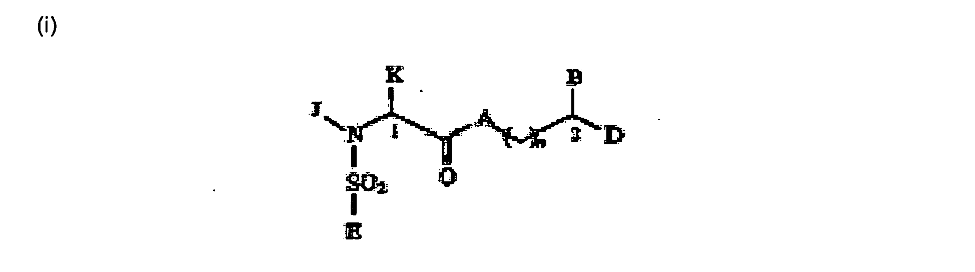 or a pharmaceutically acceptable salt thereof, wherein: A is CH₂, oxygen, NH or N-(C1-C4 alkyl);
B and D are independently Ar, hydrogen, (C1-C6)-straight or branched alkyl, (C1-C6)-straight or branched alkenyl, (C1-C6)-straight or branched alkyl or alkenyl that is substituted with a (C5-C7)-cycloalkyl, (C1-C6)-straight or branched alkyl or alkenyl that is substituted with a (C5-C7)-cycloalkenyl, or Ar substituted (C1-C6)-straight or branched alkyl or alkenyl, wherein, in each case, one or two of the CH₂ groups of the alkyl or alkenyl chains may contain 1-2 heteroatoms selected from the group consisting of oxygen, sulfur, SO and SO₂ in chemically reasonable substitution patterns, or 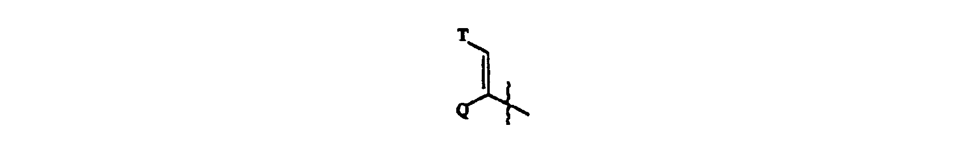 provided that both B and D are not hydrogen; Q is hydrogen, (C1-C6)-straight or branched alkyl or (C1-C6)-straight or branched alkenyl;
T is Ar or substituted 5-7 membered cycloalkyl with substituents at positions 3 and 4 which are independently selected from the group consisting of hydrogen, hydroxyl, O-(C1-C4ralkyl, O--(C1-C4)-alkenyl and carbonyl;
Ar is selected from the group consisting of phenyl, 1-napthyl, 2-naphthyl, 2-furyl, 3-furyl, 2-thienyl, 3-thienyl, 2-pyridyl, 3-pyridyl, 4-pyridyl, monocyclic and bicyclic heterocyclic ring systems with individual ring sizes being 5 or 6 which may contain in either or both rings a total of 1-4 heteroatoms independently selected from O, N and S; wherein Ar may contain one to three substituents which are independently selected from the group consisting of hydrogen, halo, hydroxyl, nitro, trifluoromethyl, trifluoromethoxy, (C1-C6)-straight or branched alkyl, (C2-C6)-straight or branched alkenyl, O-(C1-C4)-straight or branched alkyl, O--(C2-C4)-straight or branched alkenyl, O-benzyl, O-phenyl, 1,2-methylenedioxy, amino, carboxyl and phenyl;
E is (C1-C6)-straight or branched alkyl, (C1-C6)-straight or branched alkenyl, (C5-C7)-cycloalkyl, (C5-C7)-cycloalkenyl substituted with (C1-C4)-straight or branched alkyl or (C₁-C₄)-straight or branched alkenyl, [(C2-C4)-alkyl or (C2-C4)-alkenyl)]-Ar or Ar;
J is hydrogen or C1 or C2 alkyl or benzyl; K is (C1-C4)-straight or branched alkyl, benzyl or cyclohexylmethyl; or wherein J and K may be taken together to form a 5-7 membered heterocyclic ring which may contain an oxygen, sulfur, SO or SO₂ substituent therein;
n is 0 to 3; and
the stereochemistry at carbon positions 1 and 2 are R or S; or 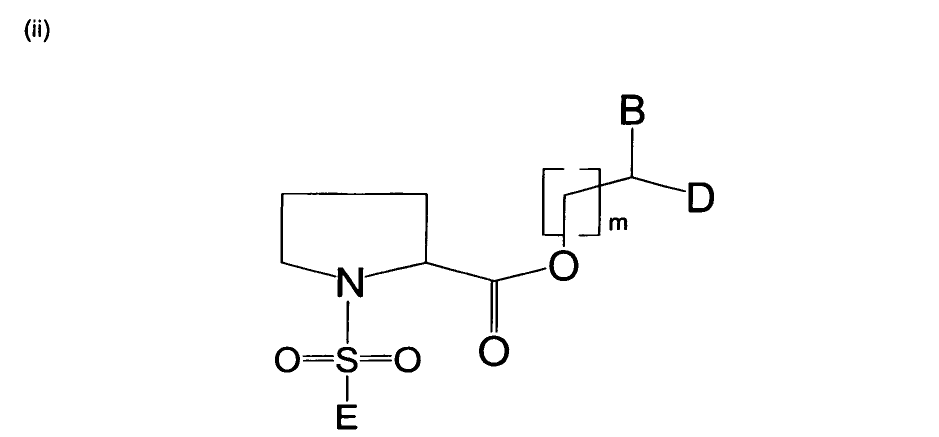 or a pharmaceutically acceptable salt thereof, wherein: B and D are independently Ar, hydrogen, (C1-C6)-straight or branched alkyl, (C1-C6)-straight or branched alkenyl, (C1-C6)-straight or branched alkyl or alkenyl that is substituted with a (C5-C7)-cycloalkyl, (C1-C6)-straight or branched alkyl or alkenyl that is substituted with a (C5-C7)-cycloalkenyl, or Ar substituted (C1-C6)-straight or branched alkyl or alkenyl, wherein, in each case, one or two of the CH₂ groups of the alkyl or alkenyl chains may contain 1-2 heteroatoms selected from the group consisting of oxygen, sulfur, SO and SO₂ in chemically reasonable substitution patterns, or 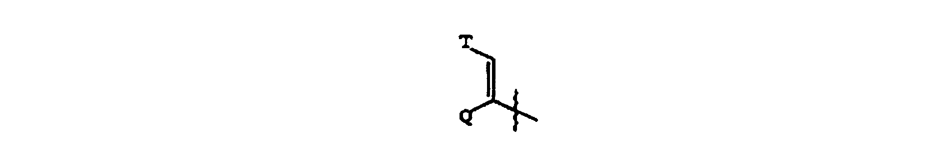 provided that both B and D are not hydrogen;
Q is hydrogen, (C1-C6)-straight or branched alkyl or (C1-C6)-straight or branched alkenyl;
T is Ar or substituted 5-7 membered cycloalkyl with substituents at positions 3 and 4 which are independently selected from the group consisting of hydrogen, hydroxyl, O-(C1-C4)-alkyl, O-(C1-C4)-alkenyl and carbonyl;
Ar is selected from the group consisting of phenyl, 1-napthyl, 2-naphthyl, 2-furyl, 3-furyl, 2-thienyl, 3-thienyl, 2-pyridyl, 3-pyridyl, 4-pyridyl, monocyclic and bicyclic heterocyclic ring systems with individual ring sizes being 5 or 6 which may contain in either or both rings a total of 1-4 heteroatoms independently selected from O, N and S; wherein Ar may contain one to three substituents which are independently selected from the group consisting of hydrogen, halo, hydroxyl, nitro, trifluoromethyl, trifluoromethoxy, (C1-C6)-straight or branched alkyl, (C2-C6)-straight or branched alkenyl, O--(C1-C4)-straight or branched alkyl, O-(C2-C4)-straight or branched alkenyl, O-benzyl, O-phenyl, 1,2-methylenedioxy, amino, carboxyl and phenyl;
E is (C1-C6)-straight or branched alkyl, (C1-C6)-straight or branched alkenyl, (C5-C7)-cycloalkyl, (C5-C7)-cycloalkenyl substituted with (C1-C4)-straight or branched alkyl or (C1-C4)-straight or branched alkenyl, [(C2-C4)-alkyl or (C2-C4)-alkenyl)]-Ar or Ar; and m is 0 to 3; or 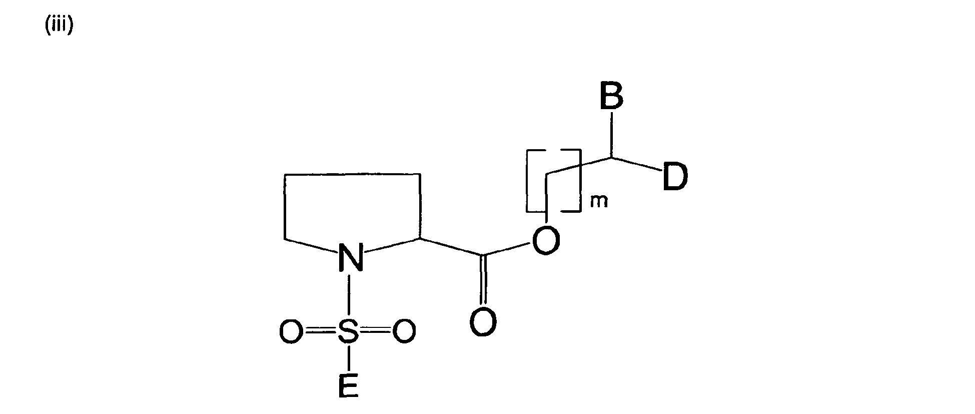 or a pharmaceutically acceptable salt thereof, wherein: B and D are independently Ar, hydrogen, (C1-C6)-straight or branched alkyl, (C1-C6)-straight or branched alkenyl, (C1-C6)-straight or branched alkyl or alkenyl that is substituted with a (C5-C7)-cycloalkyl, (C1-C6)-straight or branched alkyl or alkenyl that is substituted with a (C5-C7)-cycloalkenyl, or Ar substituted (C1-C6)-straight or branched alkyl or alkenyl, wherein, in each case, one or two of the CH₂ groups of the alkyl or alkenyl chains may contain 1-2 heteroatoms selected from the group consisting of oxygen, sulfur, SO and SO₂ in chemically reasonable substitution patterns, or 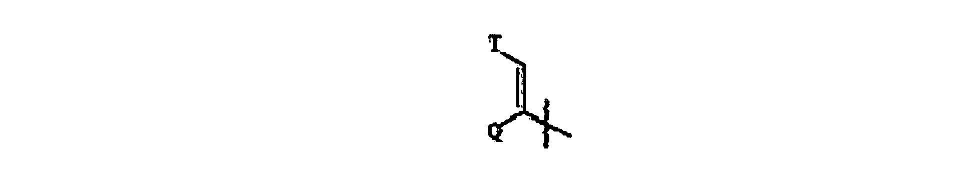 provided that both B and D are not hydrogen; Q is hydrogen, (C1-C6)-straight or branched alkyl or (C1-C6)-straight or branched alkenyl;
T is Ar or substituted 5-7 membered cycloalkyl with substituents at positions 3 and 4 which are independently selected from the group consisting of hydrogen, hydroxyl, O-(C1-C4)-alkyl, O--(C1-C4)-alkenyl and carbonyl;
Ar is selected from the group consisting of phenyl, 1-napthyl, 2-naphthyl, 2-furyl, 3-furyl, 2-thienyl, 3-thienyl, 2-pyridyl, 3-pyridyl, 4-pyridyl, monocyclic and bicyclic heterocyclic ring systems with individual ring sizes being 5 or 6 which may contain in either or both rings a total of 1-4 heteroatoms independently selected from O, N and S; wherein Ar may contain one to three substituents which are independently selected from the group consisting of hydrogen, halo, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl and phenyl;
E is (C1-C6)-straight or branched alkyl, (C1-C6)-straight or branched alkenyl, (C5-C7)-cycloalkyl, (C5-C7)-cycloalkenyl substituted with (C1-C4)-straight or branched alkyl or (C1-C4)-straight or branched alkenyl, [(C2-C4)-alkyl or (C2-C4)-alkenyl)]-Ar or Ar; and m is 0 to 3,
(d) a heterocyclic thioester or ketone which is 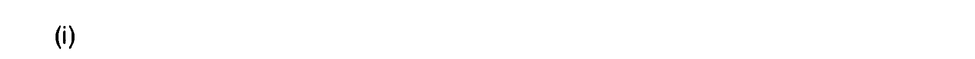 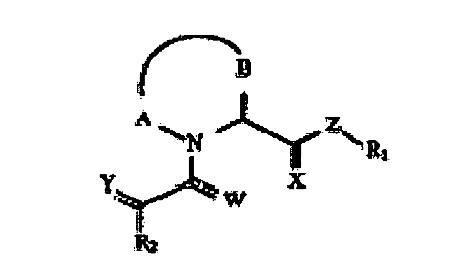 or a pharmaceutically acceptable salt thereof, wherein: A and B, together with the nitrogen and carbon atoms to which they are respectfully attached, form a 5-7 membered saturated or unsaturated heterocyclic ring containing any combination of CH₂, O, S, SO, SO₂, NH or NR₂ in any chemically stable oxidation state;
X is either O or S;
Z is either S, CH₂, CHR₁, or C(R₁)₂ ;
W and Y are independently O, S, CH₂ or H₂ ;
R₁ is C₁ -C₆ straight or branched chain alkyl or alkenyl, which is substituted in one or more position(s) with (Ar₁)ₙ, (Ar₁)ₙ connected by a C₁ -C₆ straight or branched chain alkyl or alkenyl, C₃ -C₈ cycloalkyl, C₃ -C₈ cycloalkyl connected by a C₁ -C₆ straight or branched chain alkyl or alkenyl, Ar₂, or a combination thereof;
n is 1 or 2;
R₂ is either C₁ -C₉ straight or branched chain alkyl or alkenyl, C₃ -C₈ cycloalkyl, C₅ -C₇ cycloalkenyl, or Ar₁, wherein said alkyl, alkenyl, cycloalkyl or cycloalkenyl is either unsubstituted or substituted in one or more position(s) with C₁ -C₄ straight or branched chain alkyl or alkenyl, hydroxyl, or a combination thereof; and
Ar₁ and Ar₂ are independently a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; and wherein the heterocyclic ring contains 1-6 heteroatom(s) selected from the group consisting of O, N, S, and a combination thereof; or 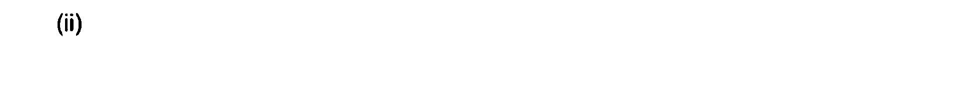 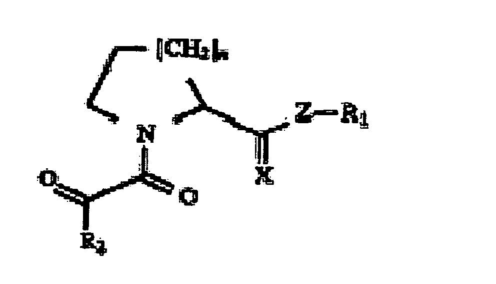 or a pharmaceutically acceptable salt thereof, wherein: n is 1 or 2;
X is O or S;
Z is either S, CH₂, CHR₁, or C(R₁)₂ ;
R₁ is either C₁ -C₅ straight or branched chain alkyl or alkenyl, which is substituted in one or more position(s) with (Ar₁)ₙ, (Ar₁)ₙ connected by a C₁ -C₅ straight or branched chain alkyl or alkenyl, or a combination thereof;
R₂ is either C₁ -C₉ straight or branched chain alkyl or alkenyl, C₃ -C₈ cycloalkyl, C₅-C₇ cycloalkenyl, or (Ar₁)ₙ ; and
Ar₁ is a mono- or bi- or tricyclic, carbo- or heterocyclic ring, which is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; and wherein the heterocyclic ring contains 1-6 heteroatom(s) selected from the group consisting of O, N, S, and a combination thereof; or 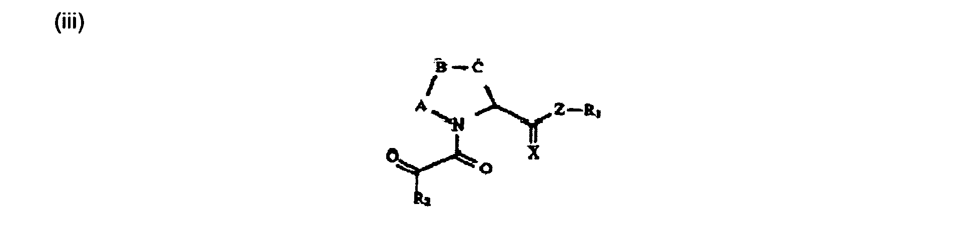 or a pharmaceutically acceptable salt thereof, wherein: A, B, C and D are independently CH₂, O, S, SO, SO₂, NH or NR₂ ;
X is O or S;
Z is S, CH₂, CHR₁ or C(R₁)₂ ;
R₁ is C₁ -C₆ straight or branched chain alkyl or alkenyl, which is substituted in one or more position(s) with (Ar₁)ₙ, (Ar₁)ₙ connected by a C₁ -C₆ straight or branched chain alkyl or alkenyl, C₃ -C₈ cycloalkyl, C₃ -C₈ cycloalkyl connected by a C₁ -C₆ straight or branched chain alkyl or alkenyl, Ar₂, or a combination thereof;
n is 1 or 2;
R₂ is either C₁ -C₉ straight or branched chain alkyl or alkenyl, C₃ -C₈ cycloalkyl, C₅ -C₇ cycloalkenyl, or Ar₁, wherein said alkyl, alkenyl, cycloalkyl or cycloalkenyl is either unsubstituted or substituted in one or more position(s) with C₁ -C₄ straight or branched chain alkyl or alkenyl, hydroxyl, or a combination thereof; and
Ar₁ and Ar₂ are independently a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; and wherein the heterocyclic ring contains 1-6 heteroatom(s) selected from the group consisting of O, N, S, and a combination thereof; or 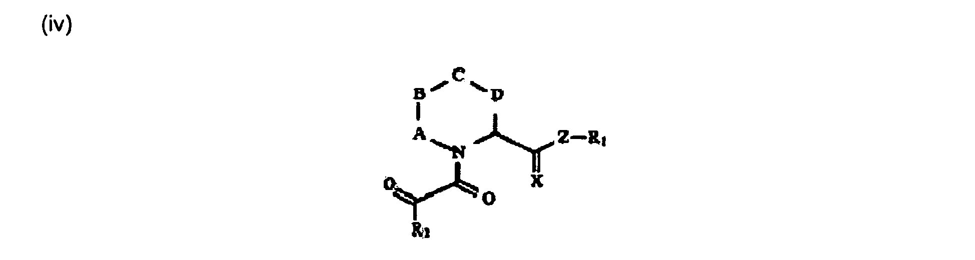 or a pharmaceutically acceptable salt thereof, wherein: A, B, C and D are independently CH₂, O, S, SO, SO₂, NH or NR₂ ;
X is O or S;
Z is S, CH₂, CHR₁ or C(R₁)₂;
R₁ is C₁ -C₆ straight or branched chain alkyl or alkenyl, which is substituted in one or more position(s) with (Ar₁)ₙ, (Ar₁)ₙ connected by a C₁ -C₆ straight or branched chain alkyl or alkenyl, C₃ -C₈ cycloalkyl, C₃ -C₈ cycloalkyl connected by a C₁ -C₆ straight or branched chain alkyl or alkenyl, Ar₂, or a combination thereof;
n is 1 or 2;
R₂ is either C₁-C₉ straight or branched chain alkyl or alkenyl, C₃ -C₈ cycloalkyl, C₅ -C₇ cycloalkenyl, or Ar₁, wherein said alkyl, alkenyl, cycloalkyl or cycloalkenyl is either unsubstituted or substituted in one or more position(s) with C₁-C₄ straight or branched chain alkyl or alkenyl, hydroxyl, or a combination thereof; and
Ar₁ and Ar₂ are independently a mono-, bi- or tricyclic, carbo- or heterocyclic ring, wherein the ring is either unsubstituted or substituted in one to three position(s) with halo, hydroxyl, nitro, trifluoromethyl, C₁-C₆ straight or branched chain alkyl or alkenyl, C₁-C₄ alkoxy, C₁-C₄ alkenyloxy, phenoxy, benzyloxy, amino, or a combination thereof; wherein the individual ring sizes are 5-6 members; and wherein the heterocyclic ring contains 1-6 heteroatom(s) selected from the group consisting of 0, N, S, and a combination thereof; or (v)
(2S)-2-(1-oxo-4-phenyl)butyl-1-(3,3-dimethyl-1,2-dioxopentyl)pyrrolidine;
(2S)-2-(1-oxo-4-(3-pyridyl)butyl-1-(3, 3-dimethyl-1,2-dioxopentyl)pyrrolidine;
(2S)-2-(1-oxo-4-phenyl)butyl-1- (3,3-dimethyl-1,2-dioxobutyl)pyrrolidine;
(2S)-2-(1-oxo-4-(3-pyridyl)butyl-1-(3,3-dimethyl-1,2-dioxobutyl)pyrrolidine
(2S)-2-(1-oxo-4(3-pyridyl)butyl-1- (2-cyclohexyl-1,2-dioxoethyl)pyrrolidine;
(2S)-2-(1-oxo-4-(3-pyridyl)butyl-1-(2-cyclopentyl-1,2-dioxoethyl)pyrrolidine;
(2S)-2-(1-oxo-4-(3-pyridyl)butyl-1-(2-cyclopentyl-1,2-dioxoethyl)pyrrolidine;
(2S)-2-(1-oxo-3-(9-fluorenyl)propyl-1- (3,3-dimethyl-1,2-dioxopentyl)pyrrolidine;
2-phenyl-1-ethylsulfhydryl(2S) -1-(3,3-dimethyl-1,2-dioxopentyl)-2-pyrrolidinecarboxylate;
(3-thioindolyl)methylsulfhydryl(2S)-1-(3,3-dimethyl-1,2-dioxopentyl)-2-pyrrolidinecarboxylate;
2-phenyl-1-ethylsulfhydryl (2S) -1-(2-cyclohexyl-1, 2-dioxopentyl)-2-pyrrolidinecarboxylate;
(2S)-2-(1-oxo-4- (para-methoxyphenyl)butyl-1- (3,3-dimethyl-1,2-dioxopentyl)pyrrolidine;
2-phenyl-1-ethylsulfhydryl (2R,S)1-(3,3-dimethyl-1,2-dioxopentyl)-2-piperidinecarboxylate;
2-phenyl-1-ethylsulfhydryl (2R,S)-1-(3,3-dimethyl-1,2-dioxobutyl)-2-piperidinecarboxylate;
2-phenyl-l-ethylsulfhydryl (2R,S)-1-(2-phenyl-1,2-dioxoethyl-1,2-piperidinecarboxylate;
2- (1-oxo-5- (para-methoxyphenyl)pentyl-1-(3,3-dimethyl-1,2-dioxopentyl)piperidine;
2- (1-oxo-5-phenyl)pentyl-1-(3,3-dimethyl-1,2-dioxopentyl)piperidine;
2- (1-oxo-5-phenyl)pentyl-1-(2-phenyt-1,2-dioxopentyl)piperidine; and
2- (1-oxo-5-phenyl)pentyl-1-(2-cyclohexyl-1,2-dioxopentyl)piperidine; or (vi)
2-phenyl-1-ethylsulfhydryl(2S)-1-(3,3-dimethyl-1,2-dioxopentyl)-2-pyrrolidinecarboxylate;
(3-thioindolyl)methylsulfhydryl(2S)-1-(3,3-dimethyl-1,2-dioxopentyl)-2-pyrrolidinecarboxylate; or
2-phenyl-1-ethylsulfhydryl(2S)-1-(2-cyclohexyl-1,2-dioxopentyl)-2-pyrrolidinecarboxylate,
(e) a N-glyoxyl-prolyl ester derivative which is (i) a compound of the formula: 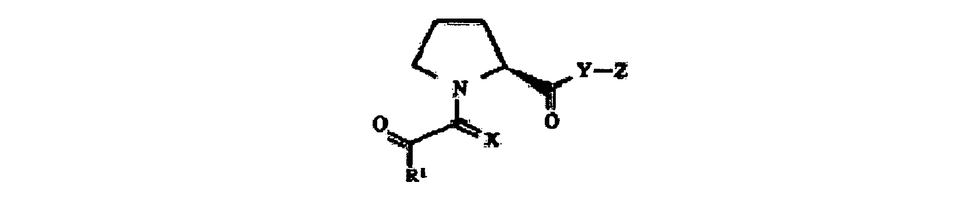 where R₁ is a C₁ -C₉ straight or branched chain alkyl or alkenyl group optionally substituted with C₃ -C₈ cycloalkyl, C₃ or C₅ cycloalkyl, C₅ -C₇ cycloalkenyl, or Ar₁, where said alkyl, alkenyl, cycloalkyl or cycloalkenyl groups may be optionally substituted with C₁ -C₄ alkyl, C₁ -C₄ alkenyl, or hydroxy, and where Ar₁ is selected from the group consisting of 1-napthyl, 2-napthyl, 2-indolyl, 3-indolyl, 2-furyl, 3-furyl, 2- thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁ -C₆ straight or branched alkyl or alkenyl, C₁ -C₄ alkoxy or C₁ -C₄ alkenyloxy, phenoxy, benzyloxy, and amino;
X is oxygen, sulfur, methylene (CH₂) , or H₂ ;
Y is oxygen or NR₂ where R₂ is hydrogen or C₁ -C₆ alkyl; and
Z is a C₂-C₆ straight or branched chain alkyl or alkenyl, wherein the alkyl chain is substituted in one or more positions with Ar₁ as defined above, C₃-C₈ cycloalkyl, cycloalkyl connected by a C₁-C₆ straight or unbranched alkyl or alkenyl chain, or Ar₂ where Ar₂ is selected from the group consisting of 2-indolyl, 3-indolyl, 2-furyl, 3-furyl, 2-thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁-C₆ straight or branched alkyl or alkenyl, C₁-C₄ alkoxy or C1-C4 alkenyloxy, phenoxy, benzyloxy, and amino;
Z may also be the fragment: 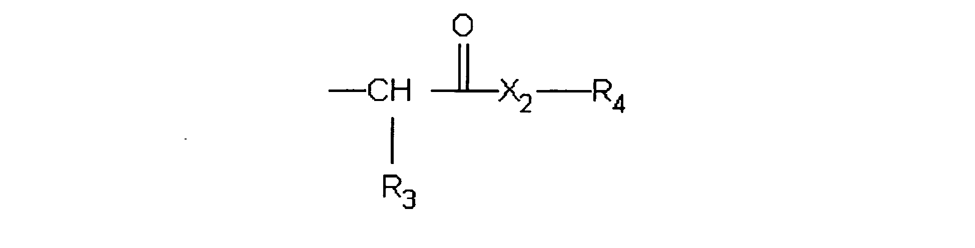 where R₃ is selected from the group consisting of straight or branched alkyl C₁-C₈ optionally substituted with C₃ -C₈ cycloalkyl, or Ar₁ as defined above, and unsubstituted Ar₁;
X₂ is O or NR₅ where R₅ is selected from the group consisting of hydrogen, C₁-C₆ straight or branched alkyl and alkenyl;
R₄ is selected from the group consisting of phenyl, benzyl, C₁ -C₅ straight or branched alkyl or alkenyl, and C₁ -C₅ straight or branched alkyl or alkenyl substituted with phenyl; or pharmaceutically acceptable salts or hydrates thereof;
(ii) a compound of formula: 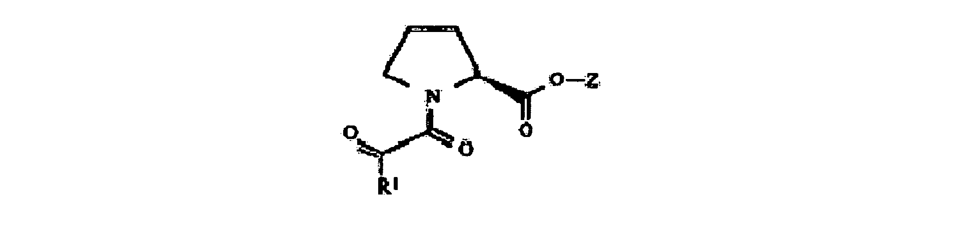 where R₁ is a C₁ -C₉ straight or branched chain alkyl or alkenyl group optionally substituted with C₃-C₆ cycloalkyl, C₃ or C₅ cycloalkyl, C₅ -C₇ cycloalkenyl, or Ar₁, where said alkyl, alkenyl, cycloalkyl or cycloalkenyl groups may be optionally substituted with C₁-C₄ alkyl, C₁-C₄ alkenyl, or hydroxy, and where Ar₁ is selected from the group consisting of 1-napthyl, 2-napthyl, 2-indolyl, 3-indolyl, 2-furyl, 3-furyl, 2- thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁ -C₆ straight or branched alkyl or alkenyl, C₁ -C₄ alkoxy or C₁ -C₄ alkenyloxy, phenoxy, benzyloxy, and amino;
Z is a C₂ -C₆ straight or branched chain alkyl or alkenyl, wherein the alkyl chain is substituted in one or more positions with Ar₁ as defined above, C₃ -C₈ cycloalkyl, cycloalkyl connected by a C₁ -C₆ straight or unbranched alkyl or alkenyl chain, or Ar₂ where Ar₂ is selected from the group consisting of 2-indolyl, 3-indolyl, 2-furyl, 3-furyl, 2-thiazolyl, 2-thienyl, 3-thienyl, 2-, 3-, or 4-pyridyl, or phenyl, having one to three substituents which are independently selected from the group consisting of hydrogen, halo, hydroxyl, nitro, trifluoromethyl, C₁ -C₆ straight or branched alkyl or alkenyl, C₅ -C₄ alkoxy or C₁ -C₄ alkenyloxy, phenoxy, benzyloxy, and amino; or pharmaceutically acceptable salts or hydrates thereof; or
(iii) a compound of formula: 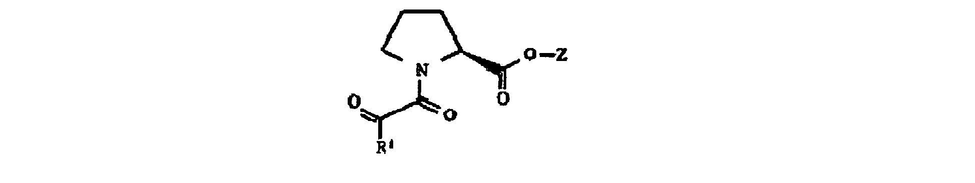 where R₁ is a C₁ -C₅ straight or branched chain alkyl or alkenyl group optionally substituted with C₃ to C₆ cycloalkyl, or Ar₁, where Ar₁ is selected from the group consisting of 2-furyl, 2-thienyl, or phenyl;
X is selected from the group consisting of oxygen and sulfur;
Y is oxygen; and
Z is a straight or branched chain alkyl or alkenyl, wherein the alkyl chain is substituted in one or more positions with Ar₁ as defined above, C₃-C₆ cycloalkyl, Ar₂ where Ar₂ is selected from the group consisting of 2-, 3-, or 4-pyridyl, or phenyl, having one to three substituents which are independently selected from the group consisting of hydrogen and C₁ -C₄ alkoxy; or
(iv) 3-phenyl-1-propyl (2S)-1-(3,3-dimethyl-1,2-dioxopentyl)-2-pyrrolidinecarboxylate,
3-phenyl-1-prop-2-(E)-enyl (2S)-I-(3,3-dimethyl-1,2-dioxopentyl)-2-pyrrolidinecarboxylate,
3-(3,4,5-trimethoxyphenyl)-1-propyl (2S)-1-(3,3-dimethyl-1,2-dioxopentyl)-2-pyrrolidinecarboxylate,
3-(3,4,5-trimethoxyphenyl)-1-prop-2-(E)-enyl (2S)-1-(3,3-dimethyl-1,2-dioxopentyl)-2-pyrrolidine carboxylate,
3-(4,5-methylenedioxyphenyl)-1-propyl (2S)-1-(3,3-dimethyl-1,2-dioxopentyl)-2-pyrrolidinecarboxylate,
3-(4,5-methylenedioxyphenyl)-1-prop-2-(E)-enyl (2S)-1-(3,3-dimethyl-1,2-dioxopentyl)-2-pyrrolidinecarboxylate,
3-cyclohexyl-1-propyl (2S)-1-(3,3-dimethyl-1,2-dioxopentyl)-2-pyrrolidinecarboxylate,
3-cyclohexyl-1-prop-2-(E)-enyl (28)-1-(3,3-dimethyl-1,2-dioxopentyl)-2-pyrrolidinecarboxylate,
(1R)-1,3-diphenyl-1-propyl (2S)-1-(3,3-dimethyl-1,2-dioxopentyl)-2-pyrrolidinecarboxylate,
3-phenyl-1-propyl(2S)-1-(1,2-dioxo-2-[2-furanyl])ethyl-2-pyrrolidinecarboxylate,
3-phenyl-1-propyl(2S)-1-(1,2-dioxo-2-[2-thienyl])ethyl-2-pyrrolidinecarboxylate,
3-phenyl-1-propyl(2S)-1-(1,2-dioxo-2-[2-thiazolyl])ethyl-2-pyrrolidinecarboxylate,
3-phenyl-1-propyl (2S)-1-(1,2-dioxo-2-phenyl)ethyl-2-pyrrolidinecarboxylate,
3-(2,5-dimethoxyphenyl)-1-propyl (2S)-1-(3,3-dimethy)-1,2-dioxopentyl)-2-pyrrolidinecarboxylate,
3-(2,5-dimethoxyphenyl)-1-prop-2-(E)-enyl (2S)-1-(3,3-dimethyl-1,2-dioxopentyl)-2-pyrrolidine-carboxylate,
2-(3,4,5-trimethoxyphenyl)-1-ethyl (2S)-1-(3,3-dimethyl-1,2-dioxopentyl)-2-pyrrolidinecarboxylate,
3-(3-Pyridyl)-1-propyl (2S)-1-(3,3-dimethyl-1,2-dioxopentyl-1,2-pyrrolidinecarboxylate,
3-(2-Pyridyl)-1-propyl (2S)-1-(3,3-dimethyl-1,2-dioxopentyl)-2-pyrrolidinecarboxylate,
3-(4-Pyridyl)-1-propyl (2S)-1-(3,3-dimethyl-1,2-dioxopentyl)-2-pyrrolidinecarboxylate,
3-phenyl-1-propyl (2S)-I-(2-cyclohexyl-1,2-dioxoethyl)-2-pyrrolidinecarboxylate,
3-phenyl-1-propyl(2S)-1-(2-tert-butyl-1,2-dioxoethyl)-2-pyrrolidinecarboxylate,
3-phenyl-1-propyl(2S)-1-(2-cyclohexylethyl-1,2-dioxoethyl)-2-pyrrolidinecarboxylate,
3-(3-pyridyl)-1-propyl(2S)-1-(2-cyclohexylethyl-1,2-dioxoethyl)-2-pyrrolidinecarboxylate,
3-(3-pyridyl)-1-propyl(2S)-1-(2-tert-butyl-1,2-dioxoethyl)-2-pyrrolidinecarboxylate,
3,3-diphenyl-1-propyl (2S)-(3,3-dimethyl-1,2-dioxopentyl)-2-pyrrolidinecarboxylate,
3-(3-pyridyl)-1-propyl (2S)-I-(2-cyclohexyl-1,2-dioxoethyl)-2-pyrrolidinecarboxylate,
3-(3-Pyridyl)-1-propyl (2S)-N-([2-thienyl] glyoxyl)pyrrolidinecarboxylate,
3,3-Diphenyl-1-propyl (2S)-1-(3,3-dimethyl-1,2-dioxobutyl)-2-pyrrolidinecarboxylate,
3,3-Diphenyl-1-propyl (2S)-1-cyclohexylglyoxyl-2-pyrrolidinecarboxylate,
3,3-Diphenyl-1-propyl (2S)-1-(2-thienyl)glyoxyl-2-pyrrolidinecarboxylate,
3-(2,5-dimethoxyphenyl)-1-propyl (2S)-1-(3,3-dimethyl-1,2-dioxopentyl)-2-pyrrolidinecarboxylate,
3-(2,5-dimethoxyphenyl)-1-prop-2-(E)-enyl (2S)-1-(3,3-dimethyl-1,2-dioxopentyl)-2-pyrrolidine-carboxylate,
2-(3,4,5-trimethoxyphenyl)-1-ethyl (2S)-1-(3,3-dimethyl-1,2-dioxopentyl)-2-pyrrolidinecarboxylate,
3-(3-Pyridyl)-1-propyl (2S)-1-(3,3-dimethyl-1,2-dioxopentyl)-2-pyrrolidinecarboxylate,
3-(2-Pyridyl)-1-propyl (2S)-1-(3,3-dimethyl-1,2-dioxopentyl)-2-pyrrolidinecarboxylate,
3-(4-Pyridyl)-1-propyl (2S)-1-(3,3-dimethyl-1,2-dioxopentyl)-2-pyrrolidinecarboxylate,
3-phenyl-1-propyl (2S)-1-(2-tert-butyl-1,2-dioxoethyl)-2-pyrrolidinecarboxylate,
3-phenyl-1-propyl(2S)-1-(2-cyclohexylethyl-1,2-dioxoethyl)-2-pyrrolidinecarboxylate,
3-(3-pyridyl)-1-propyl(2S)-l-(2-cyclohexyletho-1,2-dioxoethyl)-2-pyrrolidinecarboxylate,
3-(3-pyridyl)-1-propyl (2S)-l-(2-tert-butyl-1,2-dioxoethyl)-2-pyrrolidinecarboxylate,
3,3-diphenyl-1-propyl (2S)-1-(3,3-dimethyl-1,2-dioxopentyl-1,2-pyrrolidinecarboxylate,
3-(3-pyridyl)-1-propyl (2S)-I-(2-cyclohexyl-1,2-dioxoethyl)-2-pyrrolidinecarboxylate,
3-(3-Pyridyl)-1-propyl (2S)-N-([2-thienyl] glyoxyl)pyrrolidinecarboxylate,
3,3-Diphenyl-i-propyl (25)-1-(3,3-dimethyl-1,2-dioxobutyl)-2-pyrrolidinecarboxylate,
3,3-Diphenyl-1-propyl (2S)-i-cyclohexylglyoxyl-2-pyrrolidinecarboxylate, and
3,3-Diphenyl-1-propyl (2S)-I-(2-thienyl)glyoxyl-2-pyrrolidinecarboxylate,
(f) a pipecolic acid derivative which is 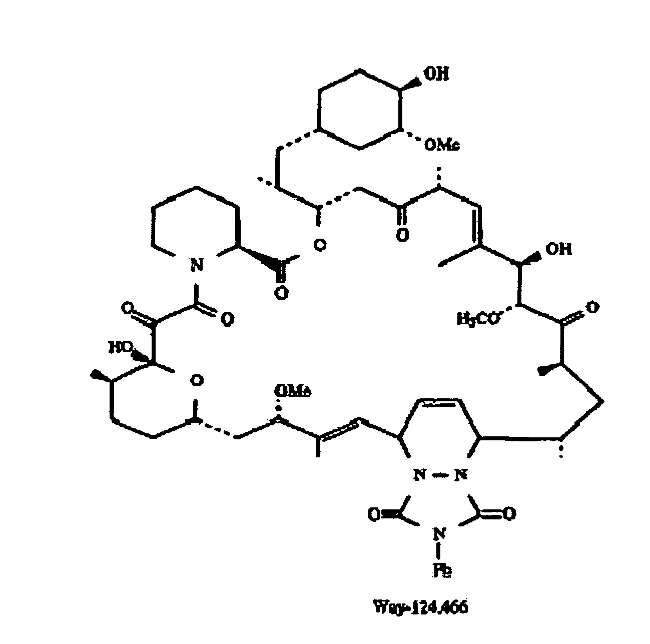 or 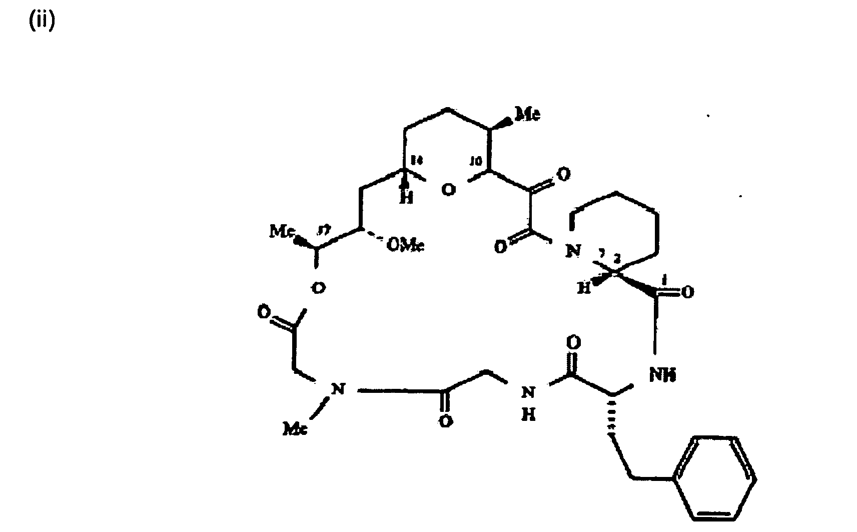 or 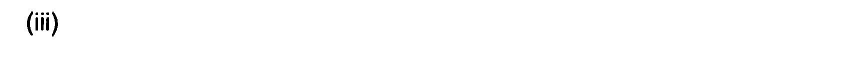 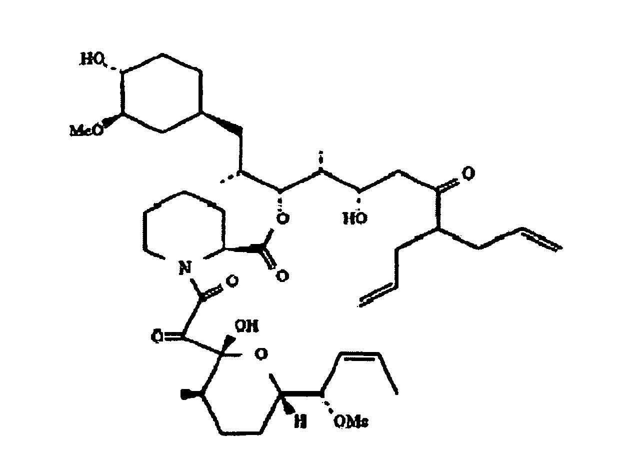 or
(iv)
4-(4-methoxyphenyl)butyl(2S)-1-[2-(3,4,5-trimethoxphenyl)acryloyl]hexahydro-2-pyridinecarboxylate,
4-(4-methoxyphenyl)butyl(2S)-1-[2-(3,4,5-trimethoxyphenyl)propanoyl]hexahydro-2-pyridinecarboxylate,
4-(4-methoxyphenyl)butyl(2S)-1-[2-oxo-2-(3,4,5-trimethoxyphenyl)acetyl]hexahydro-2-pyridinecarboxylate,
3-cyclohexylpropyl(2S)-1-(3,3-dimethyl-2-oxopentanoyl)hexahydro-2-pyridinecarboxylate,
3-phenylpropyl(2S)-1-(3,3-dimethyl-2-oxopentanoyl)hexahydro-2-pyridinecarboxylate,
3-(3,4,5-trimethoxyphenyl)propyl(2S)-1-(3, 3-dimethyl-2-oxopentanoyl)hexahydro-2-pyridinecarboxylate,
(1 R)-2,2-dimethyl-1-phenethyl-3-butenyl(2S)-1-(3,3-dimethyl-2-oxopentanoyl)hexahydro-2-pyridinecarboxylate,
(1 R)-1,3-diphenylpropyl(2S)-1-(3,3-dimethyl-2-oxopentanoyl)hexahydro-2-pyridinecarboxylate,
(1 R)-1-cyclohexyl-3-phenylpropyl(2S)-1-(3,3-dimethyl-2-oxopentanoyl)hexahydro-2-pyridinecarboxylate,
(1 S)-1,3-diphenylpropyl(2S)-1(3,3-dimethyl-2-oxopentanoyl)hexahydro-2-pyridinecarboxylate,
(1S)-1-cyclohexyl-3-phenylpropyl(2S)-1-(3,3-dimethyl-2-oxopentanoyl)hexahydro-2-pyridinecarboxylate,
(22aS)-15,15-dimethylperhydropyrido[2,1-c][1,9,4]dioxazacyclononadecine-1, 12, 16, 17-tetraone,
(24aS)-17,17-dimethylperhydropyrido[2,1-c][1,9,4]dioxazacyclohenicosine-1, 14, 18, 19-tetraone,
(3R,4R,23aS)-8-allyl-4,10-dimethyl-3-[2-(3-pyridyl)ethyl]-1,3,4,5,6,7,8,11,12,15,16,17,18,20,21,22,23,23a-octadecahydro-14H-pyrido[2,1-c][1,10,4]dioxazacycloicosine-1,7,14,17,18-pentaone,
(3R,4R,24aS)-8-allyl-4-,10-dimethyl-3-[2-(3-pyridyl)ethyl]-1,3,4,5,6,7,8,11,12,14,15,16,17,18,19,21,22,23,24,24a-icosahydropyrido[2,1-c](1,11,4]dioxazacyclohenicosine-1,7,14,18,19-pentaone,
(3R,4R,25aS)-8-allyl-4,10-dimethyl-3-[2-(3-pyridyl)ethyl]-1,3,4,5, 6, 7, 8,11,12,15,16,17,18,19,20,22,23,24,25, 25a-icosahydro-14H-pyrido[2,1-c][1,12,4]dioxazacyclodocosine-1,7,14,19,20-pentaone,
(1 R)-1-(3-benzoylphenyl)-3-phenylpropyl(1 R)-2-(3,3-dimethyl-2-oxopentanoyl)cyclohexane-1-carboxylate,
(1 R)-1 -[3-(dia))y)carbamoy))pheny)]-3-pheny)propy<(1 R)-2-(3,3-dimethyl-2-oxopentanoyl)cyclohexane-1-carboxylate,
ethyl1-(2-oxo-3-phenylpropanoyl)-2-piperidinecarboxylate,
ethyl1-pyruvoyl-2-piperidinecarboxylate,
ethyl1-(2-oxobutanoyl)-2-piperidinecarboxylate,
ethyl1-(3-methyl-2-oxobutanoyl)-2-piperidinecarboxylate,
ethyl1-(4-methyl-2-oxopentanoyl)-2-piperidinecarboxylate,
ethyl1-(3,3-dimethyl-2-oxobutanoyl)-2-piperidinecarboxylate,
ethyl1-(3,3-dimethyl-2-oxopentanoyl)-2-piperidinecarboxylate,
4-[2-(ethyloxycarbonyl)piperidinol-2,2-dimethyl-3,4-dioxobutyl acetate,
ethyl1-[2-(2-hydroxytetrahydro-2H-2-pyranyl)2-oxoacetyl]-2-piperidinecarboxylate,
ethyl1-[2-(2-methoxytetrahydro-2H-2-pyranyl)-2-oxoacetyl]-2-piperidinecarboxylate,
ethyl1-[2-(1-hydroxycyclohexyl)-2-oxoacetyt]-2-piperid inecarboxylate,
ethyl1-[2-(1-methoxycyclohexyl)-2-oxoeretyl[]-2-piperidi necarboxylate,
ethyl1-(2-cyclohexyl-2-oxoacetyl)-2-pipi peridinecarboxylate,
ethyl1-(2-oxo-2-piperidinoacetyl)2-piperidinecarboxylate,
ethyl1-[2-(3,4-dihydro-2H-6-pyranyl)-2-oxoacetyl)-2-piperidinecarboxylate,
ethyl1-(2-oxo-2-phenylacetyl)-2-piperidinecarboxylate,
ethyl1-(4-methyl-2-oxo-1-thioxopentyl)-2-piperidinecarboxylate,
3-phenylpropyl1-(2-hydroxy-3, 3-dimethylpentanoyl)-2-piperidi necarboxylate,
(1R)-1-phenyl-3-(3,4,5-trimethoxyphenyl)propyl1-(3,3-dimethylbutanoyl)-2-piperidinecarboxylate,
(1 R)-1,3-diphenylpropyl1-(benzylsulfonyl)-2-piperidinecarboxylate,
3-(3,4,5-trimethoxyphenyl)propyl1-(benzylsulfonyl)-2-piperidinecarboxylate
1-(2-[(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methy)tetrahydro-2H-2-pyranyl)-2-oxoacetyl)-2-piperidinecarboxylic acid,
methyl1-(2-[(2R,3R,6S)-6-[(2S,3E,5E,7E,9S,11R)-2,13,-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate,
isopropyl1-(2-[(2R,3R,6S)6-[(2S,3E,5E,7E,9S,11R)-2,13-dimethoxy-3,9,11-trimethyl-12-oxo-3,5,7-tridecatrienyl] -2-hydroxy-3-methyltetrahydro-2H-2-pyranyl)-2-oxoacetyl)-2-piperidinecarboxylate,
benzyl1-(2-[(2R,3R,6S)-6-[(2S,3E,5E,7E,9S,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate,
1-phenylethyl1-(2-[(2R,3R,6S)-6-[(2S,3E,5E,7E,9S,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate,
(Z)-3-phenyl-2-propenyl1-(2-(2,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate,
3-(3,4-dimethoxyphenyl)-propyl1-(2-[(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate,
N2-benzyl-1-(2-[(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate, and
N2-(3-phenylpropyl)-1-(2-(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylate; or 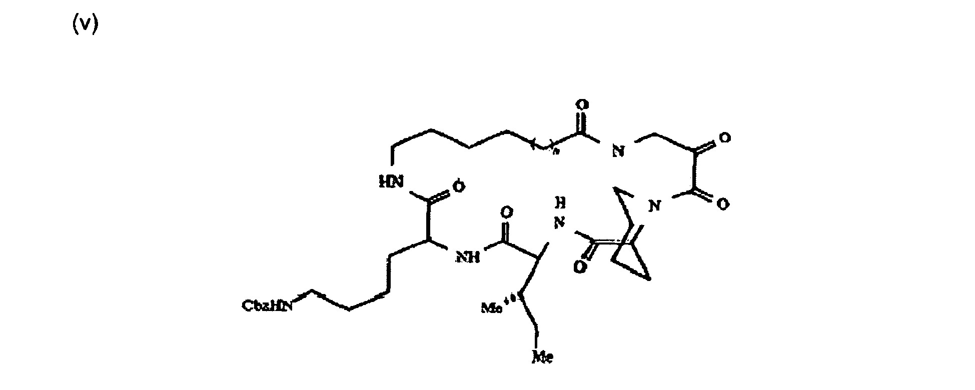 wherein n is 1, 2, or 3; 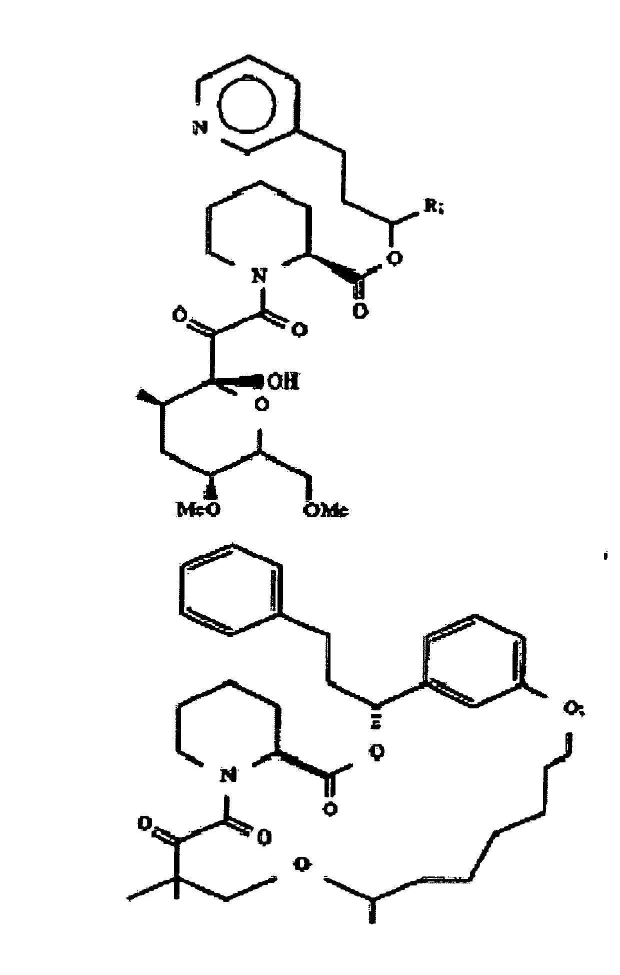 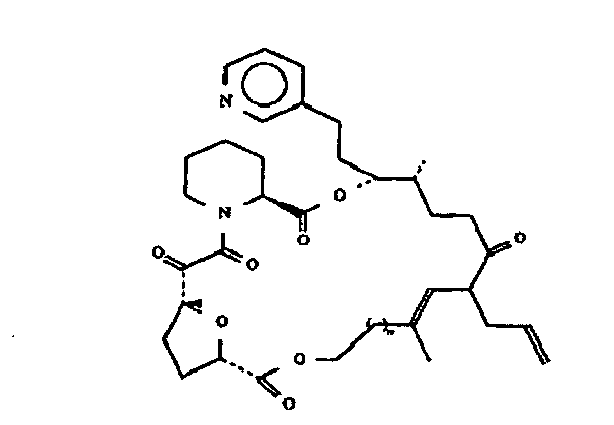 wherein n is 1, 2, or 3, 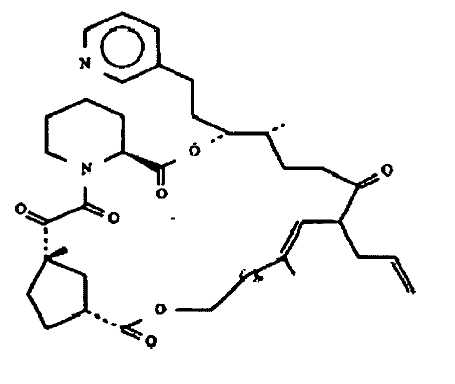 wherein n is 1, 2, or 3, 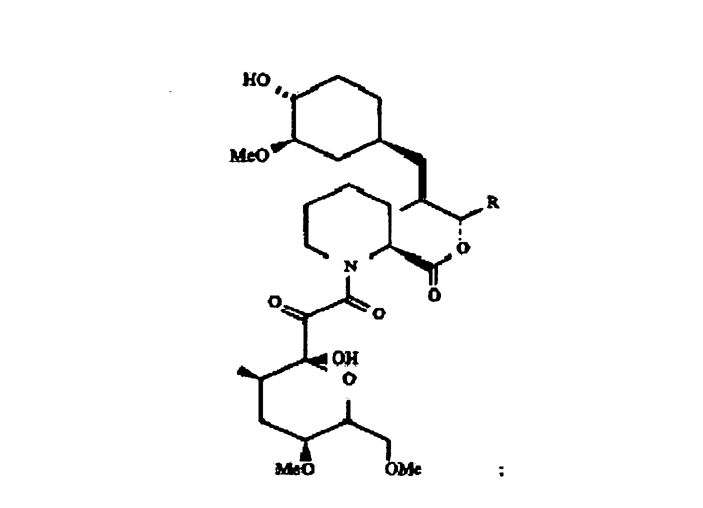 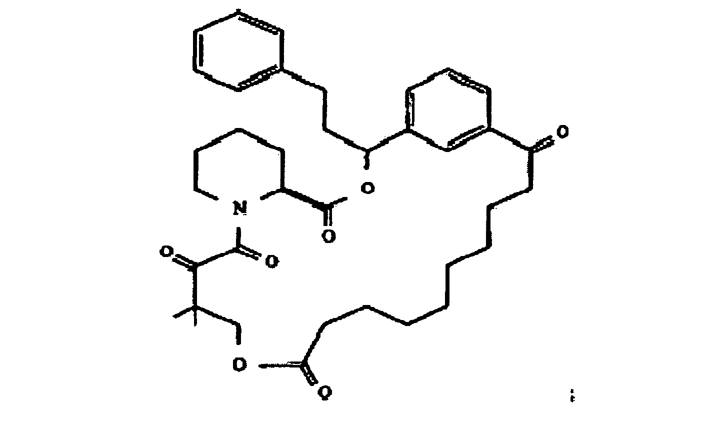 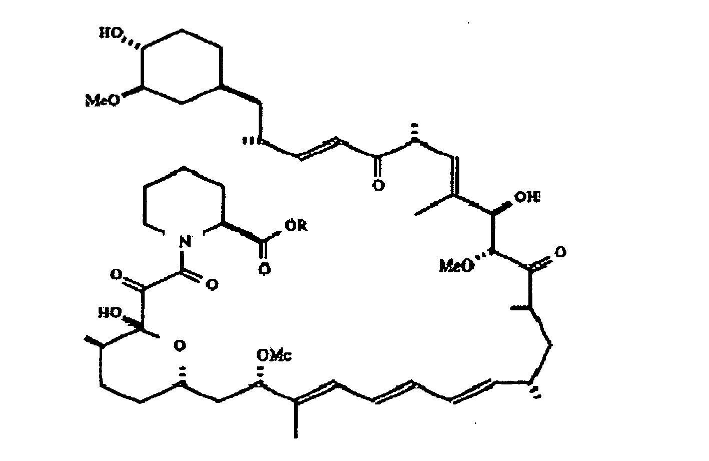 wherein R is Methyl (Me) or benzyl (Bn), 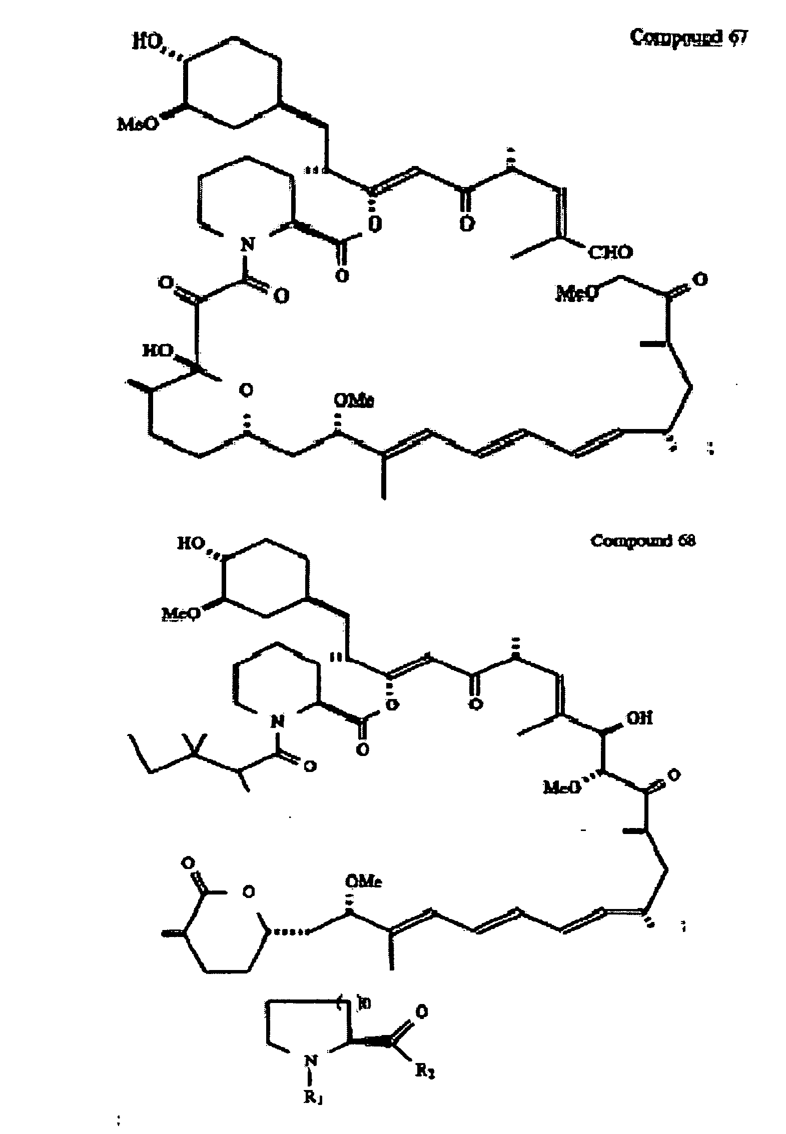 wherein n=2
R₁= 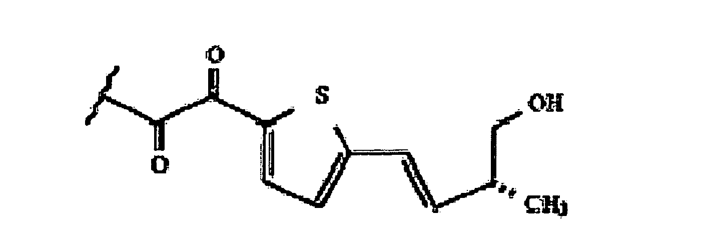 or 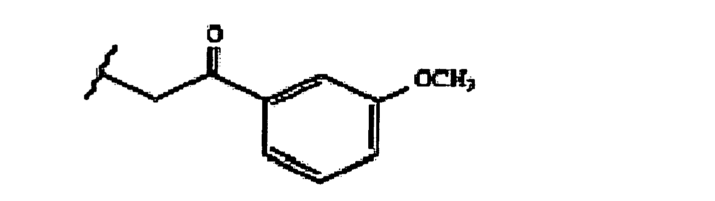 and R₂ =Phe-o-tert-butyl, 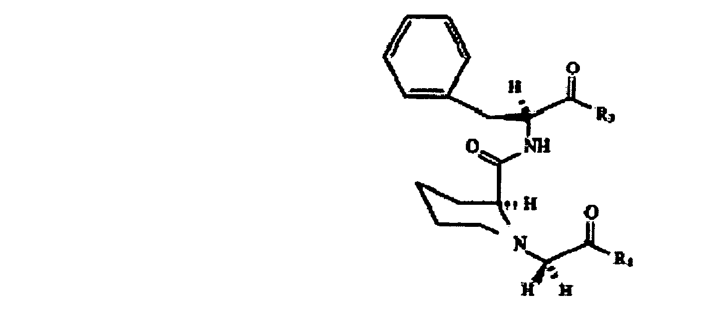 wherein
R₁ =m-OCH₃ Ph, R₃ =Val-o-tert-butyl;
R₁ =m-OCH₃ Ph, R₃ =Leu-o-tert-butyl;
R₁ =m-OCH₃ Ph, R₃ =Ileu-o-tert-butyl;
R₁ =m-OCH₃ Ph, R₃ =hexahydro-Phe-o-tert-butyl;
R₁ =m-OCH₃ Ph, R₃ =allylalanine-o-tert-butyl;
R₁ =B-naphthyl, R₃ =Val-o-tert-butyl; 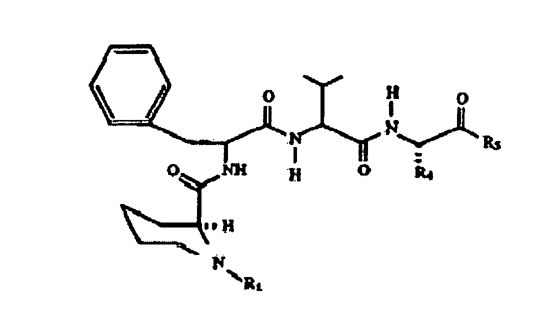 wherein R₁ =CH₂ (CO)-m-OCH₃ Ph
R₄ =CH₂ Ph
R₅ =OCH₃
or
R₁ =CH₂ (CO)-B-naphthyl
R₄ =CH₂ Ph
R₅ =OCH₃ 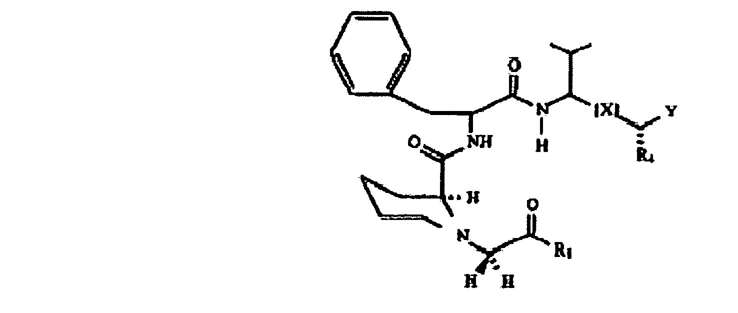 wherein R₁ =m-OCH₃ Ph
X=trans-CH=CH
R₄ =H
Y=OC(o)Ph
R₁ =m-OCH₃ Ph
X=trans-CH=CH
R₄ =H
Y=OC(o)CF₃R₁ =m-OCH₃ Ph
X=trans-CH=CH I
R₄ = -
Y=-
R₁ =m-OCH₃ Ph
X=trans-CH=CH
R₄ =H
Y=OCH₂ CH=CH₂
R₁ =m-OCH₃ Ph
X=C=O
R₄ =H
Y=Ph 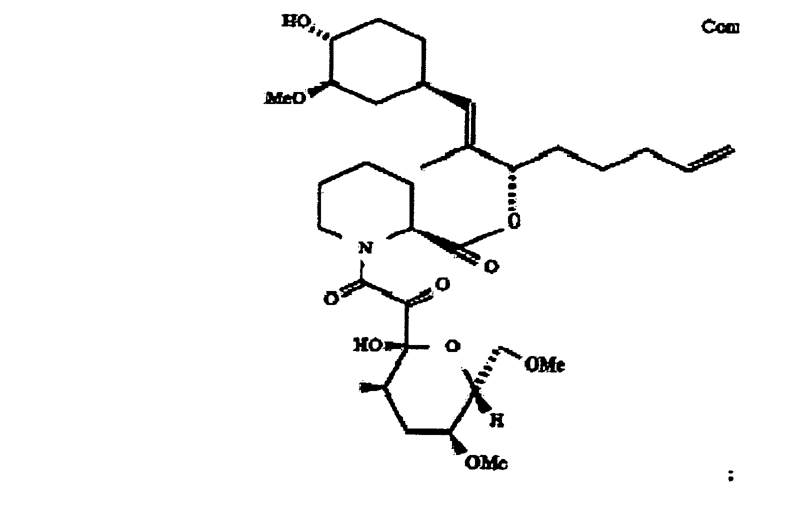 wherein R₁ =H, R₂ =OMe, and R₃ =CH₂ OMe;
R₁ =H, R₂ =H, and R₃ =H; and
R₁ =Me, R₂ =H, and R₃ =H,
(g) a heterocyclic ester or amide derivative which is 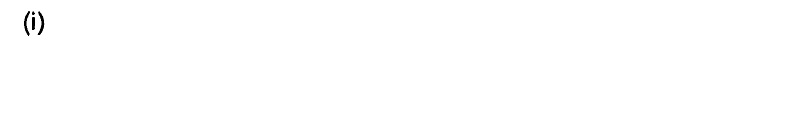 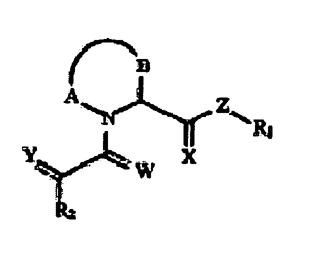 or a pharmaceutically acceptable salt thereof, wherein:
A and B, together with the nitrogen and carbon atoms to which they are respectively attached, form a 5-7 membered saturated or unsaturated heterocyclic ring containing, in addition to the nitrogen atom, at least one additional O, S, SO, SO₂, NH or NR₁ heteroatom in any chemically stable oxidation state;
X is O or S;
Z is O, NH or NR₁ ;
W and Y are independently O, S, CH₂ or H₂;
R₁ is C₁ -C₆ straight or branched chain alkyl or alkenyl, which is substituted in one or more position(s) with (Ar₁)ₙ, (Ar₁)ₙ connected by a C₁ -C₆ straight or branched chain alkyl or alkenyl, C₃ -C₈ cycloalkyl, C₃ -C₈ cycloalkyl connected by a C₁-C₆ straight or branched chain alkyl or alkenyl, Ar₂, or a combination thereof;
n is 1 or 2;
R₂ is either C₁ -C₉ straight or branched chain alkyl or alkenyl, C₃ -C₈ cycloalkyl, C₅ -C₇ cycloalkenyl, or Ar₁, wherein said alkyl, alkenyl, cycloalkyl or cycloalkenyl is either unsubstituted or substituted in one or more position(s) with C₁ -C₄ straight or branched chain alkyl or alkenyl, hydroxyl, or a combination thereof; and
Ar₁ and Ar₂ are independently a mono-, bi- or tricyclic, carbo- or heterocyclic ring,
wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy; amino, or a combination thereof; wherein the individual ring sizes are 5-6 members; and wherein the heterocyclic ring contains 1-6 heteroatom(s) selected from the group consisting of O, N, S, and a combination thereof; or 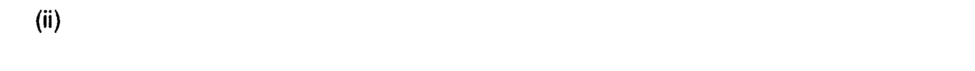 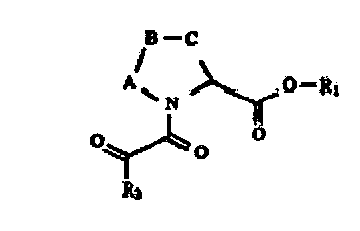 or a pharmaceutically acceptable salt thereof, wherein:
A, B and C are independently CH₂, O, S, SO, SO₂, NH or NR₁;
R₁ is C₁-C₅ straight or branched chain alkyl or alkenyl, which is substituted in one or more position(s) with (Ar₁)ₙ, (Ar₁)ₙ connected by a C₁-C₆ straight or branched chain alkyl or alkenyl, or a combination thereof;
n is 1 or 2;
R₂ is either C₁ -C₉ straight or branched chain alkyl or alkenyl, C₃ -C₈ cycloalkyl, C₅ -C₇ cycloalkenyl, or Ar₁ ; and
Ar₁ is a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; and wherein the heterocyclic ring contains 1-6 heteroatom(s) selected from the group consisting of O, N, S, and a combination thereof; or 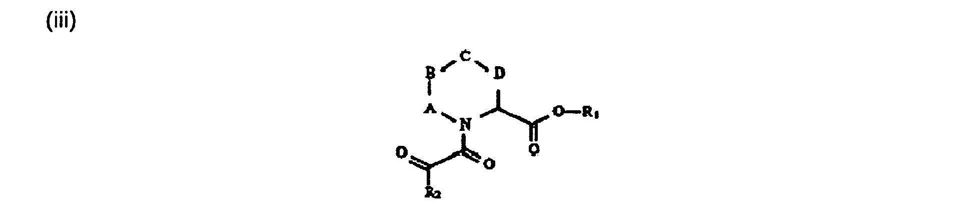
or a pharmaceutically acceptable salt thereof, wherein:
A, B, C and D are independently CH₂, O, S, SO, SO₂, NH or NR₁ ;
R₁ is C₁-C₅ straight or branched chain alkyl or alkenyl, which is substituted in one or more position(s) with (Ar₁)ₙ, (Ar₁)ₙ connected by a C₁ -C₆ straight or branched chain alkyl or alkenyl, or a combination thereof;
n is 1 or 2;
R₂ is either C₁ -C₉ straight or branched chain alkyl or alkenyl, C₃ -C₈ cycloalkyl, C₅ -C₇ cycloalkenyl, or Ar₁ ; and
Ar₁ is a mono-, bi- or tricyclic, carbo- or heterocyclic ring, wherein the ring is either unsubstituted or substituted in one to three position(s) with halo, hydroxyl, nitro, trifluoromethyl, C₁-C₆ straight or branched chain alkyl or alkenyl, C₁-C₄ alkoxy, C₁-C₄ alkenyloxy, phenoxy, benzyloxy, amino, or a combination thereof; wherein the individual ring sizes are 5-6 members; and wherein the heterocyclic ring contains 1-6 heteroatom(s) selected from the group consisting of O, N, S, and a combination thereof; or
(iv) 3-phenyl-1-propyl(2S)-1-(3,3-dimethyl-1,2-dioxopentyl)-2-(4-thiazolidine)carboxylate; and
3-(3-pyridyl)-1-propyl(2S)-1-(3,3-dimethy)-1,2-dioxopentyl)-2-(4-thiazotidine) carboxylate,
(h) a piperidine derivative which is 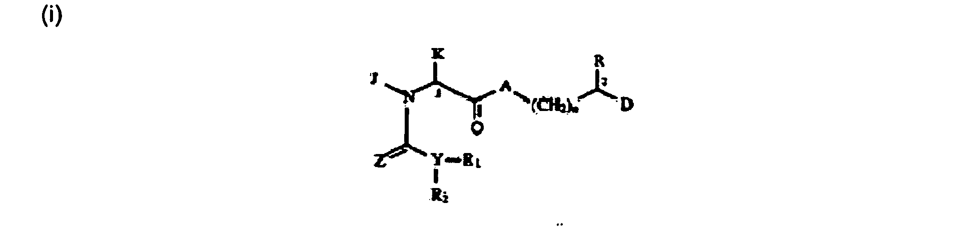 and pharmaceutically acceptable derivatives thereof, wherein A is CH₂ oxygen, NR₁ ; wherein R₁, B and D are independently:
Ar, (C1-C6)-straight or branched alkyl, (C2-C6)-straight or branched alkenyl or alkynyl, (C5-C7)-cycloalkyl substituted (C₁ -C6)-straight or branched alkyl, (C3-C6)-straight or branched alkenyl or alkynyl, (C5-C7)-cycloalkenyl substituted (C1-C6)-straight or branched alkyl, (C3-C6)-straight or branched alkenyl or alkynyl, Ar-substituted (C1-C6)-straight or branched alkyl, or Ar-substituted (C3-C6)-straight or branched alkenyl or alkynyl,
wherein any one of the CH₂ groups of said alkyl chains is optionally replaced by a heteroatom selected from the group consisting of O, S, SO, SO₂, and NR, wherein R is selected from hydrogen, (C1-C4)-straight or branched alkyl, (C3-C4)-straight or branched alkenyl or alkynyl, or (C₁-C₄) bridging alkyl wherein a bridge is formed between the nitrogen and a carbon atom of said heteroatom containing chain to form a ring, and wherein said ring is optionally fused to an Ar group;
J is selected from hydrogen, (C1-C6)-straight or branched alkyl, (C3-C6)-straight or branched alkenyl, or -CH₂ Ar;
K is selected from (C1-C4)-straight or branched alkyl, -CH₂ Ar, or cyclohexylmethyl; or J and K are taken together to form a 5-7 membered heterocyclic ring which optionally contains a heteroatom selected from O, S, SO or SO₂;
Z is O or S;
Y is O or N, wherein,
when Y is O, then R₁ is a lone pair (as used herein, the term "lone pair" refers to a lone pair of electrons, such as the lone pair of electrons present on divalent oxygen) and R₂ is selected from Ar, (C1-C6)-straight or branched alkyl, or (C3-C6)-straight or branched alkenyl or alkynyl; and
when Y is N, then R₁ and R₂ are independently selected from Ar, (C1-C6)-straight or branched alkyl, or (C3-C6)-straight or branched alkenyl or alkynyl; or R₁ and R₂ are taken together to form a heterocyclic 5-6 membered ring selected from pyrrolidine, imidazolidine, pyrazolidine, piperidine, or piperazine;
wherein Ar is selected from phenyl, 1-naphthyl, 2-naphthyl, indenyl, azulenyl, fluorenyl, or anthracenyl,
2-furyl, 3-furyl, 2-thienyl, 3-thienyl, 2-pyridyl, 3-pyridyl, 4-pyridyl, pyrrolyl, oxazolyl, thiazolyl, imidazolyl, pyrazolyl, 2-pyrazolinyl, pyrazolidinyl, isoxazolyl, isotriazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, benzo[b]thiophenyl, 1H-indazolyl, benzimidazolyl, benzthiazolyl, purinyl, 4H-quinolizinyl, quinolinyl, 1,2,3,4-tetrahydro-quinolinyl, isoquinolinyl, 1,2,3,4-tetrahydro-isoquinolinyl, cinnolinyl, phthalazinyl, quinazolinyl, quinoxalinyl, 1,8-naphthyridinyl, pteridinyl, carbazolyl, acridinyl, phenazinyl, phenothiazinyl, or phenoxazinyl;
wherein Ar optionally contains one to three substituents which are independently selected from hydrogen, halogen, hydroxyl, nitro, -SO₃ H, trifluoromethyl, trifluoromethoxy, (C1-C6)-straight or branched alkyl, (C2-C6)-straight or branched alkenyl, O-[(C1-C6)-straight or branched alkyl], O-[(C3-C4)-straight or branched alkenyl], O-benzyl, O-phenyl, 1,2-methylenedioxy, -NR₃ R₄, carboxyl, N-(C1-C5-straight or branched alkyl or C3-C5-straight or branched alkenyl) carboxamides, N,N-di-(C1-C5-straight or branched alkyl or C3-C5-straight or branched alkenyl) carboxamides, morpholinyl, piperidinyl, O-Z, CH₂ -(CH₂)_{q} -Z, O-(CH₂)_{q} -Z, (CH₂)_{q-}Z--O-Z, or CH=CH--Z;
wherein R₃ and R₄ are independently selected from (C1-C6)-straight or branched alkyl, (C3-C6)-straight or branched alkenyl, hydrogen or benzyl; or wherein R₃ and R₄ are taken together to form a 5-6 membered or a 8-11 membered heterocyclic ring such as, for example, piperidinyl, morpholinyl or pyrrolidinyl;
wherein Z is selected from 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, or pyrimidyl;
wherein q is 0-2; and
n is 0 or 1; or
(ii)
S)-1-((3,4,5-Trimethoxyphenyl)-methyl-carbamoyl)piperidine-2-carboxylic acid 4-pyridin-3-yl-1-(3-pyridin-3-yl-propyl)-butyl ester;
(S)-1-((3-Trifluoromethylphenyl)-methyl-carbamoyl)piperidine-2-carboxylic acid 4-pyridin-3-yl-1-(3-pyridin-3-yl-propyl)-butyl ester;
(S)-1-((4-Tert-butylphenyl)-methyl-carbamoyl)piperidine-2-carboxylic acid 4-pyridin-3-yl-1-(3-pyridin-3-yl-propyl)-butyl ester;
(S)-1-((4-Isopropylphenyl)-methyl-carbamoyl)piperidine-2-carboxylic acid 4-pyridin-3-yl-1-(3-pyridin-3-yl-propyl)-butyl ester;
(S)-1-(Piperidine-1-carbonylypiperidine-2-carboxylic acid 4-pyridin-3-yl-1-(3-pyridin-3-yl-propylrbutyl ester;
(S)-1-((3,4,5-Trimethoxyphenyl)-methyl-carbamoyl)piperidine-2-carboxylic acid 4-pyridin-1-yl-1-(3-pyridin-1-yl-propyl)-butyl ester;
(S)-Piperidine-1,2-dicarboxylic acid 1-(3,4,5-trimethoxyphenyl)ester-2-(4-pyridin-3-yl-1-(3-pyridin-3-yl-propyl)-butyl)ester;
(S)-1-((3,4,5-Trimethoxyphenyl)-methyl-carbamoyl)piperidine-2-carboxylic acid 1-(2-phenyl-ethyl)-3-phenyl-propyl ester;
4-(Methyl-(2-(1-phenethyl-3-phenyl-propoxycarbonyl)piperidine-1-carbonyl)-amino)-benzenesulfonic acid;
(8)-Piperidine-2-carboxylic acid 1-benzyloxy-methyl-2-benzyloxyethyl ester;
(S)-1-(Methyl-(4-morpholin-1-yl-phenyl)-carbamoyl)piperidine-2-carboxylic acid 2-benzyloxy-1-(benzyloxy-methyl)-ethyl ester;
(S)-1-(Methyl-(4-piperidin-1-yl-phenyl)-carbamoyl)piperidine-2-carboxylic acid 2-benzyloxy-1-(benzyloxy-methyl)-ethyl ester;
(S)-Piperidine-1,2-dicarboxylic acid 2-(2-benzyloxy-1-(benzyloxymethyl)-ethyl)ester-1-quinolin-5-yl ester;
(S)-Piperidine-1,2-dicarboxylic acid 2-(2-benzyloxy-1-(benzyloxymethyl)-ethyl)ester-1-pyridin-3-yl ester;
(i) a N-oxide of a heterocyclic ester, amide, thioester, or ketone which is 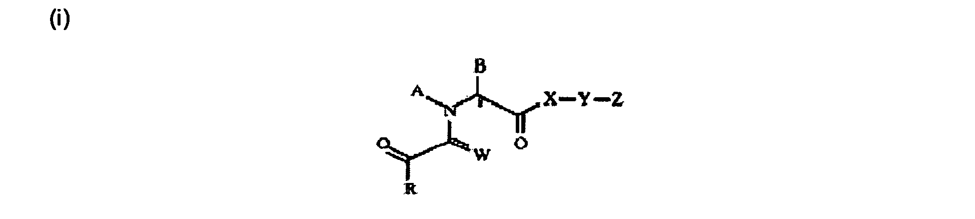 or a pharmaceutically acceptable salt thereof, wherein:
A and B are taken together, with the nitrogen and carbon atoms to which they are respectively attached, to form a 5-7 membered saturated or unsaturated heterocyclic ring containing any combination of CH₂, O, S, SO, SO₂, NH or NR₁ in any chemically stable oxidation state;
W is O, S, CH₂, or H₂;
R is a C₁-C₆ straight or branched chain alkyl or alkenyl group optionally substituted with C₃ -C₈ cycloalkyl, C₃ or C₅ cycloalkyl, C₅ -C₇ cycloalkenyl, or Ar₁, where said alkyl, alkenyl, cycloalkyl, or cycloalkenyl groups may be optionally substituted with C₁-C₄ alkyl, C₁ -C₄ alkenyl, or hydroxy, and where Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl, or phenyl, having one to three substituents which are independently selected from the group consisting of hyrogen, halo, hydroxyl, nitro, trifluoromethyl, C₁ - C₆ straight or branched alkyl or alkenyl, C₁-C₄ alkenyloxy, phenoxy, benzyloxy, and amino;
X is O, NH, NR₁, S, CH, CR₁, or C(R₁)₂;
Y is a direct bond, or a C₁ -C₆ straight or branched chain alkyl or alkenyl which is optionally substituted in one or more positions with C₁-C₆ straight or branched chain alkyl or alkenyl, or C₃ -C₈ cycloalkyl, or C₅-C₇ cycloalkenyl, or hydroxyl, or carbonyl oxygen, or with Ar, where said alkyl, alkenyl, cycloalkyl, cycloalkenyl, or Ar group is optionally substituted with C₁ -C₄ alkyl, C₁ -C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₂, S, SO, or SO₂, where R₂ is selected from the group consisting of hydrogen, (C₁ -C₄)-straight or branched chain alkyl, (C₃ -C₄)-straight or branched chain alkenyl or alkynyl, and (C₁ -C₄) bridging alkyl wherein a bridge is formed between the nitrogen and a carbon atom of said alkyl or alkenyl chain containing said heteroatom to form a ring, wherein said ring is optionally fused to an Ar group; and
Z is an aromatic or tertiary alkyl amine oxidized to a corresponding N-oxide, wherein the aromatic amine is Ar oxidized to a corresponding N-oxide where Ar is a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; wherein the heterocyclic ring contains 1-6 heteroatom(s) selected from the group consisting of O, N, S, and a combination thereof wherein at least one of the heteroatoms is N, and wherein the alkyl amine is oxidized to a corresponding N-oxide where alkyl is a C₁ -C₆ straight or branched chain alkyl or alkenyl which is optionally substituted in one or more positions with C₁-C₆ straight or branched chain alkyl or alkenyl, or C₃-C₈ cycloalkyl, or C₅-C₇ cycloalkenyl, or hydroxyl, or carbonyl oxygen, or with Ar, where said alkyl, alkenyl, cycloalkyl, cycloalkenyl, or Ar group is optionally substituted with C₁-C₄ alkyl, C₁-C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₁, S, SO, or SO₂; and,
R₁ is hydrogen, (C₁-C₄)-straight or branched chain alkyl, (C₃-C₄)-straight or branched chain alkenyl or alkynyl, or R₁ is Y--Z, as defined above; or 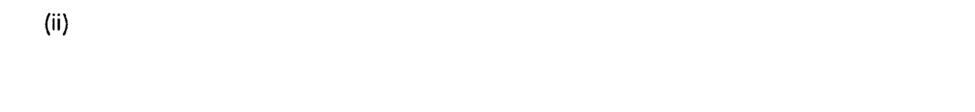 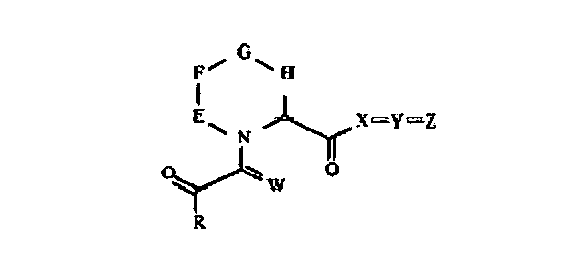 or a pharmaceutically acceptable salt thereof, wherein: E, F, G and H are independently CH₂, O, S, SO, SO₂, NH or NR₁ ;
W is O, S, CH₂, or H₂ ;
R is a C₁ -C₆ straight or branched chain alkyl or alkenyl group optionally substituted with C₃ -C₈ cycloalkyl, C₃ or C₅ cycloalkyl, C₅ -C₇ cycloalkenyl, or Ar₁, where said alkyl, alkenyl, cycloalkyl, or cycloalkenyl groups may be optionally substituted with C₁ -C₄ alkyl, C₁ -C₄ alkenyl, or hydroxy, and where Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl, or phenyl, having one to three substituents which are independently selected from the group consisting of hyrogen, halo, hydroxyl, nitro, trifluoromethyl, C₁ - C₆ straight or branched alkyl or alkenyl, C₁ -C₄ alkenyloxy, phenoxy, benzyloxy, and amino;
X is O, NH, NR₁, S, CH, CR₁, or C(R₁)₂ ;
Y is a direct bond, or a C₁-C₆ straight or branched chain alkyl or alkenyl which is optionally substituted in one or more positions with C₁-C₆ straight or branched chain alkyl or alkenyl, or C₃-C₈ cycloalkyl, or C₅-C₇ cycloalkenyl, or hydroxyl, or carbonyl oxygen, or with Ar, where said alkyl, alkenyl, cycloalkyl, cycloalkenyl, or Ar group is optionally substituted with C₁-C₄ alkyl, C₁-C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₂, S, SO, or SO₂, where R₂ is selected from the group consisting of hydrogen, (C₁ -C₄)-straight or branched chain alkyl, (C₃ -C₄)-straight or branched chain alkenyl or alkynyl, and (C₁ -C₄) bridging alkyl wherein a bridge is formed between the nitrogen and a carbon atom of said alkyl or alkenyl chain containing said heteroatom to form a ring, wherein said ring is optionally fused to an Ar group; and
Z is an aromatic or tertiary alkyl amine oxidized to a corresponding N-oxide, wherein the aromatic amine is Ar oxidized to a corresponding N-oxide where Ar is a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; wherein the heterocyclic ring contains 1-6 heteroatom(s) selected from the group consisting of O, N, S, and a combination thereof wherein at least one of the heteroatoms is N, and wherein the alkyl amine is oxidized to a corresponding N-oxide where alkyl is a C₁ -C₆ straight or branched chain alkyl or alkenyl which is optionally substituted in one or more positions with C₁ -C₆ straight or branched chain alkyl or alkenyl, or C₃ -C₈ cycloalkyl, or C₅ -C₇ cycloalkenyl, or hydroxyl, or carbonyl oxygen, or with Ar, where said alkyl, alkenyl, cycloalkyl, cycloalkenyl, or Ar group is optionally substituted with C₁ -C₄ alkyl, C₁ -C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₁, S, SO, or SO₂ ; and,
R₁ is hydrogen, (C₁ -C₄)-straight or branched chain alkyl, (C₃ -C₄)-straight or branched chain alkenyl or alkynyl, or R₁ is Y-Z, as defined above; or 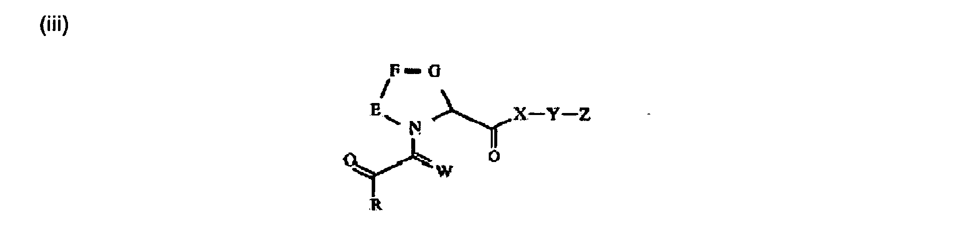 or a pharmaceutically acceptable salt thereof, wherein: E, F, and G are independently CH₂, O, S, SO, SO₂, NH or NR₁ ;
W is O, S, CH₂, or H₂ ;
R is a C₁ -C₆ straight or branched chain alkyl or alkenyl group optionally substituted with C₃ -C₈ cycloalkyl, C₃ or C₅ cycloalkyl, C₅ -C₇ cycloalkenyl, or Ar₁, where said alkyl, alkenyl, cycloalkyl, or cycloalkenyl groups may be optionally substituted with C₁ -C₄ alkyl, C₁ -C₄ alkenyl, or hydroxy, and where Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl, or phenyl, having one to three substituents which are independently selected from the group consisting of hyrogen, halo, hydroxyl, nitro, trifluoromethyl, C₁ - C₆ straight or branched alkyl or alkenyl, C₁-C₄ alkenyloxy, phenoxy, benzyloxy, and amino;
X is O, NH, NR₁, S, CH, CR₁, or C(R₁)₂ ;
Y is a direct bond, or a C₁-C₆ straight or branched chain alkyl or alkenyl which is optionally substituted in one or more positions with C₁-C₆ straight or branched chain alkyl or alkenyl, or C₃ -C₈ cycloalkyl, or C₅ -C₇ cycloalkenyl, or hydroxyl, or carbonyl oxygen, or with Ar, where said alkyl, alkenyl, cycloalkyl, cycloalkenyl, or Ar group is optionally substituted with C₁ -C₄ alkyl, C₁ -C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₂, S, SO, or SO₂, where R₂ is selected from the group consisting of hydrogen, (C₁ -C₄)-straight or branched chain alkyl, (C₃ -C₄)-straight or branched chain alkenyl or alkynyl, and (C₁ -C₄) bridging alkyl wherein a bridge is formed between the nitrogen and a carbon atom of said alkyl or alkenyl chain containing said heteroatom to form a ring, wherein said ring is optionally fused to an Ar group; and
Z is an aromatic or tertiary alkyl amine oxidized to a corresponding N-oxide, wherein the aromatic amine is Ar oxidized to a corresponding N-oxide where Ar is a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; wherein the heterocyclic ring contains 1-6 heteroatom(s) selected from the group consisting of O, N, S, and a combination thereof wherein at least one of the heteroatoms is N, and wherein the alkyl amine is oxidized to a corresponding N-oxide
where alkyl is a C₁ -C₆ straight or branched chain alkyl or alkenyl which is optionally substituted in one or more positions with C₁ -C₆ straight or branched chain alkyl or alkenyl, or C₃ -C₈ cycloalkyl, or C₅ -C₇ cycloalkenyl, or hydroxyl, or carbonyl oxygen, or with Ar, where said alkyl, alkenyl, cycloalkyl, cycloalkenyl, or Ar group is optionally substituted with C₁ -C₄ alkyl, C₁ -C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₁, S, SO, or SO₂ ; and,
R₁ is hydrogen, (C₁ -C₄)-straight or branched chain alkyl, (C₃ -C₄)-straight or branched chain alkenyl or alkynyl, or R₁ is Y-Z, as defined above; or 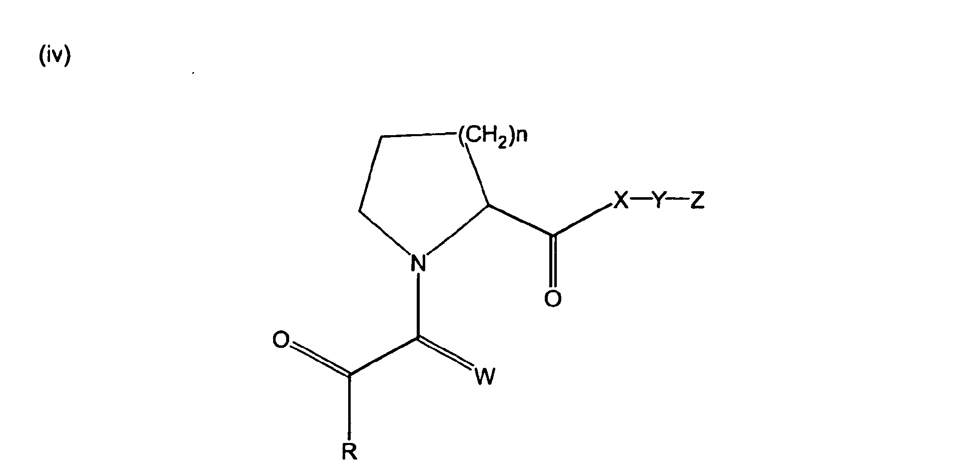 or a pharmaceutically acceptable salt thereof, wherein: n is 1, 2 or 3 forming a 5-7 member heterocyclic ring;
W is O, S, CH₂, or H₂ ;
R is a C₁ -C₆ straight or branched chain alkyl or alkenyl group optionally substituted with C₃ -C₈ cycloalkyl, C₃ or C₅ cycloalkyl, C₅ -C₇ cycloalkenyl, or Ar₁, where said alkyl, alkenyl, cycloalkyl, or cycloalkenyl groups may be optionally substituted with C₁ -C₄ alkyl, C₁ -C₄ alkenyl, or hydroxy, and where Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl, or phenyl, having one to three substituents which are independently selected from the group consisting of hyrogen, halo, hydroxyl, nitro, trifluoromethyl, C₁ - C₆ straight or branched alkyl or alkenyl, C₁ -C₄ alkenyloxy, phenoxy, benzyloxy, and amino;
X is O, NH, NR₁, S, CH, CR₁, or C(R₁)₂;
Y is a direct bond, or a C₁-C₆ straight or branched chain alkyl or alkenyl which is optionally substituted in one or more positions with C₁-C₆ straight or branched chain alkyl or alkenyl, or C₃-C₈ cycloalkyl, or C₅-C₇ cycloalkenyl, or hydroxyl, or carbonyl oxygen, or with Ar, where said alkyl, alkenyl, cycloalkyl, cycloalkenyl, or Ar group is optionally substituted with C₁-C₄ alkyl, C₁-C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₂, S, SO, or SO₂, where R₂ is selected from the group consisting of hydrogen, (C₁-C₄)-straight or branched chain alkyl, (C₃-C₄)-straight or branched chain alkenyl or alkynyl, and (C₁-C₄) bridging alkyl wherein a bridge is formed between the nitrogen and a carbon atom of said alkyl or alkenyl chain containing said heteroatom to form a ring, wherein said ring is optionally fused to an Ar group; and
Z is an aromatic or tertiary alkyl amine oxidized to a corresponding N-oxide, wherein the aromatic amine is Ar oxidized to a corresponding N-oxide where Ar is a mono-, bi- or tricyclic, carbo- or heterocyclic ring, wherein the ring is either unsubstituted or substituted in one to three position(s) with halo, hydroxyl, nitro, trifluoromethyl, C₁ -C₆ straight or branched chain alkyl or alkenyl, C₁ -C₄ alkoxy, C₁ -C₄ alkenyloxy, phenoxy, benzyloxy, amino, or a combination thereof; wherein the individual ring sizes are 5-6 members; wherein the heterocyclic ring contains 1-6 heteroatom(s) selected from the group consisting of O, N, S, and a combination thereof wherein at least one of the heteroatoms is N, and wherein the alkyl amine is oxidized to a corresponding N-oxide where alkyl is a C₁-C₆ straight or branched chain alkyl or alkenyl which is optionally substituted in one or more positions with C₁-C₆ straight or branched chain alkyl or alkenyl, or C₃-C₈ cycloalkyl, or C₅-C₇ cycloalkenyl, or hydroxyl, or carbonyl oxygen, or with Ar, where said alkyl, alkenyl, cycloalkyl, cycloalkenyl, or Ar group is optionally substituted with C₁ -C₄ alkyl, C₁ -C₄ alkenyl, or hydroxy, or carbonyl oxygen, or wherein any of the carbon atoms of said alkyl, alkenyl, cycloalkyl, cycloalkenyl, or Ar group is optionally replaced with O, NH, NR₁, S, SO, or SO₂; and,
R₁ is hydrogen, (C₁ -C₄)-straight or branched chain alkyl, (C₃ -C₄)-straight or branched chain alkenyl or alkynyl, or R₁ is Y-Z, as defined above; or (v)
3-(2-Pyridyl)-1-propyl(2S)-1-(3,3-Dimethyl-1,2-dioxo-pentyl)-2-pyrrolidinecarboxylate, N-oxide;
3-(3-Pyridyl)-1-propyl(2S)-1-(3,3-Dimethyl-1,2-dioxo-pentyl)-2-pyrrolidinecarboxylate, N-oxide;
3-(4-Pyridyl)-1-propyl(2S)-1-(3,3-Dimethyl-1,2-dioxo-pentyl)-2-pyrrolidinecarboxylate, N-oxide;
3-(2-Quinolyl)-1-propyl(2S)-1-(1,1-Dimethyl-1,2-dioxo-pentyl)-2-pyrrolidinecarboxylate, N-oxide;
3-(3-Quinolyl)-1-propyl(2S)-1-(1,1-Dimethyl-1,2-dioxo-pentyl)-2-pyrrolidinecarboxylate, N-oxide; and
3-(4-Quinolyl)-1-propyl(2S)-1-(1,1-Dimethyl-1,2-dioxo-pentyl)-2-pyrrolidinecarboxylate, N-oxide, or
(j) a cycloheximide derivative which is 4-[2-(3,5-Dimethylcyclohexyl 2-oxime)-2-hydroxyethyll-2,6-piperidinedione;
4-[2-(3,5-Dimethyl-2-oxocyclohexyt)-2-acetoxyethylj-2,6-piperidinedione;
4-[2-(3,5-Dimethyl-2-oxocyclohexyl)-2-(oxycarbonyl-1-adamantylamino)ethylj-2,6-piperidinedione-1-(ethyl ethanoate);
4-[2-3,5-Dimethyl-2-oxocyclohexyl)-2-hydroxyethyl]-1-methyl-2,6-piperidinedione;
4-[2-(3,5-Dimethyl-2-oxoclohexyl)-2-hydroxyethyl]-1 benzyl-2,6-piperidinedione;
4-[2-3,5-Dimethyl-2-oxocyclohexyl)-2-hydroxyethyl]-1-(4-cyanobenzyl)-2,6-piperidinedione;
4-[2-(3,5-Dimethyl-2-oxocyclohexyl)-2-hydroxyethyl]-2,6-pieridinedione-1-(acetic acid amide);
4-[2-(3,5-Dimethyl-2-oxocyclohexyl)-2-hydroxyethyl]-2,6-piperidinedione-1-(4-ethyl butanoate); or
4-[2-(3,5-Dimethyl-2-oxocyclohexyl)-2-hydroxyethyl]-2,6-piperidinedione-1-(ethyl ethanoate); or wherein
(k) the neurophilin ligand is (S)-N-benzyl-3-(4-chlorophenyl)-2-(methyl-(2-oxo-2-(3,4,5-trimethoxy-phenyl)acetyl)amino)-N-(3-pyridinyl-4-yl)-4-yl)-1-(2-pyrodinyl-4-yl)-ethyl)propyl)propionamide or 1-(3,3-dimethyl 1,2-dioxopentyl)-(L)-proline 3-(3-pyridinyl)propyl ester.

2. The use of a neurophilin ligand which does not exhibit immunosuppressant activity for the manufacture of a medicament for lowering IOP in a mammal suffering from elevated IOP, wherein the neurophilin ligand is a compound as claimed in claim 1.

3. The use of a neurophilin ligand which does not exhibit immunosuppressant activity, for the manufacture of a medicament for preventing visual field loss associated with glaucoma, wherein the neurophilin ligand is a compound as claimed in claim 1.

4. The use a neurophilin ligand which does not exhibit immunosuppressant activity, for the manufacture of a medicament for lowering IOP and providing neuroprotection in a mammal suffering from elevated IOP, wherein the neurophilin ligand is a compound as claimed in claim 1.

## Patentansprüche

1. Verwendung eines Neurophilin-Liganden der keine Immunosuppressant-Aktivität aufweist, für die Herstellung eines Arzneimittels für die Behandlung eines Säugetiers, das unter einem Glaucom leidet, wobei der Neurophilin-Ligand ist
(a) ein Pipecolsäure-Derivat bei dem es sich handelt um (E)-3-(3,4-Dichlorphenyl)-2-propenyl-1-(3,3-dimethyl-2-oxopentanoyl)-2-piperidincarboxylat; (E)-3-(3,4,5-Trimethoxyphenyl)2-propenyl-1-(3,3-dimethyl-2-oxopentanoyl)-2-piperidincarboxylat; (E)-3-Phenyl-2-propenyl-1-(3,3-dimethyl-2-oxopentanoyl)-2-piperidincarboxylat; (E)-3-((3-(2,5-Dimethoxy)-phenylpropyl)phenyl)-2-propenyl-1-(3,3-dimethyl-2-oxopentanoyl)-2-piperidincarboxylat; (E)-3-(1,3-Benzodioxol-5-yl)-2-propenyl-1-(3,3-dimethyl-2-oxopentanoyl)-2-piperidincarboxylat; 4-(4-Methoxyphenyl)butyl-1-(2-oxo-2-phenylacetyl)-2-piperidincarboxylat; 3-Phenylpropyl-1 -(2-oxo-2-phenylacetyl)-2-piperidincarboxylat; 3-(3-Pyridyl)propyl-1-(2-oxo-2 -phenylacetyl)-2-piperidincarboxylat; 3-(3-Pyridyl)propyl-1-(3,3-dimethyl-2-oxopentanoyl)-2-piperidincarboxylat; 4-Phenyl-1-(3-phenylpropyl)butyl-1-(3,3-dimethyl-2-oxopentanoyl)-2-piperidincarboxylat; 4-(4-Methoxyphenyl)butyl-(3,3-dimethyl-2-oxopentanoyl)-2-piperidincarboxylat; 1-(4-Methoxyphenethyl)-4-phenylbutyt-1-(3,3-dimethyl-2-oxopentanoyl)2-piperidincarboxylat; 3-(2,5-Dimethoxyphenyl)propyl-1-(3,3-dimethyl-2-oxopentanoyl)-2-piperidincarboxytat; 3-(1,3-Benzodioxol-5-yl)propyl-1-(3,3-dimethyl-2-oxopentanoyl)-2-pipendincarboxylat; 1-Phenethyl-3-phenylpropyl-1-(3,3-dimethyl-2-oxopentanoyl)-2-piperidincarboxylat; 4-(4-Methoxyphenyl)butyl-1-(2-cyclohexyl-2-oxoacetyl)-2-piperidincarboxylat; 3-Cyclohexytpropyl-1-(2-cyclohexyl-2-oxoacetyl-1-2-pipehdincarboxylat; 3-Phenylpropyl-1-(2-cyclohexyl-2-oxoacetyl)-2-piperid incarboxylat; 3-Cyclohexylpropyl-1-(3,3-dimethyl-2-oxobutanoyl)-2-piperidincarboxylat; 3-Phenylpropyl-1-(3,3-dimethyl-2-oxobutanoyl)-2-piperidincarboxylat; 4-(4-Methoxyphenyl)butyl-1-(3,3-dimethyl-2-oxobutanoyl)-2-piperidincarboxytat; oder 4-Phenyl-1-(3-phenylpropyl)-butyl1-(3,3-dimethyl-2-oxobutanoyl)-2-piperidincarboxylat,
(b) ein Prolin-Derivat, bei dem es sich handelt um
(i) eine Verbindung der Formel: 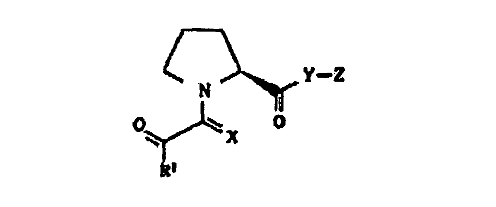 worin:
R₁ steht für eine geradkettige (unverzweigte) oder verzweigtkettige C₁-C₉-Alkyl- oder -Alkenyl-Gruppe, die gegebenenfalls substituiert ist durch C₃-C₈₋Cycloalkyl, C₃- oder C₅-Cycloalkyl, C₅-C₇-Cycloalkenyl, oder Ar₁, worin die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy und worin Ar₁ ausgewählt ist aus der Gruppe, die besteht aus 1-Naphthyl, 2-Naphthyl, 2-Indolyl, 3-Indolyl, 2-Furyl, 3-Furyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, geradkettigem (unverzweigtem) oder verzweigtkettigem C₁-C₆-Alkyl- oder -Alkenyl, C₁-C₄-Alkoxy oder C₁-C₄₋Alkenyloxy, Phenoxy, Benzyloxy und Amino;
X ausgewählt ist aus der Gruppe, die besteht aus Sauerstoff und Schwefel;
Y steht für Sauerstoff oder NR₂, worin R₂ Wasserstoff oder C₁-C₆-Alkyl darstellt; und
Z steht für ein geradkettiges (unverzweigtes) oder verzweigtkettiges C₂-C₆-Alkyl- oder -Alkenyl, worin die Alkylkette in einer oder mehreren Positionen substituiert ist durch Ar₁, wie oben definiert, C₃-C₈-Cycloalkyl, Cycloalkyl, das gebunden ist durch eine geradkettige (unverzweigte) oder verzweigtkettige C₁-C₆₋Alkyl- oder -Alkenyl-Kette, oder Ar₂, worin Ar₂ ausgewählt ist aus der Gruppe, die besteht aus 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁-C₆-Alkyl oder -Alkenyl, C₁-C₄-Alkoxy oder C₁-C₄-Alkenyloxy, Phenoxy, Benzyloxy und Amino;
wobei Z außerdem das Fragment darstellen kann: 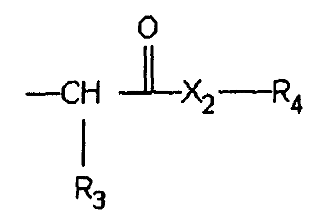
worin R₃ ausgewählt ist aus der Gruppe, die besteht aus unverzweigtem oder verzweigtem C₁-C₈-Alkyl, das gegebenenfalls substituiert ist durch C₃-C₈₋Cycloalkyl, oder Ar₁, wie oben definiert;
X₂ steht für O oder NR₅, worin R₅ ausgewählt ist aus der Gruppe, die besteht aus Wasserstoff, unverzweigtem oder verzweigtem C₁-C₆-Alkyl und -Alkenyl;
R₄ ausgewählt ist aus der Gruppe, die besteht aus Phenyl, Benzyl, unverzweigtem oder verzweigtem C₁-C₅-Alkyl oder -Alkenyl, und unverzweigtem oder verzweigtem C₁-C₅-Alkyl oder -Alkenyl, das substituiert ist durch Phenyl;
oder pharmazeutisch akzeptable Salze oder Hydrate davon;
einschließlich der Folgenden Verbindungen:
3-Phenyl-1-propyl-(2S)-1-(3,3-dimethyl-1,2-dioxopentyl)2-pyrrolidincarboxylat,
3-Phenyl-1-prop-2-(E)-enyl6(2S)-1-(3,3-dimethyl-1,2-dioxopentyl)2-pyrrolidincarboxylat,
3-(3,4,5-Trimethoxyphenyl)-1-propyl-(2S)-1-(3,3-dimethyl-1,2-dioxopentyl)-2-pyrrolidincarboxylat,
3-(3,4,5-Trimethoxyphenyl)-1-prop-2-(E)-enyl-(2S)1-(3,3-dimethyl-1,2-dioxopentyl)2pyrrolidincarboxylat,
3-(4,5-Dichlorphenyl)-1-propyl-(2S)-1-(3,3-dimethyl-1,2-dioxopentyl)-2-pyrrolidincarboxylat,
3-(4,5-Dichlorphenyl)-1-prop-2-(E)-enyl (2S)-1-(3,3-dimethyl-1,2-dioxopentyl)-2-pyrrolidincarboxylat,
3-(4,5-Methylendioxyphenyl)-1-propyl-(2S)-1-(3,3-dimethyl-1,2-dioxopentyl)-2-pyrrolidincarboxylat,
3-(4,5-Methylendioxyphenyl)-1-prop-2-(E)-enyl-(2S)-1-(3,3-dimethyl-1,2-dioxopentyl)-2-pyrrolidincarboxylat,
3-Cyclohexyl-1-propyl-(2S)-1-(3,3-dimethyl-1,2-dioxopentyl)-2-pyrrolidincarboxylat,
3-Cyclohexyl-1-prop-2-(E)-enyl-(2S)-1-(3,3-dimethyl-1,2-dioxopentyl)-2-pyrrolidincarboxylat,
(1 R)-1,3-Diphenyl-1-propyl-(2S)-1-(3,3-dimethyl-1,2-dioxopentyl)-2-pyrrolidincarboxylat,
(1 R)-1,3-Diphenyl-1-prop-2r(E)-enyl-(2S)-1-(3,3-dimethyl-1,2-dioxopentyl)-2-pyrrolidincarboxylat,
(1R)-1-Cyclohexyl-3-phenyl-1-propyl-(2S)-1-(3,3-dimethyl-1,2-dioxopentyl)-2-pyrrolidincarboxylat,
(1R)-1-Cyclohexyl-3-phenyl-1-prop-2-(E)enyl-(2S)-1-(3,3-dimethyl-1,2-dioxopentyl)-2-pyrrolidincarboxylat,
(1R)-1-(4,5-Dichlorphenyl)-3-phenyl-1-propyl-(2S)-1-(3,3-dimethyl-1,2-dioxopentyl)-2-pyrrolidincarboxylat,
3-Phenyl-1-propyl-(2S)-1-(1,2-dioxo-2-cyclohexyl)ethyl-2-pyrrolidincarboxylat,
3-Phenyl-1-propyl-(2S)-1-(1,2-dioxo-4-cyclohexyl)butyl-2-pyrrolidincarboxylat,
3-Phenyl-1-propyl-(2S)-1-(1,2-dioxo-2-[2-furanyl])ethyl-2-pyrrolidincarboxylat,
3-Phenyl-1-propyl-(2S)-1-(1,2-dioxo-2-[2-thienyl])ethyl-2-pyrrolidincarboxylat,
3-Phenyl-1-propyl-(2S)-1-(1,2-dioxo-2-[2-thiazolyl])ethyl-2-pyrrolidincarboxylat,
3-Phenyl-1-propyl-(2S)-1-(1,2-dioxo-2-phenyl)ethyl-2-pyrrolidincarboxylat,
1, 7-Diphenyl-4-heptyl-(2S)-1-(3,3-dimethyl-1,2-dioxopentyl)-2-pyrrolidincarboxylat,
3-Phenyl-1-propyl-(2S)-1-(3,3-dimethyl-1,2-dioxo-4-hydroxybutyl)-2-pyrrolidincarboxylat,
3-Phenyl-1-propyl-(2S)-1-(3,3-dimethyl-1,2-dioxopentyl)-2-pyrrolidincarboxamid,
[1-(3,3-Dimethyl-1,2-dioxopentyl)-L-prolin]-L-phenylalanin-ethylester,
1-[1-(3,3-Dimethyl-1,2-dioxopentyl)-L-prolin]-L-leucin-ethylester,
1-[1-(3,3-Dimethyl-1,2-dioxopentyl)-L-prolin]-L-phenylglycin-ethylester,
1-[1 -(3,3-Dimethyl-1,2-dioxopentyl)-L-prolin]-L-phenylalanin-phenylester,
1-[1-(3,3-Dimethyl-1,2-dioxopentyl)-L-prolin]-L-phenylalanin-benzylester und
1-[1-(3,3-Dimethyl-1,2-dioxopentyl)-L-prolin]-L-isoleucin-ethylester, oder
(ii) eine Verbindung der Formel: 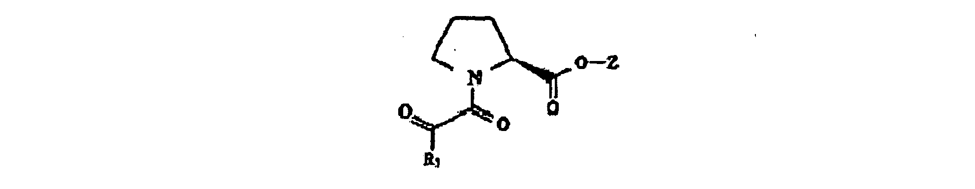 worin:
R₁ steht für eine geradkettige (unverzweigte) oder verzweigtkettige C₁-C₉-Alkyl-Gruppe oder -Alkenyl-Gruppe, die gegebenenfalls substituiert ist durch C₃-C₈₋Cycloalkyl, C₃- oder C₅-Cycloalkyl, C₅-C₇-Cycloalkenyl, oder Ar₁, worin die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy, und worin Ar₁ ausgewählt ist aus der Gruppe, die besteht aus 1-Naphthyl, 2-Naphthyl, 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁₋C₆-Alkyl oder -Alkenyl, C₁-C₄-Alkoxy oder C₁-C₄-Alkenyloxy, Phenoxy, Benzyloxy und Amino;
Z steht für geradkettiges (unverzweigtes) oder verzweigtkettiges C₂-C₆-Alkyl oder -Alkenyl, worin die Alkylkette substituiert ist in einer oder mehreren Positionen durch Ar₁, wie oben definiert, C₁-C₈-Cycloalkyl, Cycloalkyl, das gebunden ist durch eine gerade oder unverzweigte C₁-C₆-Alkyl- oder -Alkenyl-Kette, oder Ar₂, das ausgewählt ist aus der Gruppe, die besteht aus 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁-C₆-Alkyl oder -Alkenyl, C₁-C₄-Alkoxy oder C₁-C₄-Alkenyloxy, Phenoxy, Benzyloxy und Amino;
oder pharmazeutisch akzeptable Salze oder Hydrate davon; oder
(iii) eine Verbindung der Formel: 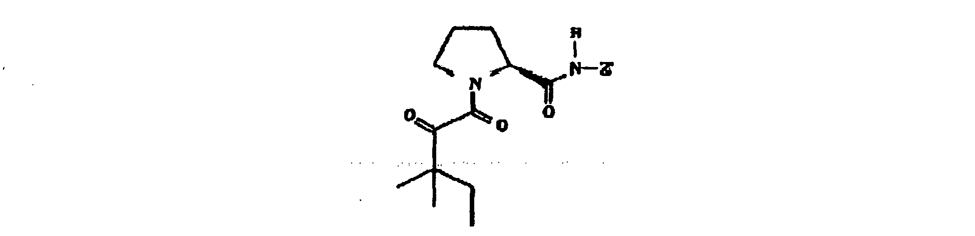 worin Z' für das Fragment steht: 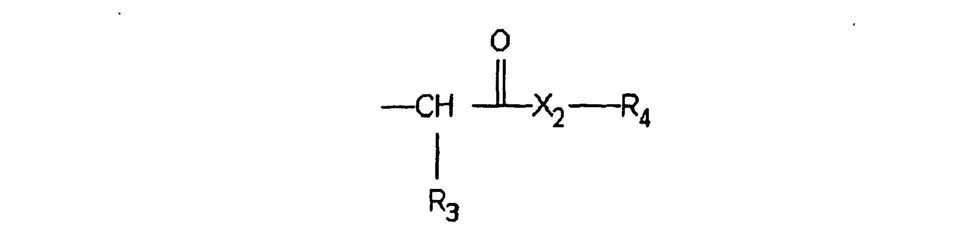 worin:
R₃ ausgewählt ist aus der Gruppe, die besteht aus unverzweigtem oder verzweigtem C₁-C₈-Alkyl, das gegebenenfalls substituiert ist durch C₃-C₈-Cycloalkyl, oder Ar₁, wie oben definiert, und unsubstituiertem Ar₁;
X₂ steht für O oder NR₅, worin R₅ ausgewählt ist aus der Gruppe, die besteht aus Wasserstoff, unverzweigtem oder verzweigtem C₁-C₆-Alkyl und -Alkenyl;
R₄ ausgewählt ist aus der Gruppe, die besteht aus Phenyl, Benzyl, unverzweigtem oder verzweigtem C₁-C₅-Alkyl oder -Alkenyl und unverzweigtem oder verzweigtem C₁-C₅-Alkyl oder -Alkenyl, das substituiert ist durch Phenyl;
oder pharmazeutisch akzeptable Salze oder Hydrate davon,
(c) ein N-Sulfonylpipecolyl- oder Prolyl-Derivat, bei dem es sich handelt um
(i) 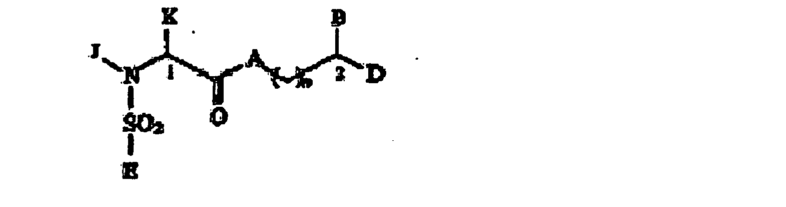 oder ein pharmazeutisch akzeptables Salz davon, worin:
A steht für CH₂, Sauerstoff, NH oder N-(C₁-C₄-Alkyl);
B und D stehen unabhängig voneinander für Ar, Wasserstoff, unverzweigtes oder verzweigtes (C₁-C₆)-Alkyl, unverzweigtes oder verzweigtes (C₁-C₆)-Alkenyl, unverzweigtes oder verzweigtes (C₁-C₆)-Alkyl oder -Alkenyl, das substituiert ist durch ein (C₅-C₇)-Cycloalkyl, unverzweigtes oder verzweigtes (C₁-C₆)-Alkyl oder -Alkenyl, das substituiert ist durch ein (C₅-C₇)-Cycloalkenyl, oder Ar, das substituiert ist durch unverzweigtes oder verzweigtes (C₁-C₆)-Alkyl oder -Alkenyl, wobei in jedem Falle eine oder zwei der CH₂-Gruppen der Alkyl- oder Alkenyl-Ketten 1 bis 2 Heteroatome enthalten können, ausgewählt aus der Gruppe, die besteht aus Sauerstoff, Schwefel, SO und SO₂ in chemisch vernünftigen Substitutions-Mustem, oder 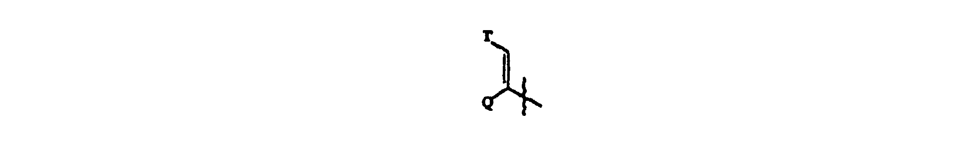 mit der Maßgabe, dass B und D nicht beide Wasserstoff darstellen;
Q steht für Wasserstoff, unverzweigtes oder verzweigtes (C₁-C₆)-Alkyl oder unverzweigtes oder verzweigtes (C₁-C₆)-Alkenyl;
T steht für Ar oder ein substituiertes 5- bis 7-gliedriges Cycloalkyl, mit Substituenten in den Positionen 3 und 4, die unabhängig voneinander ausgewählt sind aus der Gruppe, die besteht aus Wasserstoff, Hydroxyl, O-(C₁-C₄)-Alkyl, O-(C₁-C₄)-Alkenyl und Carbonyl;
Ar ausgewählt ist aus der Gruppe, die besteht aus Phenyl, 1-Naphthyl, 2-Naphthyl, 2-Furyl, 3-Furyl, 2-Thienyl, 3-Thienyl, 2-Pyridyl, 3-Pyridyl, 4-Pyridyl, monocyclischen und bicyclischen heterocyclischen Ringsystemen, die einzelne 5- oder 6-gliedrige Ringe aufweisen, die in einem oder beiden Ringen insgesamt 1 bis 4 Heteroatome enthalten können, die unabhängig voneinander ausgewählt sind aus O, N und S; worin Ar 1 bis 3 Substituenten enthalten kann, die unabhängig voneinander ausgewählt sind aus der Gruppe, die besteht aus Wasserstoff, Halogen, Hydroxyl, Nitro, Trifluormethyl, Trifluormethoxy, unverzweigtem oder verzweigtem (C₁-C₆)-Alkyl, unverzweigtem oder verzweigtem (C₂-C₆)-Alkenyl, O-unverzweigtem oder verzweigtem (C₁-C₄)-Alkyl, O-unverzweigtem oder verzweigtem (C₂-C₄)-Alkenyl, O-Benzyl, O-Phenyl, 1,2-Methylendioxy, Amino, Carboxyl und Phenyl;
E steht für unverzweigtes oder verzweigtes (C₁-C₆)-Alkyl, unverzweigtes oder verzweigtes (C₁-C₆)-Alkenyl, (C₅-C₇)-Cycloalkyl, (C₅-C₇)-Cycloalkenyl, das substituiert ist durch unverzweigtes oder verzweigtes (C₁-C₄)-Alkyl oder unverzweigtes oder verzweigtes (C₁-C₄)-Alkenyl, [(C₂-C₄)-Alkyl oder (C₂-C₄)-Alkenyl)]-Ar oder Ar;
J steht für Wasserstoff oder C₁- oder C₂-Alkyl oder Benzyl;
K steht für unverzweigtes oder verzweigtes (C₁-C₄)-Alkyl, Benzyl oder Cyclohexylmethyl; oder worin J und K gemeinsam einen 5- bis 7-gliedrigen heterocyclischen Ring bilden können, der einen Sauerstoff-, Schwefel-, SO- oder SO₂-Substituenten darin enthalten kann;
n steht für eine Zahl von 0 bis 3; und
worin die Stereochemie an den Kohlenstoffatom-Positionen 1 und 2 R oder S ist; oder 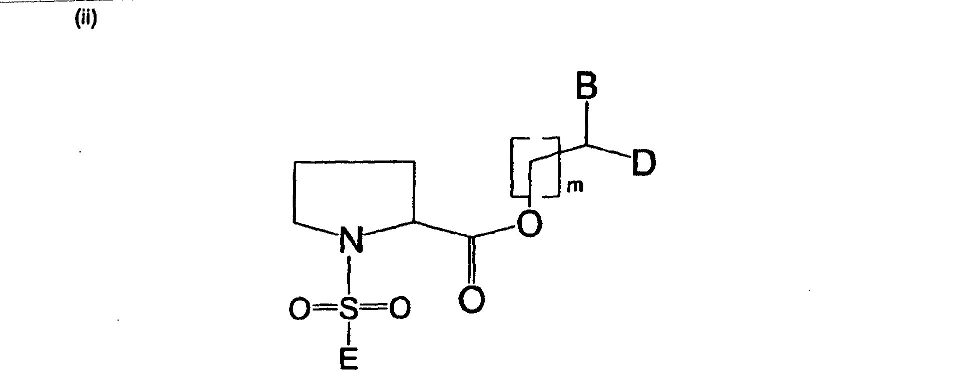 oder ein pharmazeutisch akzeptables Salz davon, worin:
B und D D unabhängig voneinander stehen für Ar, Wasserstoff, unverzweigtes oder verzweigtes (C₁-C₆)-Alkyl, unverzweigtes oder verzweigtes (C₁-C₆)-Alkenyl, unverzweigtes oder verzweigtes (C₁-C₆)-Alkyl oder -Alkenyl, das substituiert ist durch ein (C₅-C₇)-Cycloalkyl, unverzweigtes oder verzweigtes (C₁-C₆)-Alkyl oder -Alkenyl, das substituiert ist durch ein (C₅-C₇)-Cycloalkenyl, oder Ar, das substituiert ist durch unverzweigtes oder verzweigtes (C₁-C₆)-Alkyl oder -Alkenyl, wobei in jedem Fall eine oder zwei der CH₂-Gruppen der Alkyl- oder Alkenylketten 1 bis 2 Heteroatome enthalten können, ausgewählt aus der Gruppe, die besteht aus Sauerstoff, Schwefel, SO und SO₂ in chemisch vemünftigen Substitutions-Mustem; oder 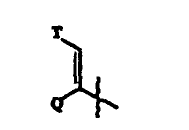 mit der Maßgabe, dass B und D nicht beide Wasserstoff sein können;
Q steht für Wasserstoff, unverzweigtes oder verzweigtes (C₁-C₆)-Alkyl oder unverzweigtes oder verzweigtes (C₁-C₆)-Alkenyl;
T steht für Ar oder substituiertes 5- bis 7-gliedriges Cycloalkyl mit Substituenten in den Positionen 3 und 4, die unabhängig voneinander ausgewählt sind aus der Gruppe, die besteht aus Wasserstoff, Hydroxyl, O-(C₁-C₄)-Alkyl, O-(C₁₋C₄)-Alkenyl und Carbonyl;
Ar ausgewählt ist aus der Gruppe, die besteht aus Phenyl, 1-Naphthyl, 2-Naphthyl, 2-Furyl, 3-Furyl, 2-Thienyl, 3-Thienyl, 2-Pyridyl, 3-Pyridyl, 4-Pyridyl, monocyclischen und bicyclischen heterocyclischen Ringsystemen mit einzelnen 5- oder 6-gliedrigen Ringen, die in einem oder in beiden Ringen insgesamt 1 bis 4 Heteroatome enthalten können, unabhängig voneinander ausgewählt aus O, N und S; worin Ar 1 bis 3 Substituenten enthalten kann, die unabhängig voneinander ausgewählt sind aus der Gruppe, die besteht aus Wasserstoff, Halogen, Hydroxyl, Nitro, Trifluormethyl, Trifluormethoxy, unverzweigtem oder verzweigtem (C₁-C₆)-Alkyl, unverzweigtem oder verzweigtem (C₂₋C₆)-Alkenyl, O-unverzweigtem oder verzweigtem (C₁-C₄)-Alkyl, O-unverzweigtem oder verzweigtem (C₂-C₄)-Alkenyl, O-Benzyl, O-Phenyl, 1,2-Methylendioxy, Amino, Carboxyl und Phenyl;
E steht für unverzweigtes oder verzweigtes (C₁-C₆)-Alkyl, unverzweigtes oder verzweigtes (C₁-C₆)-Alkenyl, (C₅-C₇)-Cycloalkyl, (C₅-C₇)-Cycloalkenyl, das substituiert ist durch unverzweigtes oder verzweigtes (C₁-C₄)-Alkyl oder unverzweigtes oder verzweigtes (C₁-C₄)-Alkenyl, [(C₂-C₄)-Alkyl oder (C₂-C₄)-Alkenyl)]-Ar oder Ar; und
m steht für eine Zahl von 0 bis 3; oder 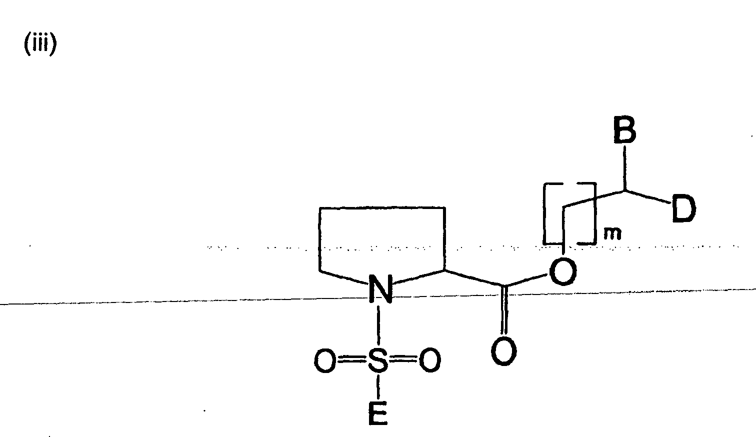 oder ein pharmazeutisch akzeptables Salz davon, worin:
B und D unabhängig voneinander stehen für Ar, Wasserstoff, unverzweigtes oder verzweigtes (C₁-C₆)-Alkyl, unverzweigtes oder verzweigtes (C₁-C₆)-Alkenyl, unverzweigtes oder verzweigtes (C₁-C₆)-Alkyl oder -Alkenyl, das substituiert ist durch ein (C₅-C₇)-Cycloalkyl, unverzweigtes oder verzweigtes (C₁-C₆)-Alkyl oder -Alkenyl, das substituiert ist durch ein (C₅-C₇)-Cycloalkenyl, oder Ar, das substituiert ist durch unverzweigtes oder verzweigtes (C₁-C₆)-Alkyl oder Alkenyl, wobei in jedem Fall eine oder zwei der CH₂-Gruppen der Alkyl- oder-Alkenyl-Ketten 1 bis 2 Heteroatome enthalten können, ausgewählt aus der Gruppe, die besteht aus Sauerstoff, Schwefel, SO und SO₂ in chemisch vernünftigen Substitutionsmustem, oder 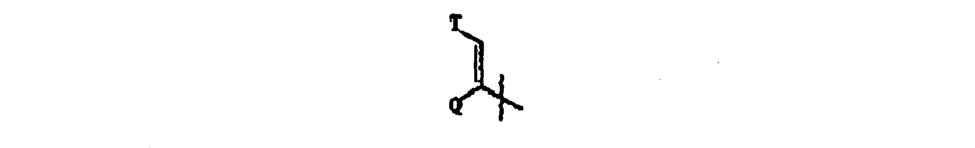 mit der Maßgabe, dass B und D nicht beide für Wasserstoff stehen können;
Q steht für Wasserstoff, unverzweigtes oder verzweigtes (C₁-C₆)-Alkyl oder unverzweigtes oder verzweigtes (C₁-C₆)-Alkenyl;
T steht für Ar oder substituiertes 5- bis 7-gliedriges Cycloalkyl mit Substituenten in den Positionen 3 und 4, die unabhängig voneinander ausgewählt sind aus der Gruppe, die besteht aus Wasserstoff, Hydroxyl, O-(C₁-C₄)-Alkyl, O-(C₁₋C₄)-Alkenyl und Carbonyl;
Ar ausgewählt ist aus der Gruppe, die besteht aus Phenyl, 1-Naphthyl, 2-Naphthyl, 2-Furyl, 3-Furyl, 2-Thienyl, 3-Thienyl, 2-Pyridyl, 3-Pyridyl, 4-Pyridyl, monocyclischen und bicyclischen heterocyclischen Ringsystemen mit einzelnen 5- oder 6-gliedrigen Ringen, die in einem oder in beiden Ringen insgesamt 1 bis 4 Heteroatome enthalten können, die unabhängig voneinander ausgewählt sind aus der Gruppe, die besteht aus O, N und S; worin Ar 1 bis 3 Substituenten enthalten kann, die unabhängig voneinander ausgewählt sind aus der Gruppe, die besteht aus Wasserstoff, Halogen, Hydroxyl, Nitro, Trifluormethyl, Trifluormethoxy, unverzweigtem oder verzweigtem (C₁-C₆)-Alkyl, unverzweigtem oder verzweigtem (C₂-C₆)-Alkenyl, O-unverzweigtes oder verzweigtem (C₁-C₄)-Alkyl, O-unverzweigtem oder verzweigtem (C₂-C₄)-Alkenyl, O-Benzyl, O-Phenyl, 1,2-Methylendioxy, Amino, Carboxyl und Phenyl;
E steht für unverzweigtes oder verzweigtes (C₁-C₆)-Alkyl, unverzweigtes oder verzweigtes (C₁-C₆)-Alkenyl, (C₅-C₇)-Cycloalkyl, (C₅-C₇)-Cycloalkenyl, das substituiert ist durch unverzweigtes oder verzweigtes (C₁-C₄)-Alkyl oder unverzweigtes oder verzweigtes (C₁-C₄)-Alkenyl, [(C₂-C₄)-Alkyl oder (C₂-C₄)-Alkenyl)]-Ar oder Ar; und
m steht für eine Zahl von 0 bis 3,
(d) ein heterocyclischer Thioester oder Keton, bei dem es sich handelt um 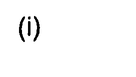 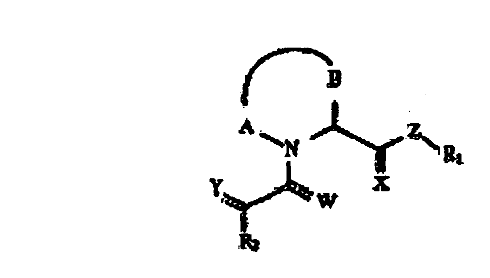 oder ein pharmazeutisch akzeptables Salz davon, worin:
A und B zusammen mit den Stickstoff- und den Kohlenstoffatomen, an die sie jeweils gebunden sind, einen 5- bis 7-gliedrigen gesättigten oder ungesättigten heterocyclischen Ring bilden, der eine beliebige Kombination von CH₂, O, S, SO, SO₂, NH oder NR₂ in einem beliebigen chemisch stabilen Oxidationszustand enthält;
X steht für O oder S;
Z steht für S, CH₂, CHR₁ oder C(R₁)₂;
W und Y unabhängig voneinander stehen für O, S, CH₂ oder H₂;
R₁ steht für ein geradkettiges (unverzweigtes) oder verzweigtkettiges C₁-C₆-Alkyl oder -Alkenyl, das in einer oder mehreren Positionen substituiert ist durch (Ar₁)ₙ, (Ar₁)ₙ, das gebunden ist durch ein geradkettiges (unverzweigtes) oder verzweigtkettiges C₁-C₆-Alkyl oder -Alkenyl, C₃-C₈-Cycloalkyl, C₃-C₈₋Cycloalkyl, das durch ein geradkettiges (unverzweigtes) oder verzweigtkettiges C₁-C₆-Alkyl oder -Alkenyl gebunden ist, Ar₂, oder eine Kombination davon;
n steht für die Zahl 1 oder 2;
R₂ steht entweder für geradkettiges (unverzweigtes) oder verzweigtkettiges C₁₋C₉-Alkyl oder -Alkenyl, C₃-C₈-Cycloalkyl, C₅-C₇-Cycloalkenyl oder für Ar₁, wobei das Alkyl, das Alkenyl, das Cycloalkyl oder das Cycloalkenyl entweder unsubstituiert ist oder substituiert ist in einer oder mehreren Positionen durch geradkettiges (unverzweigtes) oder verzweigtkettiges C₁-C₄-Alkyl oder -Alkenyl, Hydroxyl oder eine Kombination davon; und
Ar₁ und Ar₂ unabhängig voneinander stehen für einen mono-, bi- oder tricyclischen, carbocyclischen oder heterocyclischen Ring, wobei der Ring entweder unsubstituiert oder substituiert ist in 1 bis 3 Positionen durch Halogen, Hydroxyl, Nitro, Trifluormethyl, geradkettiges (unverzweigtes) oder verzweigtkettiges C₁₋C₆-Alkyl oder -Alkenyl, C₁-C₄-Alkoxy, C₁-C₄-Alkenyloxy, Phenoxy, Benzyloxy, Amino oder eine Kombination davon;
wobei die einzelnen Ringe 5- bis 6-gliedrige Ringe sind; und wobei der heterocyclische Ring 1 bis 6 Heteroatome enthält, ausgewählt aus der Gruppe, die besteht aus O, N, S, und eine Kombination davon; oder 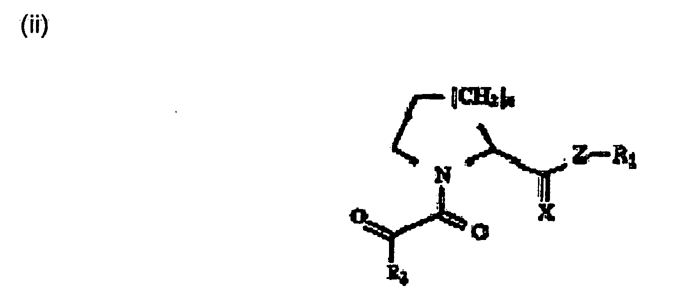 oder ein pharmazeutisch akzeptables Salz davon, worin:
n steht für die Zahl 1 oder 2;
X steht für O oder S;
Z steht für S, CH₂, CHR₁ oder C(R₁)₂;
R₁ steht entweder für ein geradkettiges (unverzweigtes) oder verzweigtkettiges C₁-C₅-Alkyl oder -Alkenyl, das in einer oder mehreren Positionen substituiert ist durch (Ar₁)ₙ, (Ar₁)ₙ, das durch ein geradkettiges (unverzweigtes) oder verzweigtkettiges C₁-C₅-Alkyl- oder -Alkenyl gebunden ist, oder eine Kombination davon;
R₂ steht entweder für ein geradkettiges (unverzweigtes) oder verzweigtkettiges C₁-C₉-Alkyl oder -Alkenyl, C₃-C₈-Cycloalkyl, C₅-C₇-Cycloalkenyl oder für (Ar₁)ₙ; und
Ar₁ steht für einen mono- oder bi- oder tricyctischen, carbocyclischen oder heterocyclischen Ring, der entweder unsubstituiert ist oder substituiert ist in 1 bis 3 Positionen durch Halogen, Hydroxyl, Nitro, Trifluormethyl, geradkettiges (un-verzweigtes) oder verzweigtkettiges C₁-C₆-Alkyl oder -Alkenyl, C₁-C₄-Alkoxy, C₁-C₄-Alkenyloxy, Phenoxy, Benzyloxy, Amino oder eine Kombination davon; wobei die einzelnen Ringe 5- bis 6-Ringatome aufweisen; und der heterocyclische Ring 1 bis 6 Heteroatome enthält, ausgewählt aus der Gruppe, die besteht aus O, N, S, und eine Kombination davon; oder 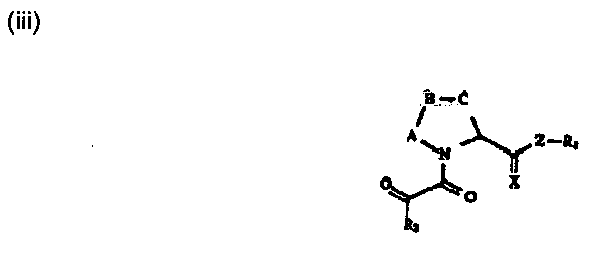
oder ein pharmazeutisch akzeptables Salz davon, worin:
A, B, C und D unabhängig voneinander stehen für CH₂, O, S, SO, SO₂, NH oder NR₂;
X steht für O oder S;
Z steht für S, CH₂, CHR₁ oder C(R₁)₂;
R₁ steht für geradkettiges (unverzweigtes) oder verzweigtkettiges C₁-C₆-Alkyl oder -Alkenyl, das in einer oder mehreren Positionen substituiert ist durch (Ar₁)ₙ, (Ar₁)ₙ, das gebunden ist durch ein geradkettiges (unverzweigtes) oder verzweigtkettiges C₁-C₆-Alkyl oder -Alkenyl, C₃-C₈-Cycloalkyl, C₃-C₈₋Cycloalkyl, das gebunden ist durch ein geradkettiges (unverzweigtes) oder verzweigtkettiges C₁-C₆-Alkyl oder -Alkenyl, Ar₂ oder eine Kombination davon;
n steht für die Zahl 1 oder 2;
R₂ steht entweder für geradkettiges (unverzweigtes) oder verzweigtkettiges C₁-C₉₋Alkyl oder -Alkenyl, C₃-C₈-Cycloalkyl, C₅-C₇-Cycloalkenyl oder Ar₁, worin das Alkyl, Alkenyl, Cycloalkyl oder Cycloalkenyl entweder unsubstituiert ist oder substituiert ist in einer oder mehreren Positionen durch geradkettiges (unverzweigtes) oder verzweigtkettiges C₁-C₄-Alkyl oder -Alkenyl, Hydroxyl oder eine Kombination davon; und
Ar₁ und Ar₂ unabhängig voneinander stehen für einen mono-, bi- oder tricyclischen, carbocyclischen oder heterocyclischen Ring, wobei der Ring entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁₋C₆-Alkyl oder -Alkenyl, C₁-C₄-Alkoxy, C₁-C₄-Alkenyloxy, Phenoxy, Benzyloxy, Amino oder eine Kombination davon;
wobei die einzelnen Ringe 5- bis 6-gliedrige Ringe sind; und der heterocyclische Ring 1 bis 6 Heteroatome enthält, ausgewählt aus der Gruppe, die besteht aus O, N, S, und eine Kombination davon; oder 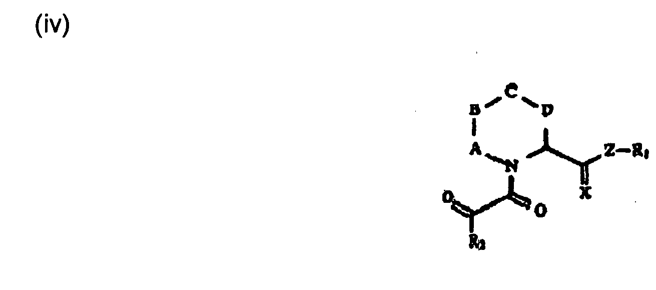 oder ein pharmazeutisch akzeptables Salz davon, worin:
A, B, C und D unabhängig voneinander stehen für CH₂, O, S, SO, SO₂, NH oder NR₂;
X steht für O oder S;
Z steht für S, CH₂, CHR₁ oder C(R₁)₂;
R₁ steht für geradkettiges (unverzweigtes) oder verzweigtkettiges C₁-C₆-Alkyl oder -Alkenyl, das substituiert ist in einer oder mehreren Positionen durch (Ar₁)ₙ, (Ar₁)ₙ, das gebunden ist durch ein geradkettiges (unverzweigtes) oder verzweigtkettiges C₁-C₆-Alkyl oder -Alkenyl, C₃-C₈-Cycloalkyl, C₃-C₈₋Cycloalkyl, das gebunden ist durch ein geradkettiges (unverzweigtes) oder verzweigtkettiges C₁-C₆-Alkyl oder -Alkenyl, Ar₂ oder eine Kombination davon;
n steht für die Zahl 1 oder 2;
R₂ steht entweder für geradkettiges (unverzweigtes) oder verzweigtkettiges C₁₋C₉-Alkyl oder -Alkenyl, C₃-C₈-Cycloalkyl, C₅-C₇-Cycloalkenyl oder für Ar₁ wobei das Alkyl, das Alkenyl, das Cycloalkyl oder das Cycloalkenyl entweder unsubstituiert ist oder substituiert ist in einer oder mehreren Positionen durch geradkettiges (unverzweigtes) oder verzweigtkettiges C₁-C₄-Alkyl oder -Alkenyl, Hydroxyl, oder eine Kombination davon; und
Ar₁ und Ar₂ unabhängig voneinander stehen für einen mono-, bi- oder tricyclischen, carbocyclischen oder heterocyclischen Ring, wobei der Ring entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁₋C₆-Alkyl oder -Alkenyl, C₁-C₄-Alkoxy, C₁-C₄-Alkenyloxy, Phenoxy, Benzyloxy, Amino oder eine Kombination davon;
wobei die einzelnen Ringe 5- bis 6-gliedrige Ringe sind; und der heterocyclische Ring 1 bis 6 Heteroatome enthält, ausgewählt aus der Gruppe, die besteht aus O, N, S, und eine Kombination davon; oder
(v)
(2S)-2-(1 -Oxo-4-phenyl)butyl-1 -(3,3-dimethyl-1,2-dioxopentyl)pyrrolidin;
(2S)-2-(1 -Oxo-4-(3-pyridyl)butyl-1 -(3,3-dimethy)-1,2-dioxopentyl)pyrrolidin;
(2S)-2-(1 -Oxo-4-phenyl)butyl-1 -(3,3-dimethyl-1,2-dioxobutyl)pyrrolidin;
(2S)-2-(1-Oxo-4-(3-pyridyt)butyl-1-(3,3-dimethyl-1,2-dioxobutyl)pyrrolidin;
(2S)-2-(1-Oxo-4(3-pyridyl)butyl-1-(2-cyclohexyl=1,2-dioxoethyl)pyrrolidin;
(2S)-2-(1-Oxo-4-(3-pyridyl)butyl-1-(2-cyclopentyl-1,2-dioxoethyl)pyrrolidin;
(2S)-2-(1-Oxo-4-(3-pyridyl)butyl-1-(2-cyclopenty)-1,2-dioxoethyl)pyrrolidin;
(2S)-2-(1-Oxo-3-(9-fluorenyl)propyl-1-(3,3-dimethyl-1,2-dioxopentyl)pyrrolidin;
**2-Phenyl-1 -ethylsulfhydryl-(2S)-1 -(3,3-dimethyl-1,2-dioxopentyl)-2-pyrrolidin**carboxylat;
(3-Thioindolyl)methylsulfhydryl(2S)-1-(3,3-dimethyl-1,2-dioxopentyl)-2-pyrrolidincarboxylat;
2-Phenyl-1-ethylsulfhydryl-(2S)-1-(2-cyclohexyl-1,2-dioxopentyl)-2-pyrrolidincarboxylat;
(2S)-2-(1-Oxo-4-(p-methoxyphenyl)butyl-1-(3,3-dimethyl-1,2dioxopentyl)pyrrolidin;
2-Phenyl-1-ethylsulfhydryl-(2R,S)-1-(3,3-dimethyl-1,2-dioxopentyl)-2-piperidincarboxylat;
2-Phenyl-1-ethylsulfhydryl-(2R,S)-1-(3,3-dimethyl-1,2-dioxobutyl)-2-piperidincarboxylat;
2-Phenyl-1-ethylsulfhydryl-(2R,S)-1-(2-phenyl-1,2-dioxoethyl)-2-piperidincarboxylat;
2-(1-Oxo-5-(p-methoxyphenyl)pentyl-1-(3,3-dimethyl-1,2-dioxopentyl)piperidin;
2-(1-Oxo-5-phenyl)pentyl-1-(3,3-dimethyl-1,2-d ioxopentyi)piperidin;
2-(1-Oxo-5-phenyl)pentyl-1-(2-phenyl-1,2-dioxopentyl)piperidin; und
2-(1-Oxo-5-phenyl)pentyl-1-(2-cyclohexyl-1,2-dioxopentyl)piperidin; oder
(vi)
2-Phenyl-1-ethylsulfhydryl(2S)-1-(3,3-dimethyl-1,2-dioxopentyl)-2-pyrrolidincarboxylat;
(3-Thioindolyl)methylsulfhydryl(2S)-1-(3,3-dimethyl-1,2-dioxopentyl)-2-pyrrolidincarboxylat; oder
2-Phenyl-1-ethylsulfhydryl(2S)-1-(2-cyclohexyl-1,2-dioxopentyl)-2-pyrrolidincarboxylat,
(e) ein N-Glyoxyl-prolylester-Derivat, bei dem es sich handelt um
(i) eine Verbindung der Formel: 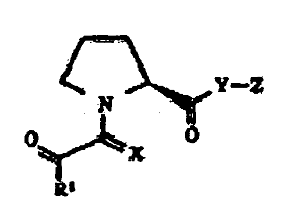 worin:
R₁ steht für eine geradkettige (unverzweigte) oder verzweigtkettige C₁-C₉-Alkyl-Gruppe oder -Alkenyl-Gruppe, die gegebenenfalls substituiert ist durch C₃-C₈₋Cycloalkyl, C₃- oder C₅-Cycloalkyl, C₅-C₇-Cycloalkenyl oder Ar₁, wobei die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy und Ar₁ ausgewählt ist aus der Gruppe, die besteht aus 1-Naphthyl, 2-Naphthyl, 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁-C₆₋Alkyl oder -Alkenyl, C₁-C₄-Alkoxy oder C₁-C₄-Alkenyloxy, Phenoxy, Benzyloxy und Amino;
X steht für Sauerstoff; Schwefel, Methylen (CH₂) oder H₂;
Y steht für Sauerstoff oder NR₂, worin R₂ Wasserstoff oder C₁-C₆-Alkyl darstellt; und
Z steht für ein geradkettiges (unverzweigtes) oder verzweigtkettiges C₂-C₆-Alkyl oder -Alkenyl, worin die Alkylkette substituiert ist in einer oder mehreren Positionen durch Ar₁ wie oben definiert, C₃-C₈-Cycloalkyl, Cycloalkyl, das gebunden ist durch eine geradkettige oder unverzweigte C₁-C₆-Alkylkette oder - Alkenylkette, oder Ar₂, worin Ar₂ ausgewählt ist aus der Gruppe, die besteht aus 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, geradkettigem (unverzweigtem) oder verzweigtkettigem C₁-C₆-Alkyl oder -Alkenyl, C₁-C₄-Alkoxy oder C₁₋C₄-Alkenyloxy, Phenoxy, Benzyloxy und Amino;
wobei Z außerdem das Fragment sein kann: 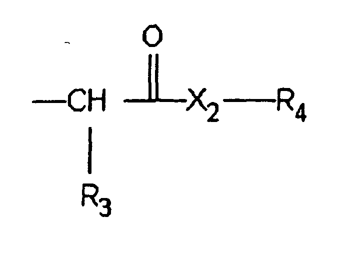 worin:
R₃ ausgewählt ist aus der Gruppe, die besteht aus unverzweigtem oder verzweigtem C₁-C₈-Alkyl, das gegebenenfalls substituiert ist durch C₃-C₈-Cycloalkyl, oder Ar₁, wie oben definiert, und unsubstituiertes Ar₁
X₂ steht für O oder NR₅, worin R₅ ausgewählt ist aus der Gruppe, die besteht aus Wasserstoff, unverzweigtem oder verzweigtem C₁-C₆-Alkyl und -Alkenyl;
R₄ ausgewählt ist aus der Gruppe, die besteht aus Phenyl, Benzyl, unverzweigtem oder verzweigtem C₁-C₅-Alkyl oder -Alkenyl, und unverzweigtem oder verzweigtem C₁-C₅-Alkyl oder -Alkenyl, das substituiert ist durch Phenyl;
oder pharmazeutisch akzeptable Salze oder Hydrate davon;
(ii) eine Verbindung der Formel: 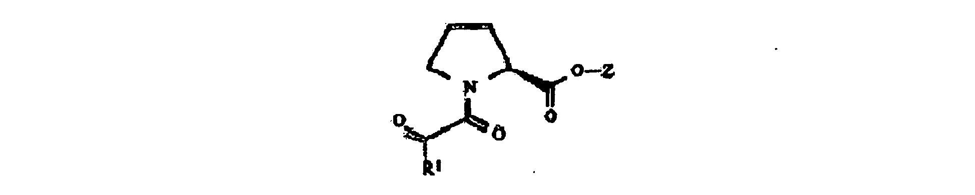 worin:
R₁ steht für eine geradkettige (unverzweigte) oder verzweigtkettige C₁-C₉-Alkyl-Gruppe oder -Alkenyl-Gruppe, die gegebenenfalls substituiert ist durch C₃-C₆₋Cycloalkyl, C₃- oder C₅-Cycloalkyl, C₅-C₇-Cycloalkenyl oder Ar₁, worin die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy und worin Ar₁ ausgewählt ist aus der Gruppe, die besteht aus 1-Naphthyl, 2-Naphthyl, 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁₋C₆-Alkyl oder -Alkenyl, C₁-C₄-Alkoxy oder C₁-C₄-Alkenyloxy, Phenoxy, Benzyloxy und Amino;
Z steht für ein geradkettiges (unverzweigtes) oder verzweigtkettiges C₂-C₆-Alkyl oder -Alkenyl, worin die Alkylkette substituiert ist in einer oder mehreren Positionen durch Ar₁, wie oben definiert, C₃-C₈-Cycloalkyl, Cycloalkyl, das gebunden ist durch eine gerade oder unverzweigte C₁-C₆-Alkyl- oder -Alkenylkette oder Ar₂, worin Ar₂ ausgewählt ist aus der Gruppe, die besteht aus 2-Indolyl, 3-Indolyl, 2-Furyl, 3-Furyl, 2-Thiazolyl, 2-Thienyl, 3-Thienyl, 2-, 3- oder 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁-C₆₋Alkyl oder -Alkenyl, C₅-C₄-Alkoxy oder C₁-C₄-Alkenyloxy, Phenoxy, Benzyloxy und Amino;
oder pharmazeutisch akzeptable Salze oder Hydrate davon; oder
(iii) eine Verbindung der Formel: 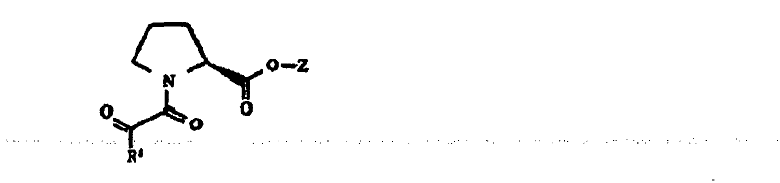 worin:
R₁ steht für eine geradkettige (unverzweigte) oder verzweigtkettige C₁-C₅-Alkyl-Gruppe oder -Alkenyl-Gruppe, die gegebenenfalls substituiert ist durch C₃-C₆₋Cycloalkyl oder Ar₁, worin Ar₁ ausgewählt ist aus der Gruppe, die besteht aus 2-Furyl, 2-Thienyl oder Phenyl;
X ausgewählt ist aus der Gruppe, die besteht aus Sauerstoff und Schwefel;
Y steht für Sauerstoff; und
Z steht für ein geradkettiges (unverzweigtes) oder verzweigtkettiges Alkyl oder - Alkenyl, worin die Alkylkette in einer oder mehreren Positionen substituiert ist durch Ar₁, wie oben definiert, C₃-C₆-Cycloalkyl, Ar₂, worin Ar₂ ausgewählt ist aus der Gruppe, die besteht aus 2-, 3- oder 4-Pyridyl oder Phenyl, das 1 bis 3 Substituenten aufweist, die unabhängig voneinander ausgewählt sind aus der Gruppe, die besteht aus Wasserstoff und C₁-C₄-Alkoxy; oder
(iv)
3-Phenyl-I-propyl-(2S)-1-(3,3-dimethyl-1,2-dioxopentyl)-2-pyrrolidin-carboxylat,
3-Phenyl-1-prop-2-(E)-enyl-(25)-1-(3,3-dimethyl-1,2-dioxopentyl)-2-pyrrolidin-carboxylat,
3-(3,4,5-Trimethoxyphenyl)-1-propyl-(2S)-1-(3,3-dimethyl-1,2-dioxopentyl)-2-pyrrolidin-carboxylat,
3-(3,4,5-Trimethoxyphenyl)-1-prop-2-(E)-enyl-(2S)-1-(3,3-dimethyl-1,2-dioxopentyl)-2-pyrrolidin-carboxylat,
3-(4,5-Methylendioxyphenyl)-1-propyl-(2S)-1-(3,3-dimethyl-1,2-dioxopentyl)-2-pyrrolidin-carboxylat,
3-(4,5-Methylendioxyphenyl)-1-prop-2-(E)-enyl-(2S)-1-(3,3-dimethyl-1,2-dioxopentyl)-2-pyrrolidin-carboxylat,
3-Cyclohexyl-1-propyl-(2S)-1-(3,3-dimethyl-1,2-dioxopentyl)-2-pyrrolidin-carboxylat,
3-Cyclohexyl-1-prop-2-(E)-enyl-(2S)-1-(3,3-dimethyl-1,2-dioxopentyl)-2-pyrrolidincarboxylat,
(1 R)-1,3-Diphenyl-1-propyl-(2S)-1-(3,3-dimethyl-1,2-dioxopentyl)-2-pyrrolidin-carboxylat,
3-Phenyl-1-propyl-(2S)-1-(1,2-dioxo-2-[2-furanyl])ethyl-2-pyrrolidin-carboxylat,
3-Phenyl-1-propyl-(2S)-1-(1,2-dioxo-2-[2-thienyl])ethyl-2-pyrrolidin-carboxylat,
3-Phenyl-1-propyl-(2S)-1-(1,2-dioxo-2-[2-thiazolyl))ethyl-2-pyrrolidin-carboxylat,
3-Phenyl-1-propyl-(2S)-1-(1,2-dioxo-2-phenyl)ethyl-2-pyrrolidin-carboxylat,
3-(2,5-Dimethoxyphenyl)-1-propyl-(2S)-1-(3,3-dimethyl-1,2-dioxopentyl)-2-pyrrolidin-carboxylat,
3-(2,5-Dimethoxyphenyl)-1-prop-2-(E)-enyl-(2S)-1-(3,3-dimethyl-1,2-dioxopentyl)-2-pyrrolidin-carboxylat,
2-(3,4,5-Trimethoxyphenyl)-1-ethyl-(2S)-1-(3,3-dimethy)-1,2-dioxopentyl)-2-pyrrolidin-carboxylat,
3-(3-Pyridyl)-1-propyl-(2S)-1-(3,3-dimethyl-1,2-dioxopentyl)-2-pyrrolidin-carboxylat,
3-(2-Pyridyl)-1-propyl-(2S)-1-(3,3-dimethyt-1,2-dioxopentyl)-2-pyrrolidin-carboxylat,
3-(4-Pyridyl)-1-propyl (2S)-1-(3,3-dimethy)-1,2-dioxopentyl)-2-pyrrolidn-carboxylat,
3-Phenyl-1-propyl-(2S)-1-(2-cyclohexyl-1,2-dioxoethyl)-2-pyrrolidin-carboxylat,
3-Phenyl-1-propyl-(2S)-1-(2-tert-butyl-1,2-dioxoethyl)-2-pyrrolidin-carboxylat,
3-Phenyl-1-propyl-(2S)-1-(2-cyclohexylethyl-1,2-dioxoethyl)-2-pyrrolidin-carboxylat,
3-(3-Pyridyl)-1-propyl-(2S)-1-(2-cyclohexylethyl-1,2-dioxoethyl)-2-pyrrolidin-carboxylat,
3-(3-Pyridyl)-1-propyl-(2S)-1-(2-tert-butyl-1,2-dioxoethyl)-2-pyrrolidin-carboxylat,
3,3-Diphenyl-1-propyl-(2S)-1-(3,3-dimethyl-1,2-dioxopentyl)-2-pyrrolidin-carboxylat,
3-(3-Pyridyl)-1-propyl-(2S)-1-(2-cyclohexyl-1,2-dioxoethyl)-2-pyrrolidin-carboxylat,
3-(3-Pyridyl)-1-propyl-(2S)-N-([2-thienyl]-glyoxyl)-pyrrolidin-carboxylat,
3,3-Diphenyl-1 -propyl-(2S)-1 -(3,3-dimethyl-l ,2-dioxobuyl)-2-pyrrolidin-carboxylat,
3,3-Diphenyl-1-propyl-(2S)-1-cyclohexylglyoxyl-2-pyn-olidin-carboxylat,
3,3-Diphenyl-1-propyl-(2S)-1-(2-thienyl)glyoxyl-2-pyrrolidin-carboxylat,
3-(2,5-Dimethoxyphenyl)-1-propyl-(2S)-1-(3,3-dimethyl-1,2-dioxopentyl)-2-pyrrolidin-carboxylat,
3-(2,5-Dimethoxyphenyl)-1-prop-2-(E)-enyl-(2S)-1-(3,3-dimethyl-1,2-dioxopentyl)-2-pyrrolidin-carboxylat,
2-(3,4,5-Trimethoxyphenyl)-1-ethyl-(2S)-1-(3,3-dimethyl-1,2-dioxopentyl)-2-pyrrolidincarboxylat,
3-(3-Pyridyl)-1-propyl-(2S)-1-(3,3-dimethyl-1,2-dioxopentyl)-2-pyrrolidin-carboxylat,
3-(2-Pyridyl)-1-propyl-(2S)-1-(3,3-dimethyl-1,2-dioxopentyl)-2-pyrrolidin-carboxylat,
3-(4-Pyridyl)-1-propyl-(2S)-1-(3,3-dimethyl-1,2-dioxopentyl)-2-pyrrolidin-carboxylat,
3-Phenyl-1-propyl-(2S)-1-(2-tert-butyl-1,2-dioxoethyl)-2-pyrrolidin-carboxylat,
3-Phenyl-1-propyl-(2S)-1-(2-cyclohexylethyl-1,2-dioxoethyl)-2-pyrrolidin-carboxylat,
3-(3-Pyridyl)-1-propyl-(2S)-1-(2-cyclohexylethyl-1,2-dioxoethyl)-2-pyrrolidin-carboxylat,
3-(3-Pyridyl)-1-propyl-(2S)-1-(2-tert-butyl-1,2-dioxoethyl)-2-pyrrolidin-carboxylat,
3,3-Diphenyl-1-propyl-(2S)-1-(3,3-dimethyl-1,2-dioxopentyl)-2-pyrrolidin-carboxylat,
3-(3-Pyridyl)-1-propyl-(2S)-1-(2-cyclohexyl-1,2-dioxoethyl)-2-pyrrolidin-carboxylat,
3-(3-Pyridyl)-1-propyl-(2S)-N-([2-thienyl]-glyoxyl)pyrrolidin-carboxylat,
3,3-Diphenyl-1-propyl-(25)-1-(3,3-dimethyl-1,2-dioxobutyl)-2-pyrrolidin-carboxylat,
3,3-Diphenyl-1-propyl-(2S)-1-cyclohexylglyoxyl-2-pyrrolidin-carboxylat, und
3,3-Diphenyl-1-propyl-(2S)-1-(2-thienyl)-glyoxyl-2-pyrrolidin-carboxylat,
(f) ein Pipecolsäure-Derivat, bei dem es sich handelt um 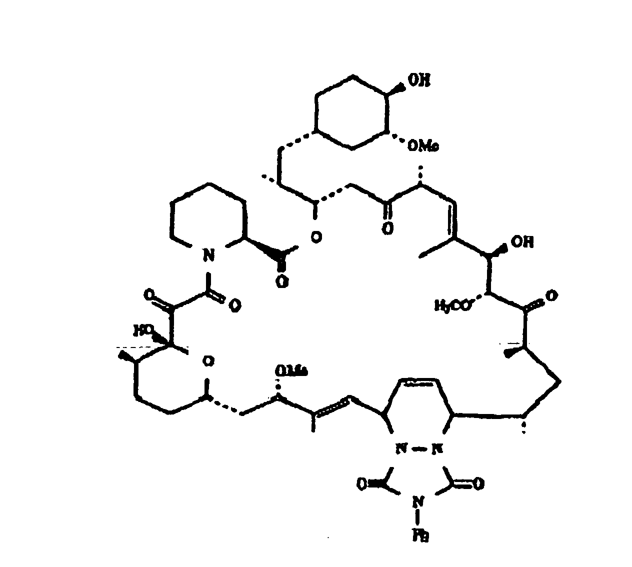 oder 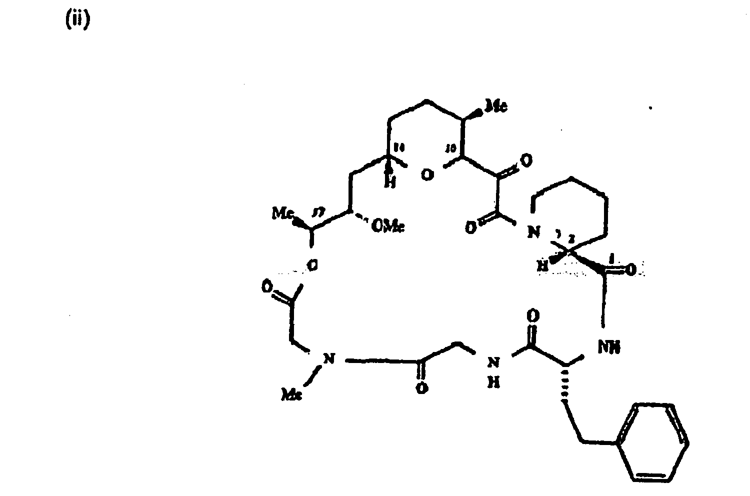 oder 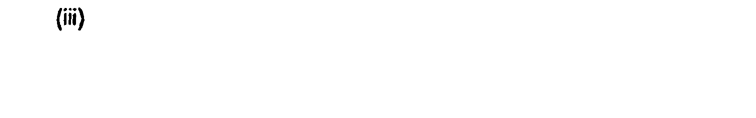 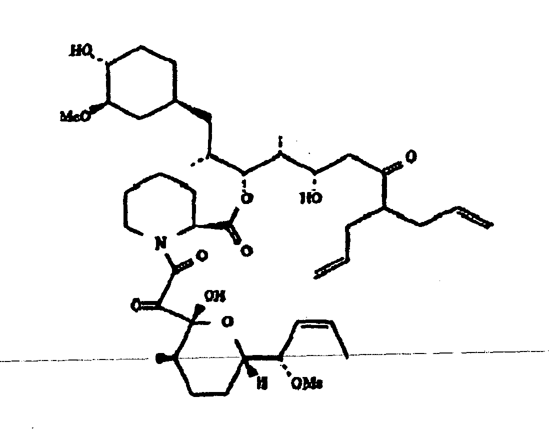 oder
(iv)
4-(4-Methoxyphenyl)butyl(2S)-1-[2-(3,4,5-trimethoxphenyl)acryloyl]-hexahydro-2-pyridin-carboxylat,
4-(4-Methoxyphenyl)butyl(2S)-1-[2-(3,4,5-trimethoxyphenyl)propanoyl]hexahydro-2-pyridin-carboxylat,
4-(4-Methoxyphenyl)butyl(2S)-1-[2-oxo-2-(3,4,5-trimethoxyphenyl)acetyl]hexahydro-2-pyridin-carboxylat,
3-Cyclohexylpropyl(2S)-1-(3,3-dimethyl-2-oxopentanoyl)hexahydro-2-pyridin-carboxylat,
3-Phenylpropyl(2S)-1-(3,3-dimethyl-2-oxopentanoyl)hexahydro-2-pyridin-carboxylat,
3-(3,4,5-Trimethoxyphenyl)-propyl(2S)-1-(3,3-dimethyl-2-oxopentanoyl)hexahydro-2-pyridin-carboxylat,
(1R)-2,2-Dimethy)-1-phenethyl-3-butenyl-(2S)-1-(3,3-dimethyl-2-oxopentanoyl)hexahydro-2-pyridin-carboxylat,
(1R)-1,3-Diphenylpropyl(2S)-1-(3,3-dimethyl-2-oxopentanoyl)hexahydro-2-pyridin-carboxylat,
(1R)-1-Cyclohexyl-3-phenylpropyl-(2S)-1-(3,3-dimethyl-2-oxopentanoyl)hexahydro-2-pyrid in-ca rboxylat,
(1S)-1,3-Diphenylpropyl(2S)-1-(3,3-dimethyl-2-oxopentanoyl)hexahydro-2-pyridin-carboxylat,
(1S)-1-Cyclohexyl-3-phenylpropyl(2S)-1-(3,3-dimethyl-2-oxopentanoyl)hexahydro-2-pyridin-carboxylat,
(22aS)-15,15-Dimethylperhydropyrido[2,1-c][1,9,4]dioxazacyclononadecin-1,12,16,-17-tetraon,
(24aS)-17,17 -Dimethylperhyd ropyrido[2, 1-c][1,9,4]dioxazacyclohenicosin-1 14,18,19-tetraon,
(3R,4R,23aS)-8-allyl-4,10-dimethyl-3-[2-(3-pyridyl)ethyl]-1,3,4, 5,6, 7,8,11,12,15,16,-17,18,20,21,22,23,23a-octadecahydro-14H-pyrido[2,1-c][1,10,4]dioxazacycloicosin-1,7,14,17,18-pentaon
(3R,4R,24aS)-8-Allyl-4,10-dimethyl-3-[2-(3-pyridyl)ethyl]-1,3,4, 5,6,7,8,11,12,14,15,-16,17,18,19,21,22,23,24,24a-icosahydropyrido[2,1-c][1,11,4]dioxazacyclohenicosin-1,7,14,18,19-pentaon,
(3R,4R,25aS)-8-Allyl-4,10-dimethyl-3-[2-(3-pyridyl)ethyl]-1,3,4,5,6, 7,8,11,12,15,16,-17,18,19,20,22,23,24,25,25a-icosahydro-14H-pyrido[2,1-c][1,12,4]dioxazacyclodocosin-1,7,14,19,20-pentaon,
(1 R)-1-(3-Benzoylphenyl)-3-phenylpropyl(1 R)-2-(3,3-dimethyl-2-oxopentanoyl)-cyclohexan-1-carboxylat,
(1 R)-1-[3-(Diallylcarbamoyl)phenyl]-3-phenylpropyl(1 R)-2-(3,3-dimethyl-2-oxopentanoyl)cyclohexan-1-carboxylat,
Ethyl-1-(2-oxo-3-phenylpropanoyl)-2-piperidin-carboxylat,
Ethyl-1-pyruvoyl-2-piperidin-carboxylat,
Ethyl-1-(2-oxobutanoyl)-2-piperidin-carboxylat,
Ethyl-1-(3-methyl-2-oxobutanoyl)-2-piperidin-carboxylat,
Ethyl-1-(4-methyl-2-oxopentanoyl)-2-piperidin-carboxylat,
Ethyl-1-(3,3-dimethyl-2-oxobutanoyl)-2-piperidin-carboxylat,
Ethyl-1-(3,3-dimethyl-2-oxopentanoyl)-2-piperidin-carboxylat,
4-[2-(Ethyloxycarbonyl)piperidinol-2,2-dimethyl-3,4-dioxobutylacetat,
Ethyl-1-[2-(2-hydroxytetrahydro-2H-2-pyranyl)-2-oxoacetyl]-2-piperidin-carboxylat,
Ethyl-1-[2-(2-methoxytetrahydro-2H-2-pyranyl)-2-oxoacetyl]-2-piperidin-carboxylat,
Ethyl-1-[2-(1-hydroxycyclohexyl)-2-oxoacetyl]-2-piperidin-carboxylat,
Ethyl-1-[2-(1-methoxycyclohexyl)-2-oxoacetyl]-2-piperidin-carboxylat,
Ethyl-1-(2-cyclohexyl-2-oxoacetyl)-2-pipiperidin-carboxylat,
Ethyl-1-(2-oxo-2-piperidinoacetyl)-2-piperidin-carboxylat,
Ethyl-1-[2-(3,4-dihydro-2H-6-pyranyl)-2-oxoacetyl)-2-piperidin-carboxylat,
Ethyl-1-(2-oxo-2-phenytacetyl)-2-piperidin-carboxylat,
Ethyl-1-(4-methyl-2-oxo-1-thioxopentyl)-2-piperidin-carboxylat,
3-Phenylpropyl-1-(2-hydroxy-3,3-dimethylpentanoyl)-2-piperidin-carboxylat,
(1R)-1-phenyl-3-(3,4,5-trimethoxyphenyl)propyl-1-(3,3-dimethylbutanoyl)-2-piperidin-carboxylat,
(1R)-1,3-Diphenylpropyl-1-(benzylsulfonyl)-2-piperidin-carboxylat,
3-(3,4,5-Trimethoxyphenyl)propyl-1-(benzyfsulfonyl)-2-piperidin-carboxylat,
1-(2-[(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carbonsäure,
Methyl-1-(2-[(2R,3R,6S)-6-[(2S,3E,5E,7E,9S,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidinecarboxylat,
Isopropyl-1-(2-[(2R,3R,6S)-6-[(2S,3E,5E,7E,9S,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat,
Benzyl-1-(2-[(2R,3R,65)-6-[(25,3E,5E,7E,95,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat,
1-Phenylethyl-1-(2-[(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat,
(Z)-3-Phenyl-2-propenyl-1-(2-(2,3R,6S)-6-[(2S,3E,5E,7E,9S,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat,
3-(3,4-Dimethoxyphenyl)-propyl-1-(2-[(2R,3R,6S)-6-[(2S,3E,5E,7E,9S,11 R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat,
N-2-Benzyl-1-(2-[(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat, und
N-2-(3-Phenylpropyl)-1-(2-(2R,3R,6S)-6-[(2S,3E,5E,7E,9S,11R)-2,13-dimethoxy-3,9,11-trimethyl-12-oxo-3,5,7-tridecatrienyl]-2-hydroxy-3-methyltetrahydro-2H-2-pyranyl)-2-oxoacetyl)-2-piperidin-carboxylat; oder 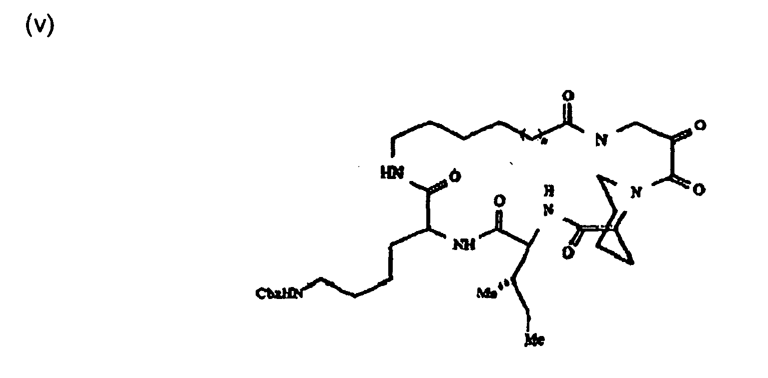 worin n steht für die Zahl 1, 2 oder 3; 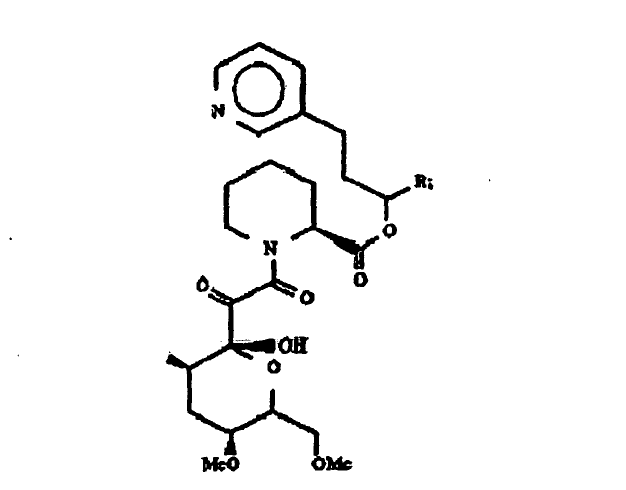 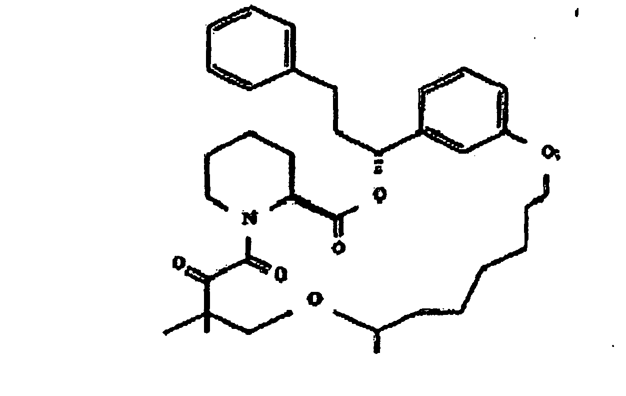 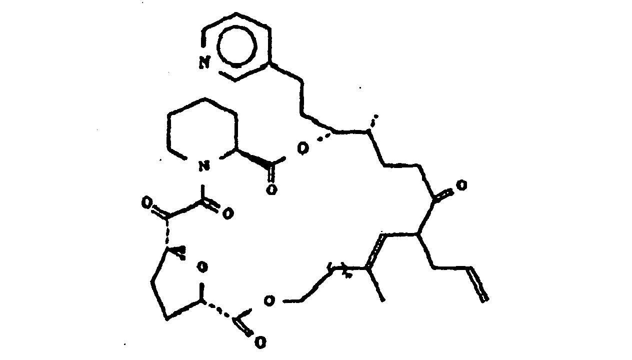 worin n steht für die Zahl 1, 2 oder 3, 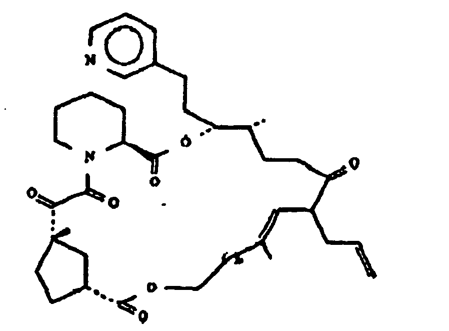 worin n steht für die Zahl 1, 2 oder 3, 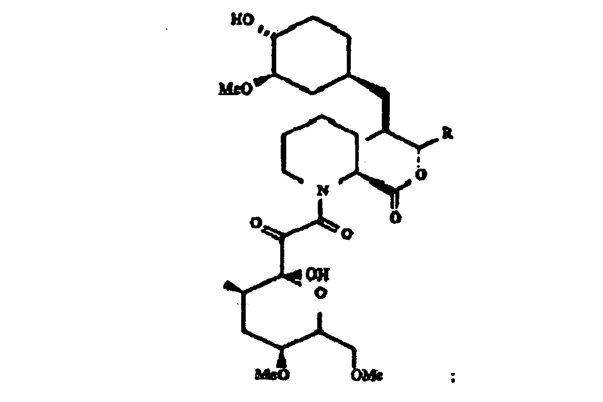 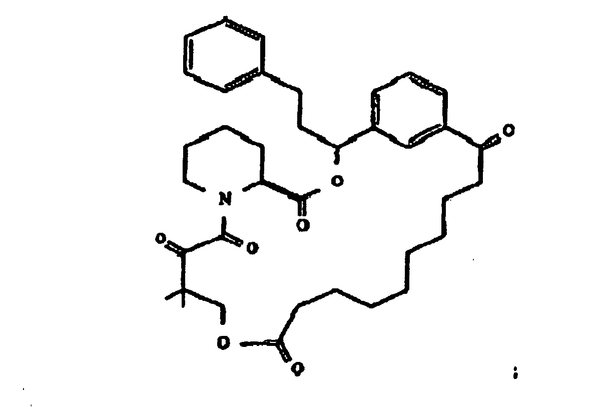 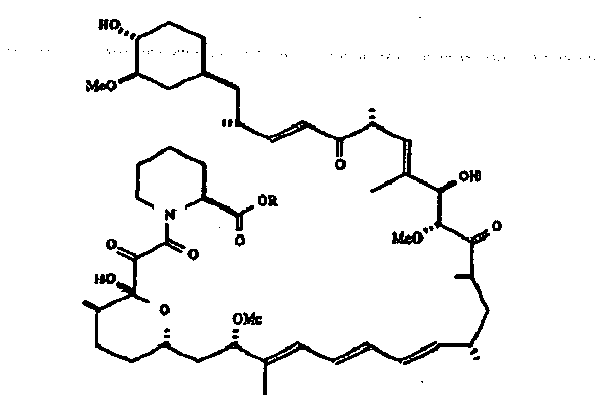 worin R steht für Methyl (Me) oder Benzyl (Bn), 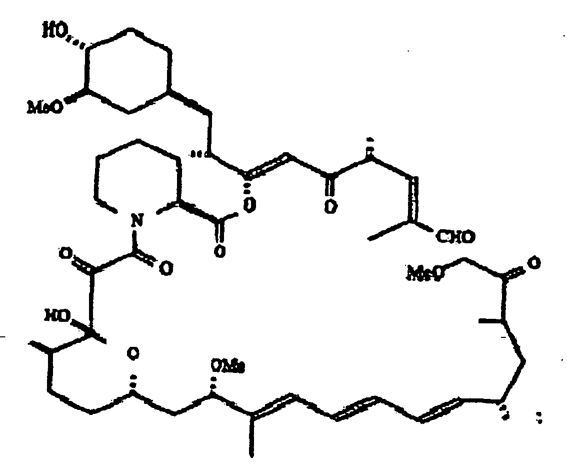 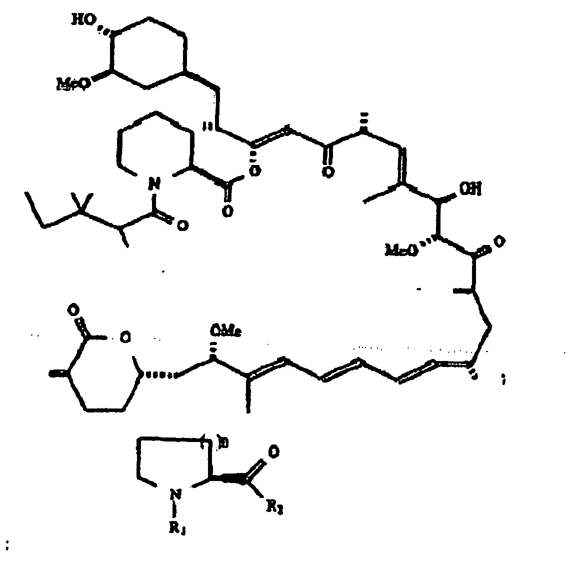 worin n = 2
R₁ = 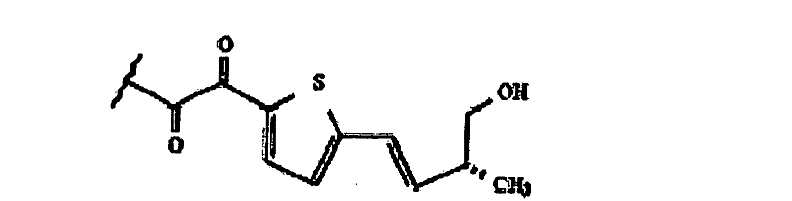 oder 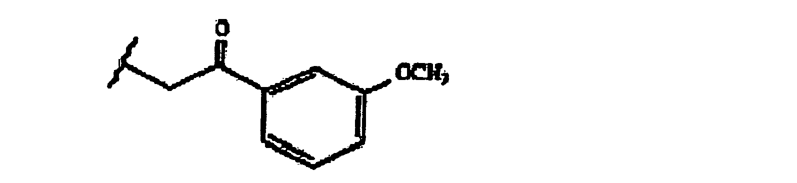 und R₂ =Phe-o-tert-butyl, 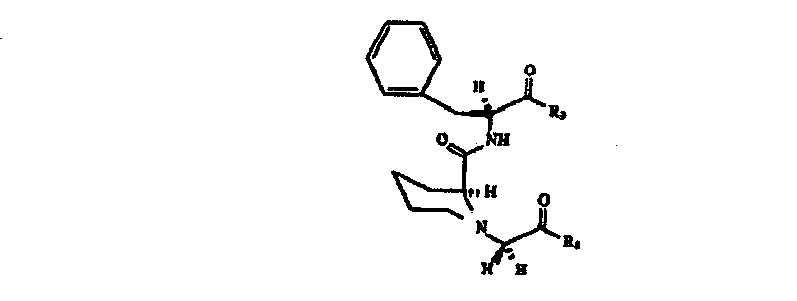 worin
R₁ =m-OCH₃ Ph, R₃ =Val-o-tert-butyl;
R₁ =m-OCH₃ Ph, R₃ =Leu-o-tert-butyl;
R₁ =m-OCH₃ Ph, R₃ =lleu-o-tert-butyl;
R₁ =m-OCH₃ Ph, R₃ = Hexahydro-Phe-o-tert-butyl;
R₁ =m-OCH₃ Ph, R₃ = Allylalanin-o-tert-butyl;
R₁ = B-Naphthyl, R₃ = Val-o-tert-butyl; 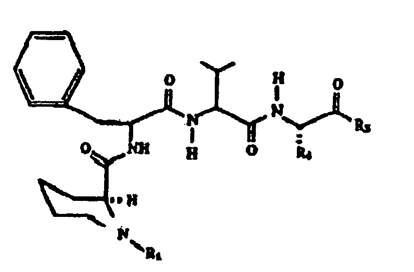 worin R₁ =CH₂(CO)-m-OCH₃Ph
R₄ =CH₂ Ph
R₅ =OCH₃
oder
R₁ =CH₂ (CO)-B-naphthyt
R₄ =CH₂Ph
R₅ =OCH₃ 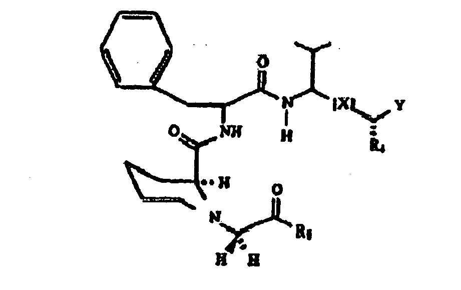 worin R₁ =m-OCH₃ Ph
X=trans-CH=CH
R₄ =H
Y=OC(o)Ph
R₁ =m-OCH₃ Ph
X=trans-CH=CH
R₄ =H
Y=OC(o)CF₃R₁ =m-OCH₃ Ph
X=trans-CH=CHI
R₄=-
Y=-
R₁ =m-OCH₃ Ph
X=trans-CH=CH
R₄ =H
Y=OCH₂ CH=CH₂
R₁ =m-OCH₃ Ph
X=C=O
R₄ =H
Y=Ph 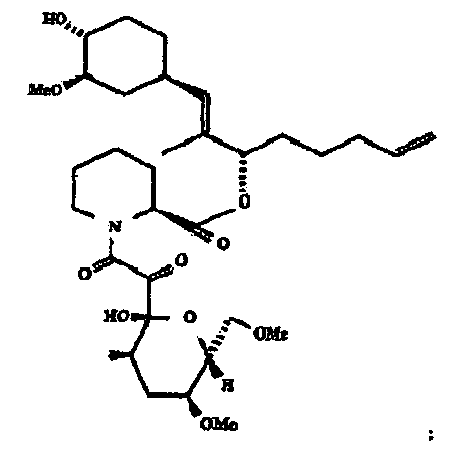 worin R₁ =H, R₂ =OMe, und R₃ =CH₂ OMe;
R₁ =H, R₂ =H, und R₃ =H; und
R₁ =Me, R₂ =H, und R₃ =H,
(g) ein heterocyclisches Ester- oder Amid-Derivat, bei dem es sich handelt um 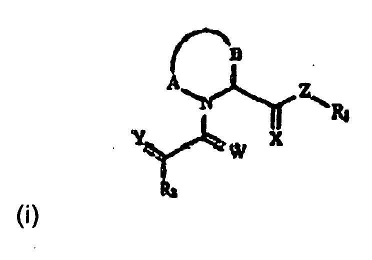 oder ein pharmazeutisch akzeptables Salz davon, worin:
A und B zusammen mit den Stickstoff- und Kohlenstoffatomen, an die sie jeweils gebunden sind, einen 5- bis 7-gliedrigen gesättigten oder ungesättigten heterocyclischen Ring bilden, der zusätzlich zu dem Stickstoffatom mindestens ein weiteres O-, S-, SO-, SO₂-, NH- oder NR₁-Heteroatom in einem chemisch stabilen Oxidationszustand enthält;
X steht für O oder S;
Z steht für O, NH oder NR₁;
W und Y unabhängig voneinander stehen für O, S, CH₂ oder H₂;
R₁ steht für geradkettiges (unverzweigtes) oder verzweigtkettiges C₁-C₆-Alkyl oder -Alkenyl, das substituiert ist in einer oder mehreren Positionen durch (Ar₁)ₙ, (Ar₁)ₙ das gebunden ist durch ein geradkettiges (unverzweigtes) oder verzweigtkettiges C₁-C₆-Alkyl oder -Alkenyl, C₃-C₈-Cycloalkyl, C₃-C₈₋Cycloalkyl, das gebunden ist durch ein geradkettiges (unverzweigtes) oder verzweigtkettiges C₁-C₆-Alkyl oder -Alkenyl, Ar₂, oder eine Kombination davon;
n steht für die Zahl 1 oder 2;
R₂ steht entweder für geradkettiges (unverzweigtes) oder verzweigtkettiges C₁₋C₉-Alkyl oder -Alkenyl, C₃-C₈-Cycloalkyl, C₅-C₇-Cycloalkenyl oder Ar₁, worin das Alkyl, Alkenyl, Cycloalkyl oder Cycloalkenyl entweder unsubstituiert oder substituiert ist in einer oder mehreren Positionen durch geradkettiges (unverzweigtes) oder verzweigtkettiges C₁-C₄-Alkyl oder -Alkenyl, Hydroxyl oder eine Kombination davon; und
Ar₁ und Ar₂ unabhängig voneinander stehen für einen mono-, bi- oder tricyclischen, carbocyclischen oder heterocyclischen Ring, worin der Ring entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁₋C₆-Alkyl oder -Alkenyl, C₁-C₄-Alkoxy, C₁-C₄-Alkenyloxy, Phenoxy, Benzyloxy, Amino oder eine Kombination davon;
wobei die einzelnen Ringe 5- bis 6-gliedrige Ringe sind und der heterocyclische Ring 1 bis 6 Heteroatome enthält, ausgewählt aus der Gruppe, die besteht aus O, N, S, und eine Kombination davon; oder
(ii) 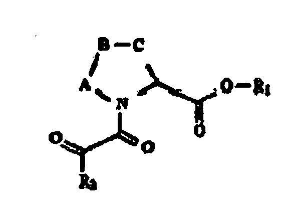 oder ein pharmazeutisch akzeptables Salz davon, worin:
A, B und C unabhängig voneinander stehen für CH₂, O, S, SO, SO₂, NH oder NR₁;
R₁ steht für geradkettiges (unverzweigtes) oder verzweigtkettiges C₁-C₅-Alkyl oder -Alkenyl, das substituiert ist in einer oder mehreren Positionen durch (Ar₁)ₙ, (Ar₁)ₙ, das gebunden ist durch ein geradkettiges (unverzweigtes) oder verzweigtkettiges C₁-C₆-Alkyl oder -Alkenyl, oder eine Kombination davon;
n steht für die Zahl 1 oder 2;
R₂ steht entweder für geradkettiges (unverzweigtes) oder verzweigtkettiges C₁₋C₉-Alkyl oder -Alkenyl, C₃-C₈-Cycloalkyl, C₅-C₇-Cycloalkenyl oder für Ar₁; und
Ar₁ steht für einen mono-, bi- oder tricyclischen, carbocyclischen oder heterocyclischen Ring, wobei der Ring entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁-C₆-Alkyl oder -Alkenyl, C₁-C₄₋Alkoxy, C₁-C₄-Alkenyloxy, Phenoxy, Benzyloxy, Amino oder eine Kombination davon;
wobei die einzelnen Ringe 5- bis 6-gliedrige Ringe sind und worin der heterocyclische Ring 1 bis 6 Heteroatome enthält, ausgewählt aus der Gruppe, die besteht aus O, N, S, und eine Kombination davon; oder 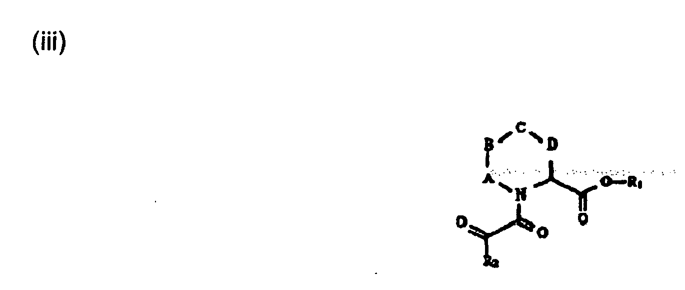
oder ein pharmazeutisch akzeptables Salz davon, worin:
A, B, C und D unabhängig voneinander stehen für CH₂, O, S, SO, SO₂, NH oder NR₁;
R₁ steht für geradkettiges (unverzweigtes) oder verzweigtkettiges C₁-C₅-Alkyl oder -Alkenyl, das substituiert ist in einer oder mehreren Positionen durch (Ar₁)ₙ, (Ar₁)ₙ, das gebunden ist durch ein geradkettiges (unverzweigtes) oder verzweigtkettiges C₁-C₆-Alkyl oder -Alkenyl, oder eine Kombination davon;
n steht für die Zahl 1 oder 2;
R₂ steht entweder für geradkettiges (unverzweigtes) oder verzweigtkettiges C₁₋C₉-Alkyl oder -Alkenyl, C₃-C₈-Cycloalkyl, C₅-C₇-Cycloalkenyl oder für Ar₁; und
Ar₁ steht für einen mono-, bi- oder tricyclischen, carbocyclischen oder heterocyclischen Ring, worin der Ring entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁-C₆-Alkyl oder -Alkenyl, C₁-C₄₋Alkoxy, C₁-C₄-Alkenyloxy, Phenoxy, Benzyloxy, Amino, oder eine Kombination davon;
wobei die einzelnen Ringe 5- bis 6-gliedrige Ringe sind und der heterocyclische Ring 1 bis 6 Heteroatome enthält, ausgewählt aus der Gruppe, die besteht aus O, N, S, und eine Kombination davon; oder
(iv)
3-Phenyl-1-propyl(2S)-1-(3,3-dimethyl-1,2-dioxopentyl)-2-(4-thiazolidin)carboxylat; und
3-(3-Pyridyi)-1-propyl(2S)-1-(3,3-dimethyf-1,2-dioxopentyl)-2-(4-thiazolidin)-carboxylat,
(h) ein Piperidin-Derivat, bei dem es sich handelt um
(i) 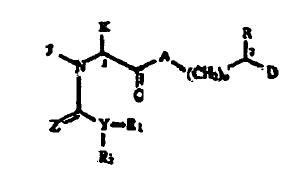 und pharmazeutisch akzeptable Derivate davon, worin:
A steht für CH₂, Sauerstoff, NR₁;
R₁, B und D voneinander unabhängig stehen für: Ar, unverzweigtes oder verzweigtes (C₁-C₆)-Alkyl, unverzweigtes oder verzweigtes (C₂-C₆)-Alkenyl oder -Alkinyl, (C₅-C₇)-Cycloalkyl-substituiertes unverzweigtes oder verzweigtes (C₁-C₆)-Alkyl, unverzweigtes oder verzweigtes (C₃₋C₆)-Alkenyl oder -Alkinyl, (C₅-C₇)-Cycloalkenyl-substituiertes unverzweigtes oder verzweigtes (C₁-C₆)-Alkyl, unverzweigtes oder verzweigtes (C₃-C₆)-Alkenyl oder -Alkinyl, Ar-substituiertes unverzweigtes oder verzweigtes (C₁₋C₆)-Alkyl oder Ar-substituiertes unverzweigtes oder verzweigtes (C₃-C₆)-Alkenyl oder -Alkinyl,
worin eine der CH₂-Gruppen der Alkylketten gegebenenfalls ersetzt ist durch ein Heteroatom, ausgewählt aus der Gruppe, die besteht aus O, S, SO, SO₂ und NR, worin R ausgewählt ist aus Wasserstoff, unverzweigtem oder verzweigtem (C₁-C₄)-Alkyl, unverzweigtem oder verzweigtem (C₃-C₄)-Alkenyl oder -Alkinyl oder (C₁-C₄)-verbrückenden-Alkyl, wobei eine Brücke zwischen dem Stickstoffatom und einem Kohlenstoffatom der ein Heteroatom enthaltenden Kette gebildet wird unter Bildung eines Ringes und worin dieser Ring gegebenenfalls an eine Ar-Gruppe ankondensiert ist;
J ausgewählt ist aus der Gruppe, die besteht aus Wasserstoff, unverzweigtem oder verzweigtem (C₁-C₆)-Alkyl, unverzweigtem oder verzweigtem (C₃-C₆)-Alkenyl oder -CH₂-Ar;
K ausgewählt ist aus der Gruppe, die besteht aus unverzweigtem oder verzweigtem (C₁-C₄)-Alkyl, -CH₂-Ar, oder Cyclohexylmethyl;
oder J und K gemeinsam einen 5- bis 7-gliedrigen heterocyclischen Ring bilden, der gegebenenfalls ein Heteroatom enthält, ausgewählt aus O, S, SO oder SO₂;
Z steht für O oder S;
Y steht für O oder N, wobei dann, wenn Y für O steht, R₁ ein freies Elektronenpaar ist (der hier verwendete Ausdruck "freies Elektronenpaar" bezieht sich auf ein freies Elektronenpaar, wie z.B. das Paar von Elektronen, das an divalentem Sauerstoff vorhanden ist) und R₂ ausgewählt ist aus Ar, unverzweigtem oder verzweigtem (C₁-C₆)-Alkyl oder unverzweigtem oder verzweigtem (C₃-C₆)-Alkenyl oder -Alkinyl; und dann, wenn Y für N steht, R₁ und R₂ unabhängig voneinander ausgewählt sind aus Ar, verzweigtem oder unverzweigtem (C₁-C₆)-Alkyl oder unverzweigtem oder verzweigtem (C₃-C₆)-Alkenyl oder -Alkinyl; oder R₁ und R₂ gemeinsam einen heterocyclischen 5- bis 6-gliedrigen Ring bilden, ausgewählt aus Pyrrolidin, Imidazolidin, Pyrazolidin, Piperidin oder Piperazin;
Ar ausgewählt ist aus der Gruppe, die besteht aus Phenyl, 1-Naphthyl, 2-Naphthyl, Indenyl, Azulenyl, Fluorenyl oder Anthracenyl, 2-Furyl, 3-Furyl, 2-Thienyl, 3-Thienyl, 2-Pyridyl, 3-Pyridyl, 4-Pyridyl, Pyrrolyl, Oxazolyl, Thiazolyl, Imidazolyl, Pyrazolyl, 2-Pyrazolinyl, Pyrazolidinyl, Isoxazolyl, Isotriazolyl, 1,2,3-Oxadiazolyl, 1,2,3-Triazolyl, 1,3,4-Thiadiazolyl, Pyridazinyl, Pyrimidinyl, Pyrazinyl, 1,3,5-Triazinyl, 1,3,5-Trithianyl, Indolizinyl, Indolyl, Isoindolyl, 3H-Indolyl, Indolinyl, Benzo[b]furanyl, Benzo[b]thiophenyl, 1H-Indazolyl, Benzimidazolyl, Benzthiazolyl, Purinyl, 4H-Chinolizinyl, Chinolinyl, 1,2,3,4-Tetrahydrochinolinyl, Isochinolinyl, 1,2,3,4-Tetrahydro-isochinolinyl, Cinnolinyl, Phthalazinyl, Chinazolinyl, Chinoxalinyl, 1,8-Naphthyridinyl, Pteridinyl, Carbazolyl, Acridinyl, Phenazinyl, Phenothiazinyl oder Phenoxazinyl;
worin Ar gegebenenfalls 1 bis 3 Substituenten enthält, die unabhängig voneinander ausgewählt sind aus der Gruppe Wasserstoff, Halogen, Hydroxyl, Nitro, -SO₃H, Trifluormethyl, Trifluormethoxy, unverzweigtem oder verzweigtem (C₁-C₆)-Alkyl, unverzweigtem oder verzweigtem (C₂-C₆)-Alkenyl, O-[unverzweigtem oder verzweigtem (C₁-C₆)-Alkyl], O-[unverzweigtem oder verzweigtem (C₃-C₄)-Alkenyl], O-Benzyl, O-Phenyl, 1,2-Methylendioxy, -NR₃R₄, Carboxyl, N-unverzweigten oder verzweigten (C₁-C₅-Alkyl) oder unverzweigten oder verzweigten (C₃-C₅-Alkenyl)-carboxamiden, N,N-Di-(unverzweigten oder verzweigten C₁-C₅-Alkyl oder unverzweigten oder verzweigten C₃-C₅-Alkenyl)-carboxamiden, Morpholinyl, Piperidinyl, O-Z, CH₂-(CH₂)_{q}-Z, O-(CH₂)_{q}-Z, (CH₂)_{q} - Z-O-Z oder CH=CH-Z;
worin R₃ und R₄ unabhängig voneinander ausgewählt sind aus der Gruppe, die besteht aus unverzweigtem oder verzweigtem (C₁-C₆)-Alkyl, unverzweigtem oder verzweigtem (C₃-C₆)-Alkenyl, Wasserstoff oder Benzyl; oder
worin R₃ und R₄ gemeinsam einen 5- bis 6-gliedrigen oder 8- bis 11-gliedrigen heterocyclischen Ring bilden, wie z.B. Piperidinyl, Morpholinyl oder Pyrrolidinyl; worin
Z ausgewählt ist aus der Gruppe, die besteht aus 4-Methoxyphenyl, 2-Pyridyl, 3-Pyridyl, 4-Pyridyl, Pyrazyl, Chinolyl, 3,5-Dimethylisoxazoyl, Isoxazoyl, 2-Methylthiazoyl, Thiazoyl, 2-Thienyl, 3-Thienyl oder Pyrimidyl;
q steht für eine Zahl von 0 bis 2; und
n steht für die Zahl 0 oder 1; oder
(ii)
(S)-1-((3,4,5-Trimethoxyphenyl)-methyl-carbamoyl)piperidin-2-carbonsäure-4-pyridin-3-yl-1-(3-pyridin-3-yl-propyl)-butylester;
(S)-1-((3-Trifluormethylphenyl)-methyl-carbamoyl)piperidin-2-carbonsäure-4-pyridin-3-yl-1-(3-pyridin-3-yl-propyl)-butylester;
(S)-1-((4-Tert-butylphenyl)-methyl-carbamoyl)piperidin-2-carbonsäure-4-pyridin-3-yl-1-(3-pyridin-3-yl-propyl)butylester;
(S)-1-((4-Isopropylphenyl)-methyl-carbamoyl)piperidin-2-carbonsäure-4-pyridin-3-yl-1-(3-pyridin-3-yl-propyl)-butylester;
(S)-1-(Piperidin-1-carbonyl)piperidin-2-carbonsäure-4-pyridin-3-yl-1-(3-pyridin-3-yl-propyl)-butylester;
(S)-1-((3,4,5-Trimethoxyphenyl)-methyl-carbamoyl)piperidin-2-carbonsäure-4-pyridin-1-yl-1-(3-pyridin-1-yl-propyl)-butylester;
(S)-Piperidin-1,2-dicarbonsäure-1-(3,4,5-trimethoxyphenyl)ester-2-(4-pyridin-3-yl-1-(3-pyridin-3-yl-propyl)-butyl)ester;
(S)-1-((3,4,5-Trimethoxyphenyl)-methyl-carbamoyl)piperidin-2-carbonsäure-1-(2-phenyl-ethyl)-3-phenyl-propylester,
4-(Methyl-(2-(-1-phenethyl-3-phenyl-propoxycarbonyl)piperidin-1-carbonyl)-amino)-benzolsulfonsäure;
(S)-Piperidin-2-carbonsäure-1-benzyloxy-methyl-2-benzyloxyethylester;
(S)-1-(Methyl-(4-morpholin-1-yl-phenyl)-carbamoyl)piperidin-2-carbonsäure-2-benzyloxy-1-(benzyloxy-methyl)-ethylester;
(S)-1-(Methyl-(4-piperidin-1-yl-phenyl)-carbamoyl)piperidin-2-carbonsäure-2-benzyloxy-1-(benzyloxy-methyl)-ethylester;
(S)-Piperidin-1,2-dicarbonsäure-2-(2-benzyloxy-1-(benzyloxymethyl)-ethyl)ester-1-chinolin-5-yl-ester;
(S)-Piperidin-1,2-dicarbonsäure-2-(2-benzyloxy-1-(benzyloxymethyl)-ethyl)ester-1-pyridin-3-yl-ester;
(i) ein N-Oxid eines heterocyclischen Esters, Amids, Thioesters oder Ketons, bei dem es sich handelt um 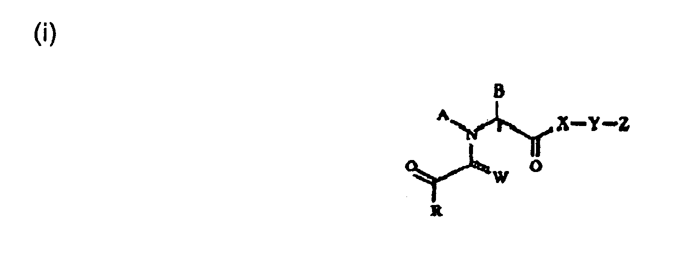 oder ein pharmazeutisch akzeptables Salz davon, worin:
A und B gemeinsam zusammen mit den Stickstoff- und Kohlenstoffatomen, an die sie jeweils gebunden sind, einen 5- bis 7-gliedrigen gesättigten oder ungesättigten heterocyclischen Ring bilden, der eine beliebige Kombination von CH₂, O, S, SO, SO₂, NH oder NR₁ in einem chemisch stabilen Oxidationszustand enthält;
W steht für O, S, CH₂ oder H₂;
R steht für eine geradkettige (unverzweigte) oder verzweigtkettige C₁-C₆-Alkyl-Gruppe oder -Alkenyl-Gruppe, die gegebenenfalls substituiert ist durch C₃-C₈₋Cycloalkyl, C₃-C₅-Cycloalkyl, C₅-C₇-Cycloalkenyl oder Ar₁, worin die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy und worin Ar₁ ausgewählt ist aus der Gruppe, die besteht aus 1-Naphthyl, 2-Naphthyl, 1-Indolyl, 2-Indolyl, 2-Furyl, 3-Furyl, 2-Thienyl, 3-Thienyl, 2-Pyridyl, 3-Pyridyl, 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, unverzweigtem oder verzweigtem C₁-C₆-Alkyl oder -Alkenyl, C₁-C₄-Alkenyloxy, Phenoxy, Benzyloxy und Amino;
X steht für O, NH, NR₁, S, CH, CR₁ oder C(R₁)₂;
Y steht für eine einfache Bindung oder ein geradkettiges (unverzweigtes) oder verzweigtkettiges C₁-C₆-Alkyl oder -Alkenyl, das gegebenenfalls substituiert ist in einer oder mehreren Positionen durch geradkettiges (unverzweigtes) oder verzweigtkettiges C₁-C₆-Alkyl oder -Alkenyl oder C₃-C₈-Cycloalkyl oder C₅-C₇₋Cycloalkenyl oder Hydroxyl oder Carbonyl-Sauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonyl-Sauerstoff oder worin eines der Kohlenstoffatome der Alkyl-, Alkenyl-, Cyclo-alkyl-, Cycloalkenyl- oder Ar-Gruppe gegebenenfalls ersetzt ist durch O, NH, NR₂, S, SO oder SO₂, wobei R₂ ausgewählt ist aus der Gruppe, die besteht aus Wasserstoff, geradkettigem (unverzweigtem) oder verzweigtkettigem (C₁₋C₄)-Alkyl, geradkettigem (unverzweigtem) oder verzweigtkettigem (C₃-C₄)-Alkenyl oder -Alkinyl und (C₁-C₄)-verbrückenden-Alkyl, wobei eine Brücke gebildet wird zwischen dem Stickstoffatom und einem Kohlenstoffatom der Alkyl- oder Alkenylkette, die das Heteroatom enthält, unter Bildung eines Ringes, wobei dieser Ring gegebenenfalls an eine Ar-Gruppe ankondensiert ist; und
Z ein aromatisches oder tertiäres Alkylamin darstellt, das zu einem entsprechenden N-Oxid oxidiert ist, wobei das aromatische Amin Ar darstellt, das zu einem entsprechenden N-Oxid oxidiert ist, worin Ar für einen mono-, bi- oder tricyclischen, carbocyclischen oder heterocyclischen Ring steht, der entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁-C₆-Alkyl oder -Alkenyl, C₁-C₄-Alkoxy, C₁-C₄-Alkenyloxy, Phenoxy, Benzyloxy, Amino oder eine Kombination davon; wobei die einzelnen Ringe 5-bis 6-gliedrige Ringe sind; unde der heterocyclische Ring 1 bis 6 Heteroatome enthält, ausgewählt aus der Gruppe, die besteht aus O, N, S, und eine Kombination davon, wobei mindestens eines der Heteroatome N ist und das Alkylamin zu einem entsprechenden N-Oxid oxidiert ist, worin Alkyl steht für ein geradkettiges (unverzweigtes) oder verzweigtkettiges C₁-C₆-Alkyl oder - Alkenyl, das gegebenenfalls substituiert ist in einer oder mehreren Positionen durch geradkettiges (unverzweigtes) oder verzweigtkettiges C₁-C₆-Alkyl- oder - Alkenyl oder C₃-C₈-Cycloalkyl oder C₅-C₇-Cycloalkenyl oder Hydroxyl oder Carbonylsauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff, oder worin eines der Kohlenstoffatome der Alkyl-, Alkenyl-, Cycloalkyl-, Cycloalkenyl- oder Ar-Gruppe gegebenenfalls ersetzt ist durch O, NH, NR₁, S, SO oder SO₂; und,
R₁ steht für Wasserstoff, geradkettiges (unverzweigtes) oder verzweigtkettiges (C₁-C₄)-Alkyl, geradkettiges (unverzweigtes) oder verzweigtkettiges (C₃-C₄)-Alkenyl oder -Alkinyl oder worin R₁ steht für Y-Z, das wie oben definiert ist; oder
(ii) 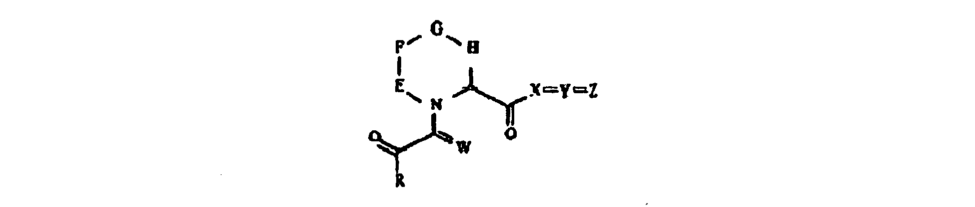 oder ein pharmazeutisch akzeptables Salz davon, worin:
E, F, G und H unabhängig voneinander stehen für CH₂, O, S, SO, SO₂, NH oder NR₁;
W steht für O, S, CH₂ oder H₂;
R steht für eine geradkettige (unverzweigte) oder verzweigtkettige C₁-C₆-Alkyl-Gruppe oder -Alkenyl-Gruppe, die gegebenenfalls substituiert ist durch C₃-C₈₋Cycloalkyl, C₃- oder C₅-Cycloalkyl, C₅-C₇-Cycloalkenyl oder Ar₁, wobei die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy und worin Ar₁ ausgewählt ist aus der Gruppe, die besteht aus 1-Naphthyl, 2-Naphthyl, 1-Indolyl, 2-Indolyl, 2-Furyl, 3-Furyl, 2-Thienyl, 3-Thienyl, 2-Pyridyl, 3-Pyridyl, 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, geradkettigem (unverzweigtem) oder verzweigtkettigem C₁-C₆-Alkyl oder -Alkenyl, C₁-C₄-Alkenyloxy, Phenoxy, Benzyloxy und Amino;
X steht für O, NH, NR₁, S, CH, CR₁ oder C(R₁)₂;
Y steht für eine einfache Bindung oder ein geradkettiges (unverzweigtes) oder verzweigtkettiges C₁-C₆-Alkyl oder -Alkenyl, das gegebenenfalls substituiert ist in einer oder mehreren Positionen durch geradkettiges (unverzweigtes) oder verzweigtkettiges C₁-C₆-Alkyl oder -Alkenyl, oder C₃-C₈-Cycloalkyl, oder C₅-C₇₋Cycloalkenyl oder Hydroxyl oder Carbonylsauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff oder worin eines der Kohlenstoffatome der Alkyl-, Alkenyl-, Cycloalkyl-, Cycloalkenyl- oder Ar-Gruppe gegebenenfalls ersetzt ist durch O, NH, NR₂, S, SO oder SO₂, worin R₂ ausgewählt ist aus der Gruppe, die besteht aus Wasserstoff, geradkettigem (unverzweigtem) oder verzweigtkettigem (C₁₋C₄)-Alkyl, geradkettigem (unverzweigtem) oder verzweigtkettigem (C₃-C₄)-Alkenyl oder -Alkinyl und (C₁-C₄)-verbrückenden-Alkyl, wobei eine Brücke gebildet wird zwischen dem Stickstoffatom und einem Kohlenstoffatom der Alkyl- oder Alkenylkette, die das Heteroatom enthält, unter Bildung eines Ringes, wobei dieser Ring gegebenenfalls an eine Ar-Gruppe ankondensiert ist; und
Z ein aromatisches oder tertiäres Alkylamin darstellt, das zu einem entsprechenden N-Oxid oxidiert ist, wobei das aromatische Amin Ar ist, das zu einem entsprechenden N-Oxid oxidiert ist, worin Ar steht für einen mono-, bi- oder tricyclischen, carbocyclischen oder heterocyclischen Ring, wobei der Ring unsubstituiert ist oder substituiert ist in 1 bis 3 Positionen durch Halogen, Hydroxyl, Nitro, Trifluormethyl, geradkettiges (unverzweigtes) oder verzweigtkettiges C₁-C₆-Alkyl oder -Alkenyl, C₁-C₄-Alkoxy, C₁-C₄-Alkenyloxy, Phenoxy, Benzyloxy, Amino, oder eine Kombination davon; wobei die einzelnen Ringe 5- bis 6-gliedrige Ringe sind und der heterocyclische Ring 1 bis 6 Heteroatome enthält, ausgewählt aus der Gruppe, die besteht aus O, N, S, und eine Kombination davon, wobei mindestens eines der Heteroatome N ist und das Alkylamin zu einem entsprechenden N-Oxid oxidiert ist, wobei Alkyl steht für ein geradkettiges (unverzweigtes) oder verzweigtkettiges C₁-C₆-Alkyl oder -Alkenyl, das gegebenenfalls substituiert ist in einer oder mehreren Positionen durch geradkettiges (unverzweigtes) oder verzweigtkettiges C₁-C₆-Alkyl oder -Alkenyl oder C₃-C₈-Cycloalkyl, oder C₅-C₇-Cycloalkenyl oder Hydroxyl, oder Carbonylsauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff oder worin eines der Kohlenstoffatome der Alkyl-, Alkenyl-, Cycloalkyl-, Cycloalkenyl- oder Ar-Gruppe gegebenenfalls ersetzt ist durch O, NH, NR₁, S, SO oder SO₂; und
R₁ steht für Wasserstoff, geradkettiges (unverzweigtes) oder verzweigtkettiges (C₁-C₄)-Alkyl, geradkettiges (unverzweigtes) oder verzweigtkettiges (C₃-C₄)-Alkenyl oder -Alkinyl oder R₁ steht für Y-Z; wie oben definiert; oder 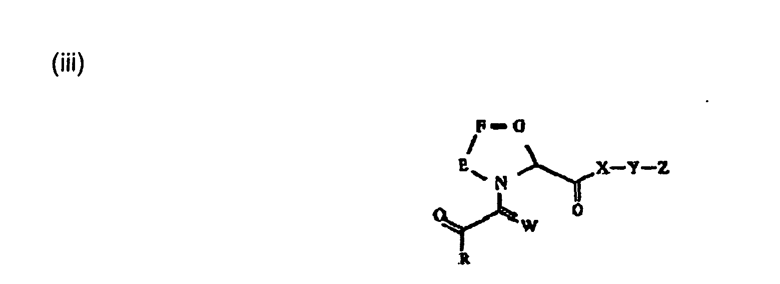
oder ein pharmazeutisch akzeptables Salz davon, worin:
E, F und G unabhängig voneinander stehen für CH₂, O, S, SO, SO₂, NH oder NR₁;
W steht für O, S, CH₂ oder H₂;
R steht für eine geradkettige (unverzweigte) oder verzweigtkettige C₁-C₆-Alkyl-Gruppe oder -Alkenyl-Gruppe, die gegebenenfalls substituiert ist durch C₃-C₈₋Cycloalkyl, C₃- oder C₅-Cycloalkyl, C₅-C₇-Cycloalkenyl oder Ar₁, wobei die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy, und Ar₁ ausgewählt ist aus der Gruppe, die besteht aus 1-Naphthyl, 2-Naphthyl, 1-Indolyl, 2-Indolyl, 2-Furyl, 3-Furyl, 2-Thienyl, 3-Thienyl, 2-Pyridyl, 3-Pyridyl, 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, geradkettigem (unverzweigtem) oder verzweigtkettigem C₁-C₆-Alkyl oder -Alkenyl, C₁-C₄-Alkenyloxy, Phenoxy, Benzyloxy und Amino;
X steht für O, NH, NR₁, S, CH, CR₁ oder C(R₁)₂;
Y steht für eine einfache Bindung oder ein geradkettiges (unverzweigtes) oder verzweigtkettiges C₁-C₆-Alkyl oder -Alkenyl, das gegebenenfalls substituiert ist in einer oder mehreren Positionen durch geradkettiges (unverzweigtes) oder verzweigtkettiges C₁-C₆-Alkyl oder -Alkenyl oder C₃-C₈-Cycloalkyl oder C₅-C₇₋Cycloalkenyl oder Hydroxyl oder Carbonylsauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff oder eines der Kohlenstoffatome der Alkyl-, Alkenyl-, Cycloalkyl-, Cycloalkenyl- oder Ar-Gruppe gegebenenfalls ersetzt ist durch O, NH, NR₂, S, SO oder SO₂, worin R₂ ausgewählt ist aus der Gruppe, die besteht aus Wasserstoff, geradkettigem (unverzweigtem) oder verzweigtkettigem (C₁-C₄)-Alkyl, geradkettigem (unverzweigtem) oder verzweigtkettigem (C₃-C₄)-Alkenyl oder-Alkinyl und (C₁-C₄)-verbrückenden-Alkyl, wobei eine Brücke gebildet wird zwischen dem Stickstoffatom und einem Kohlenstoffatom der Alkyl- oder Alkenyl-Kette, die das Heteroatom enthält, unter Bildung eines Ringes, der gegebenenfalls an eine Ar-Gruppe ankondensiert ist; und
Z steht für ein aromatisches oder tertiäres Alkylamin, däs zu einem entsprechenden N-Oxid, oxidiert ist, wobei das aromatische Amin Ar darstellt, das zu einem entsprechenden N-Oxid oxidiert ist, worin Ar steht für einen mono-, bi- oder tricyclischen, carbocyclischen oder heterocyclischen Ring, der entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁-C₆-Alkyl oder -Alkenyl, C₁-C₄-Alkoxy, C₁-C₄-Alkenyloxy, Phenoxy, Benzyloxy, Amino, oder eine Kombination davon; wobei die einzelnen Ringe 5- bis 6-gliedrige Ringe sind und der heterocyclische Ring 1 bis 6 Heteroatome enthält, ausgewählt aus der Gruppe, die besteht aus O, N, S, und eine Kombination davon, wobei mindestens eines der Heteroatome N ist und das Alkylamin oxidiert ist zu einem entsprechenden N-Oxid, worin Alkyl steht für ein geradkettiges (unverzweigtes) oder verzweigtkettiges C₁-C₆-Alkyl oder - Alkenyl, das gegebenenfalls substituiert ist in einer oder mehreren Positionen durch geradkettiges (unverzweigtes) oder verzweigtkettiges C₁-C₆-Alkyl oder - Alkenyl, oder C₃-C₈-Cycloalkyl oder C₅-C₇-Cycloalkenyl oder Hydroxyl oder Carbonylsauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff, oder worin eines der Kohlenstoffatome der Alkyl-, Alkenyl-, Cycloalkyl-, Cycloalkenyl- oder Ar-Gruppe gegebenenfalls ersetzt ist durch O, NH, NR₁, S, SO oder SO₂; und
R₁ steht für Wasserstoff, geradkettiges (unverzweigtes) oder verzweigtkettiges (C₁-C₄)-Alkyl, geradkettiges (unverzweigtes) oder verzweigtkettiges (C₃-C₄)-Alkenyl oder -Alkinyl oder R₁ steht für Y-Z, wie oben definiert; oder 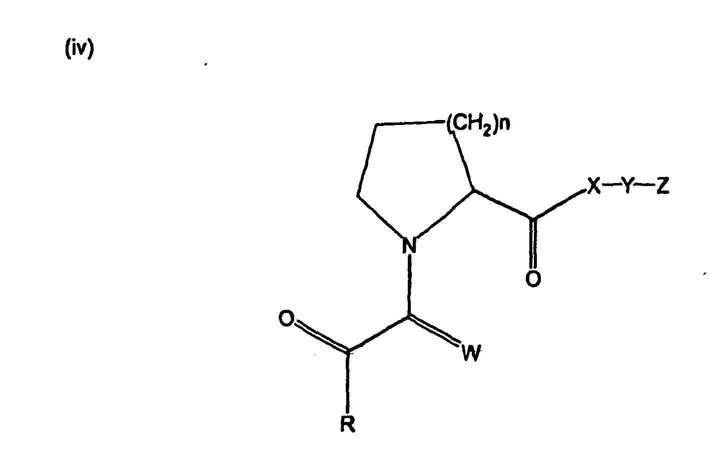 oder ein pharmazeutisch akzeptables Salz davon, worin:
n steht für die Zahl 1, 2 oder 3 zur Bildung eines 5- bis 7-gliedrigen heterocyclischen Ringes;
W steht für O, S, CH₂ oder H₂;
R steht für eine geradkettige (unverzweigte) oder verzweigtkettige C₁-C₆-Alkyl-Gruppe oder -Alkenyl-Gruppe, die gegebenenfalls substituiert ist durch C₃-C₈₋Cycloalkyl, C₃- oder C₅-Cycloalkyl, C₅-C₇-Cycloalkenyl oder Ar₁, wobei die Alkyl-, Alkenyl-, Cycloalkyl- oder Cycloalkenyl-Gruppen gegebenenfalls substituiert sein können durch C₁-C₄-Alkyl, C₁-C₄-Alkenyl oder Hydroxy und worin Ar₁ ausgewählt ist aus der Gruppe, die besteht aus 1-Naphthyl, 2-Naphthyl, 1-Indolyl, 2-Indolyl, 2-Furyl, 3-Furyl, 2-Thienyl, 3-Thienyl, 2-Pyridyl, 3-Pyridyl, 4-Pyridyl oder Phenyl, das 1 bis 3 Substituenten aufweist, die unabhängig voneinander ausgewählt sind aus der Gruppe, die besteht aus Wasserstoff, Halogen, Hydroxyl, Nitro, Trifluormethyl, geradkettigem (unverzweigtem) oder verzweigtkettigem C₁-C₆-Alkyl oder -Alkenyl, C₁-C₄-Alkenyloxy, Phenoxy, Benzyloxy und Amino;
X steht für O, NH, NR₁, S, CH, CR₁ oder C(R₁)₂;
Y steht für eine einfache Bindung oder ein geradkettiges (unverzweigtes) oder verzweigtkettiges C₁-C₆-Alkyl oder -Alkenyl, das gegebenenfalls substituiert ist in einer oder mehreren Positionen durch geradkettiges (unverzweigtes) oder verzweigtkettiges C₁-C₆-Alkyl oder -Alkenyl oder C₃-C₈-Cycloalkyl oder C₅-C₇₋Cycloalkenyl oder Hydroxyl oder Carbonylsauerstoff oder Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff oder worin eines der Kohlenstoffatome der Alkyl-, Alkenyl-, Cycloalkyl-, Cycloalkenyl- oder Ar-Gruppe gegebenenfalls ersetzt ist durch O, NH, NR₂, S, SO oder SO₂, wobei R₂ ausgewählt ist aus der Gruppe, die besteht aus Wasserstoff, geradkettigem (unverzweigtem) oder verzweigtkettigem (C₁-C₄)-Alkyl, geradkettigem (unverzweigtem) oder verzweigtkettigem (C₃-C₄)-Alkenyl oder - Alkinyl und (C₁-C₄) verbrückenden-Alkyl, wobei eine Brücke gebildet wird zwischen dem Stickstoffatom und einem Kohlenstoffatom der Alkyl- oder Alkenyl-Kette, die das Heteroatom enthält, zur Bildung eines Ringes, der gegebenenfalls an eine Ar-Gruppe ankondensiert ist; und
Z steht für ein aromatisches oder tertiäres Alkylamin, das zu einem entsprechenden N-Oxid oxidiert ist, wobei das aromatische Amin Ar darstellt, das zu einem entsprechenden N-Oxid oxidiert ist, worin Ar steht für einen mono-, bi- oder tricyclischen, carbocyclischen oder heterocyclischen Ring, der entweder unsubstituiert ist oder substituiert ist in 1 bis 3 Positionen durch Halogen, Hydroxyl, Nitro, Trifluormethyl, geradkettiges (unverzweigtes) oder verzweigtkettiges C₁-C₆-Alkyl oder -Alkenyl, C₁-C₄-Alkoxy, C₁-C₄-Alkenyloxy, Phenoxy, Benzyloxy, Amino, oder eine Kombination davon; wobei die einzelnen Ringe 5- bis 6-gliedrige Ringe sind und der heterocyclische Ring 1 bis 6 Heteroatome enthält, ausgewählt aus der Gruppe, die besteht aus O, N, S, und eine Kombination davon, wobei mindestens eines der Heteroatome N ist und das Alkylamin oxidiert ist zu einem entsprechenden N-Oxid, worin Alkyl steht für ein geradkettiges (unverzweigtes) oder verzweigtkettiges C₁-C₆-Alkyl oder - Alkenyl, das gegebenenfalls substituiert ist in einer oder mehreren Positionen durch geradkettiges (unverzweigtes) oder verzweigtkettiges C₁-C₆-Alkyl oder - Alkenyl oder C₃-C₈-Cycloalkyl oder C₅-C₇-Cycloalkenyl oder Hydroxyl oder Carbonylsauerstoff oder durch Ar, wobei die Alkyl-, Alkenyl-, Cycloalkyl-, Cycloalkenyl- oder Ar-Gruppe gegebenenfalls substituiert ist durch C₁-C₄-Alkyl, C₁-C₄-Alkenyl oder Hydroxy oder Carbonylsauerstoff oder worin eines der
R₁ Kohlenstoffatome der Alkyl-, Alkenyl-, Cycloalkyl-, Cycloalkenyl-, oder Ar-Gruppe gegebenenfalls ersetzt ist durch O, NH, NR₁, S, SO oder SO₂; und, steht für Wasserstoff, geradkettiges (unverzweigtes) oder verzweigtkettiges (C₁-C₄)-Alkyl, geradkettiges (unverzweigtes) oder verzweigtkettiges (C₃-C₄)-Alkenyl oder -Alkinyl oder worin R₁ steht für Y-Z, wie oben definiert; oder
(v)
3-(2-Pyridyl)-1-propyl(2S)-1-(3,3-dimethyl-1,2-dioxo-pentyl)-2-pyrrolidincarboxylat-N-oxid;
3-(3-Pyridyl)-1-propyl(2S)-1-(3,3-dimethyl-1,2-dioxo-pentyl)-2-pyrrolidincarboxylat-N-oxid;
3-(4-Pyndyl)-1-propyl(2S)-1-(3,3-dimethyl-1,2-dioxo-pentyl)-2-pyrrolidincarboxylat-N-oxid;
3-(2-Chinolyl)-1-propyl(2S)-1-(1,1-dimethyl-1,2-dioxo-pentyl)-2-pyrrolidincarboxylat-N-oxid;
3-(3-Chinolyl)-1-propyl(2S)-1-(1,1-dimethyl-1,2-dioxo-pentyl)-2-pyrrolidincarboxylat-N-oxid; und
3-(4-Chinolyl)-1-propyl(2S)-1-(1,1-dimethy)-1,2-dioxo-pentyl)-2-pyrrolidincarboxylat-N-oxid, oder
(j) ein Cycloheximid-Derivat, bei dem es sich handelt um
4-[2-(3,5-Dimethylcyclohexyl-2-oxim)-2-hydroxyethyl]-2,6-piperidindion;
4-[2-(3,5-Dimethyl-2-oxocyclohexyl)-2-acetoxyethyl)-2, 6-piperidindion;
4-[2-(3,5-Dimethyl-2-oxocyclohexyl)-2-(oxycarbonyl-1-adamantylamino)ethyl]-2,6-piperidindion-1-(ethylethanoat);
4-[2-3,5-Dimethyl-2-oxocyclohexyl)-2-hydroxyethyl]-1-methyl-2,6-piperidindion;
4-[2-(3,5-Dimethyl-2-oxoclohexyl)-2-hydroxyethyl]-1-benzyl-2,6-piperidindion;
4-[2-3,5-Dimethyl-2-oxocyclohexyl)-2-hydroxyethyl]-1-(4-cyanobenzyl)-2,6-piperidindion;
4-[2-(3,5-Dimethyl-2-oxocyclohexyl)-2-hydroxyethyl]-2,6-pieridindion-1-(essigsäure-amid);
4-[2-(3,5-Dimethyl-2-oxocyclohexyl)-2-hydroxyethyl]-2,6-piperidindion-1-(4-ethylbutanoat); oder
4-[2-(3,5-Dimethyl-2-oxocyclohexyl)-2-hydroxyethyl]-2,6-piperidindion-1-(ethylethanoat); oder wobei
(k) der Neurophilin-Ligand ist, bei dem es sich handelt um
(S)-N-Benzyl-3-(4-chlorphenyl)-2-(methyl-(2-oxo-2-(3,4,5-trimethoxyphenyl)acetyl)-amino)-N-(3-pyridinyl-4-yl)-4-yl)-1-(2-pyrodinyl-4-yl)-ethyl)propyl)propionamid oder 1-(3,3-Dimethyl-1,2-dioxopentyl)-(L)-prolin-3-(3-pyridinyl)propylester.

2. Verwendung eines Neurophilin-Liganden, der keine Immunosuppressant-Aktivität aufweist, für die Herstellung eines Arzneimittels zur Herabsetzung des Augeninnendrucks (IOP) bei einem Säugetier, das unter einem erhöhten Augeninnendruck (IOP) leidet, wobei der Neurophilin-Ligand eine Verbindung nach Anspruch 1 ist.

3. Verwendung eines Neurophilin-Liganden, der keine Immunosuppressant-Aktivität aufweist, für die Herstellung eines Arzneimittels zur Verhinderung eines Gesichtsfeldausfalls, der im Zusammenhang steht mit einem Glaucom, wobei der Neurophilin-Ligand eine Verbindung nach Anspruch 1 ist.

4. Verwendung eines Neurophilin-Liganden, der keine Immunosuppressant-Aktivität aufweist, für die Herstellung eines Arzneimittels zur Herabsetzung des Augeninnendrucks (IOP) und zur Erzielung einer eines Schutzes der Nervenzellen (Neuronen) in einem Säugetier, das unter einem erhöhten Augeninnendruck (IOP) leidet, wobei der Neurophilin-Ligand eine Verbindung nach Anspruch 1 ist.

## Revendications

1. Utilisation d'un ligand de neurophiline qui ne présente pas une activité immunosuppressive, pour la fabrication d'un médicament destiné au traitement d'un mammifère souffrant d'un glaucome, dans laquelle le ligand de neurophiline est
(a) un dérivé de l'acide pipécolique, qui est le 1-(3,3-diméthyl-2-oxopentanoyl)-2-pipéridinecarboxylate de (E)-3-(3,4-dichlorophényl)-2-propényle ; le 1-(3,3-diméthyl-2-oxopentanoyl)-2-pipéridinecarboxylate de (E)-3-(3,4,5-triméthoxyphényl)-2-propényle ; le 1-(3,3-diméthyl-2-oxopentanoyl)-2-pipéridinecarboxylate de (E)-3-phényl-2-propényle ; le 1-(3,3-diméthyl-2-oxopentanoyl)-2-pipéridinecarboxylate de (E)-3-((3-(2,5-diméthoxy)phénylpropyl)phényl)-2-propényle ; le 1-(3,3-diméthyl-2-oxopentanoyl)-2-pipéridinecarboxylate de (E)-3-(1,3-benzodioxol-5-yl)-2-propényle ; le 1-(2-oxo-2-phénylacétyl)-2-pipéridinecarboxylate de 4-(4-méthoxyphényl)butyle ; le 1-(2-oxo-2-phénylacétyl)-2-pipéridinecarboxylate de 3-phénylpropyle ; le 1-(2-oxo-2-phénylacétyl)-2-pipéridinecarboxylate de 3-(3-pyridyl)propyle ; le 1-(3,3-diméthyl-2-oxopentanoyl)-2-pipéridinecarboxylate de 3-(3-pyridyl)propyle ; le 1-(3,3-diméthyl-2-oxopentanoyl)-2-pipéridinecarboxylate de 4-phényl-1-(3-phénylpropyl)butyle ; le 1-(3,3-diméthyl-2-oxopentanoyl)-2-pipéridinecarboxylate de 4-(4-méthoxyphényl)butyle ; le 1-(3,3-diméthyl-2-oxopentanoyl)-2-pipéridinecarboxylate de 1-(4-méthoxyphénéthyl)-4-phénylbutyle ; le 1-(3,3-diméthyl-2-oxopentanoyl)-2-pipéridinecarboxylate de 3-(2,5-diméthoxyphényl)propyle ; le 1-(3,3-diméthyl-2-oxopentanoyl)-2-pipéridinecarboxylate de 3-(1,3-benzodioxol-5-yl)propyle ; le 1-(3,3-diméthyl-2-oxopentanoyl)-2-pipéridinecarboxylate de 1-phénéthyl-3-phénylpropyle ; le 1-(2-cyclohexyl-2-oxoacétyl)-2-pipéridinecarboxylate de 4-(4-méthoxyphényl)butyle ; le 1-(2-cyclohexyl-2-oxoacétyl)-2-pipéridinecarboxylate de 3-cyclohexylpropyle ; le 1-(2-cyclohexyl-2-oxoacétyl)-2-pipéridinecarboxylate de 3-phénylpropyle ; le 1-(3,3-diméthyl-2-oxobutanoyl)-2-pipéridinecarboxylate de 3-cyclohexylpropyle ; le 1-(3,3-diméthyl-2-oxobutanoyl)-2-pipéridinecarboxylate de 3-phénylpropyle ; le 1-(3,3-diméthyl-2-oxobutanoyl)-2-pipéridinecarboxylate de 4-(4-méthoxyphényl)butyle ; ou le 1-(3,3-diméthyl-2-oxobutanoyl)-2-pipéridinecarboxylate de 4-phényl-1-(3-phénylpropyl)butyle,
(b) un dérivé de la proline, qui est
(i) un composé de formule : 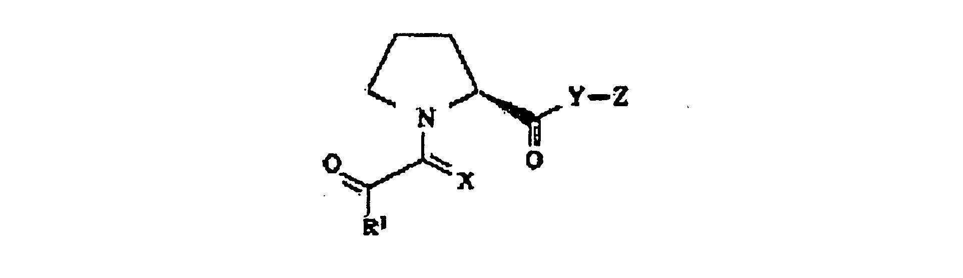 dans laquelle R₁ est un groupement alkyle ou alcényle en C₁₋Cg à chaîne droite ou ramifiée, éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où AR₁ est choisi parmi le groupe constitué de 1-naphtyle, 2-naphtyle, 2-indolyle, 3-indolyle, 2-furyle, 3-furyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ à chaîne droite ou ramifiée, alcoxy en C₁₋C₄ ou alcényloxy en C₁-C₄, phénoxy, benzyloxy et amino ;
X est choisi parmi le groupe constitué d'oxygène et de soufre ;
Y est oxygène ou NR₂, où R₂ est hydrogène, alkyle en C₁-C₆; et
Z est alkyle ou alcényle en C₂-C₆ à chaîne droite ou ramifiée, où la chaîne alkyle est substituée en une ou plusieurs positions par Ar₁ tel que défini ci-dessus, cycloalkyle en C₃-C₈, cycloalkyle relié par une chaîne alkyle ou alcényle en C₁-C₆ droite ou non ramifiée, ou Ar₂, où Ar₂ est choisi parmi le groupe constitué de 2-indolyle, 3-indolyle, 2-furyle, 3-furyle, 2-thiazolyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcoxy en C₁-C₄ ou alcényloxy en C₁-C₄, phénoxy, benzyloxy et amino ;
Z peut également être le fragment : 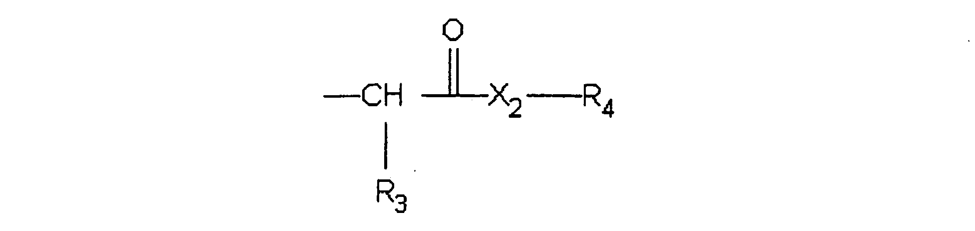 où R₃ est choisi parmi le groupe constitué d'alkyle en C₁₋C₈ linéaire ou ramifié, éventuellement substitué par cycloalkyle en C₃-C₈ ou Ar₁ tel que défini ci-dessus ;
X₂ est O ou NR₅, où R₅ est choisi parmi le groupe constitué d'hydrogène, alkyle et alcényle en C₁-C₆ linéaire ou ramifié ;
R₄ est choisi parmi le groupe constitué de phényle, benzyle, alkyle ou alcényle en C₁-C₅ linéaire ou ramifié et alkyle ou alcényle en C₁-C₅ linéaire ou ramifié substitué par phényle ; ou des sels ou des hydrates pharmaceutiquement acceptables de celui-ci ; y compris les composés
(2S)-1-(3,3-diméthyl-1,2-dioxopentyl)-2-pyrrolidinecarboxylate de 3-phényl-1-propyle, (2S)-1-(3,3-diméthyl-1,2-dioxopentyl)-2-pyrrolidinecarboxylate de 3-phényl-1-prop-2-(E)-ényle,
(2S)-1-(3,3-diméthyl-1,2-dioxopentyl)-2-pyrrolidinecarboxylate de 3-(3,4,5-triméthoxyphényl)-1-propyle,
(2S)-1-(3,3-diméthyl-1,2-dioxopentyl)-2-pyrrolidinecarboxylate de 3-(3,4,5-triméthoxyphényl)-1-prop-2-(E)-ényle,
(2S)-1-(3,3-diméthyl-1,2-dioxopentyl)-2-pyrrolidinecarboxylate de 3-(4,5-dichlorophényl)-1-propyle,
(2S)-1-(3,3-diméthyl-1,2-dioxopentyl)-2-pyrrolidinecarboxylate de 3-(4,5-dichlorophényl)-1-prop-2-(E)-ényle,
(2S)-1-(3,3-diméthyl-1,2-dioxopentyl)-2-pyrrolidinecarboxylate de 3-(4,5-méthylènedioxyphényl)-1-propyle,
(2S)-1-(3,3-diméthyl-1,2-dioxopentyl)-2-pyrrolidinecarboxylate de 3-(4,5-méthylènedioxyphényl)-1-prop-2-(E)-ényle,
(2S)-1-(3,3-diméthyl-1,2-dioxopentyl)-2-pyrrolidinecarboxylate de 3-cyclohexyl-1-propyle,
(2S)-1-(3,3-diméthyl-1,2-dioxopentyl)-2-pyrrolidinecarboxylate de 3-cyclohexyl-1-prop-2-(E)-ényle, (2S)-1-(3,3-diméthyl-1,2-dioxopentyl)-2-pyrrolidinecarboxylate de (1R)-1,3-diphényl-1-propyle,
(2S)-1-(3,3-diméthyl-1,2-dioxopentyl)-2-pyrrolidinecarboxylate de (1R)-1,3-diphényl-1-prop-2-(E)-ényle,
(2S)-1-(3,3-diméthyl-1,2-dioxopentyl)-2-pyrrolidinecarboxylate de (1R)-1-cyclohexyl-3-phényl-1-propyle,
(2S)-1-(3,3-diméthyl-1,2-dioxopentyl)-2-pyrrolidinecarboxylate de (1R)-1-cyclohexyl-3-phényl-1-prop-2-(E)-ényle,
(2S)-1-(3,3-diméthyl-1,2-dioxopentyl)-2-pyrrolidinecarboxylate de (1R)-1-(4,5-dichlorophényl)-3-phényl-1-propyle,
(2S)-1-(1,2-dioxo-2-cyclohexyl)éthyl-2-pyrrolidinecarboxylate de 3-phényl-1-propyle,
(2S)-1-(1,2-dioxo-4-cyclohexyl)butyl-2-pyrrolidinecarboxylate de 3-phényl-1-propyle,
(2S)-1-(1,2-dioxo-2-[2-furanyl])éthyl-2-pyrrolidinecarboxylate de 3-phényl-1-propyle,
(2S)-1-(1,2-dioxo-2-[2-thiényl])éthyl-2-pyrrolidinecarboxylate de 3-phényl-1-propyle,
(2S)-1-(1,2-dioxo-2-[2-thiazolyl])éthyl-2-pyrrolidinecarboxylate de 3-phényl-1-propyle,
(2S)-1-(1,2-dioxo-2-phényl)éthyl-2-pyrrolidinecarboxylate de 3-phényl-1-propyle,
(2S)-1-(3,3-diméthyl-1,2-dioxopentyl)-2-pyrrolidinecarboxylate de 1,7-diphényl-4-heptyle,
(2S)-1-(3,3-diméthyl-1,2-dioxo-4-hydroxybutyl)-2-pyrrolidinecarboxylate de 3-phényl-1-propyle,
3-phényl-1-propyl-(2S)-1-(3,3-diméthyl-1,2-dioxopentyl)-2-pyrrolidinecarboxamide,
ester éthylique de [1-(3,3-diméthyl-1,2-dioxopentyl)-L-proline]-L-phénylalanine,
ester éthylique de 1-[1-(3,3-diméthyl-1,2-dioxopentyl)-L-proline]-L-leucine,
ester éthylique de 1-[1-(3,3-diméthyl-1,2-dioxopentyl)-L-proline]-L-phénylglycine,
ester phénylique de 1-[1-(3,3-diméthyl-1,2-dioxopentyl)-L-proline]-L-phénylalanine,
ester benzylique de 1-[1-(3,3-diméthyl-1,2-dioxopentyl)-L-proline]-L-phénylalanine, et
ester éthylique de 1-[1-(3,3-diméthyl-1,2-dioxopentyl)-L-proline]-L-isoleucine ; ou
(ii) un composé de formule : 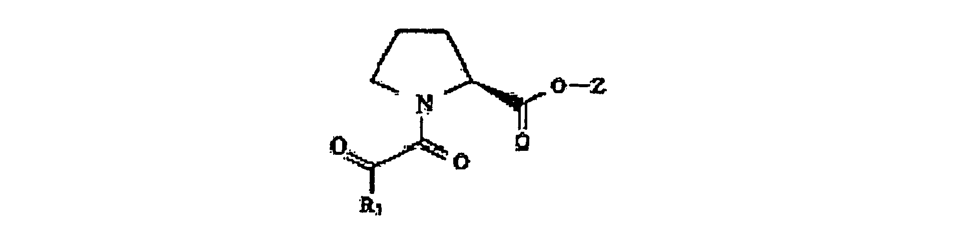 dans laquelle R₁ est un groupement alkyle ou alcényle en C₁₋Cg à chaîne droite ou ramifiée, éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 2-indolyle, 3-indolyle, 2-furyle, 3-furyle, 2-thiazolyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcoxy en C₁-C₄ ou alcényloxy en C₁-C₄, phénoxy, benzyloxy et amino ; est un alkyle ou un alcényle en C₂-C₆ à chaîne droite ou ramifiée, où la chaîne alkyle est substituée en une ou plusieurs positions par Ar₁ tel que défini ci-dessus, cycloalkyle en C₁-C₈, cycloalkyle relié par une chaîne alkyle ou alcényle en C₁-C₆ droite ou non ramifiée, ou Ar₂, où Ar₂ est choisi parmi le groupe constitué de 2-indolyle, 3-indolyle, 2-furyle, 3-furyle, 2-thiazolyle, 2-thiényle, 3-thiényle, 2-, 3- ou 4-pyridyle ou phényle, ayant un à
trois substituants choisis indépendamment parmi le groupe constitué d'hydrogène, halogéno, hydroxyle, nitro, trifluorométhyle, alkyle ou alcényle en C₁-C₆ linéaire ou ramifié, alcoxy en C₁-C₄ ou alcényloxy en C₁-C₄, phénoxy, benzyloxy et amino ; ou des sels ou des hydrates pharmaceutiquement acceptables de celui-ci ; ou
(iii) un composé de formule : 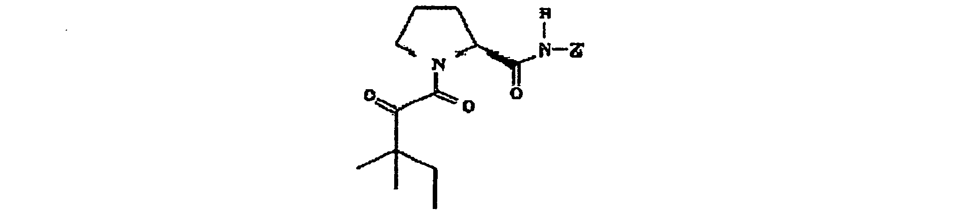 où Z' est le fragment : 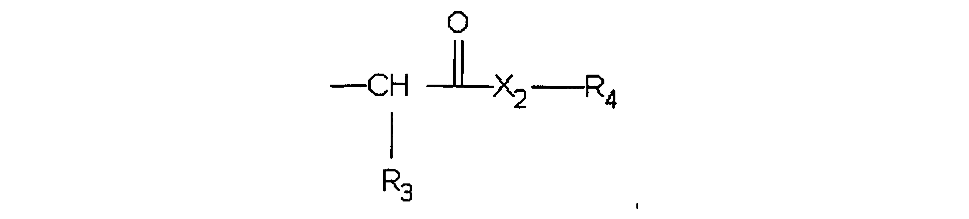 où R₃ est choisi parmi le groupe constitué d'alkyle en C₁-C₈ linéaire ou ramifié, éventuellement substitué par cycloalkyle en C₃-C₈, ou Ar₁ tel que défini ci-dessus, et Ar₁ non substitué ;
X₂ est O ou NR₅, où R₅ est choisi parmi le groupe constitué d'hydrogène, alkyle et alcényle en C₁-C₆ linéaire ou ramifié ;
R₄ est choisi parmi le groupe constitué de phényle, benzyle, alkyle ou alcényle en C₁-C₅ linéaire ou ramifié et alkyle ou alcényle en C₁-C₅ linéaire ou ramifié substitué par phényle ; ou des sels ou des hydrates pharmaceutiquement acceptables de celui-ci ; (c) un dérivé de pipécolyle ou de prolyle N-sulfonylé, qui est 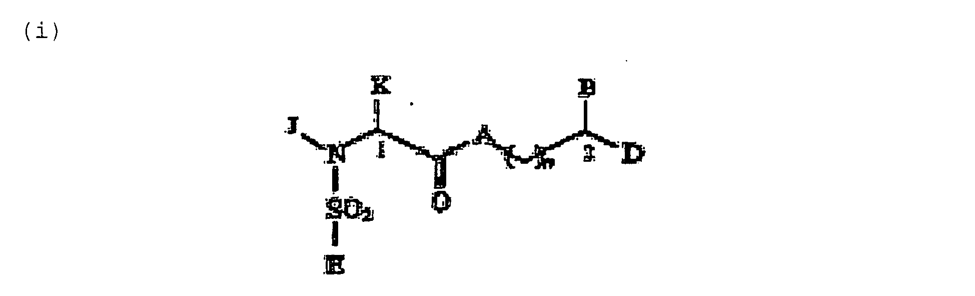 ou un sel pharmaceutiquement acceptable de celui-ci, où A est CH₂, un oxygène, NH ou N--(alkyle en C₁-C₅) ;
B et D sont indépendamment Ar, hydrogène, alkyle en (C1-C6) linéaire ou ramifié, alcényle en (C1-C6) linéaire ou ramifié, alkyle ou alcényle en (C1-C6) linéaire ou ramifié qui est substitué par cycloalkyle en (C5-C7), alkyle ou alcényle en (C1-C6) linéaire ou ramifié qui est substitué par cycloalcényle en (C5-C7) ou alkyle ou un alcényle en (C1-C6) linéaire ou ramifié substitué par Ar, où, dans chaque cas, un ou deux des groupements CH₂ des chaînes alkyle ou alcényle peut/peuvent contenir 1-2 hétéroatomes choisis parmi le groupe constitué d'oxygène, de soufre, de SO et de SO₂, selon des motifs de substitution chimiquement raisonnables, ou 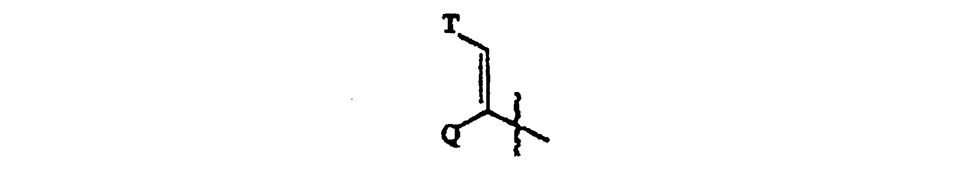 à condition que B et D ne soient ni l'un ni l'autre hydrogène; Q soit hydrogène, alkyle en (C1-C6) linéaire ou ramifié ou alcényle en (C1-C6) linéaire ou ramifié ;
T est Ar ou cycloalkyle à 5-7 chaînons substitué par des substituants en positions 3 et 4 qui sont choisis indépendamment parmi le groupe constitué d'hydrogène, d'hydroxyle, de O--alkyle en (C1-C4), de O--alcényle en (C1-C4) et de carbonyle ;
Ar est choisi parmi le groupe constitué de phényle, 1-naphtyle, 2-naphtyle, 2-furyle, 3-furyle, 2-thiényle, 3-thiényle, 2-pyridyle, 3-pyridyle, 4-pyridyle, des systèmes de cycle hétérocyclique monocyclique et bicyclique avec des tailles de cycle individuelles de 5 ou 6 qui peuvent contenir dans l'un ou l'autre des cycles, ou les deux, un total de 1-4 hétéroatomes choisis indépendamment parmi 0, N et S ; où Ar peut contenir un à trois substituants choisis indépendamment parmi le groupe constitué d'hydrogène, halogéno, hydroxyle, nitro, trifluorométhyle, trifluorométhoxy, alkyle en (C1-C6) linéaire ou ramifié, alcényle en (C2-C6) linéaire ou ramifié, O--alkyle en (C1-C4) linéaire ou ramifié, 0-alcényle en (C2-C4) linéaire ou ramifié, O-benzyle, 0-phényle, 1,2-méthylènedioxy, amino, carboxyle et phényle ;
E est alkyle en (C1-C6) linéaire ou ramifié, alcényle en (C1-C6) linéaire ou ramifié, cycloalkyle en (C5-C7), cycloalcényle en (C5-C7) substitué par alkyle en (C1-C4) linéaire ou ramifié ou alcényle en (C1-C4) linéaire ou ramifié, [alkyle en (C2-C4) ou alcényle en (C2-C4)]-Ar ou Ar ;
J est hydrogène ou alkyle en C1 ou C2 ou benzyle ; K est alkyle en (C1-C4) linéaire ou ramifié, benzyle ou cyclohexylméthyle ; ou où J et K peuvent être pris ensemble pour former un cycle hétérocyclique à 5-7 chaînons pouvant y contenir un substituant oxygène, soufre, SO ou SO₂ ;
n vaut de 0 à 3 ; et
la stéréochimie aux positions 1 et 2 du carbone est R ou S ; ou 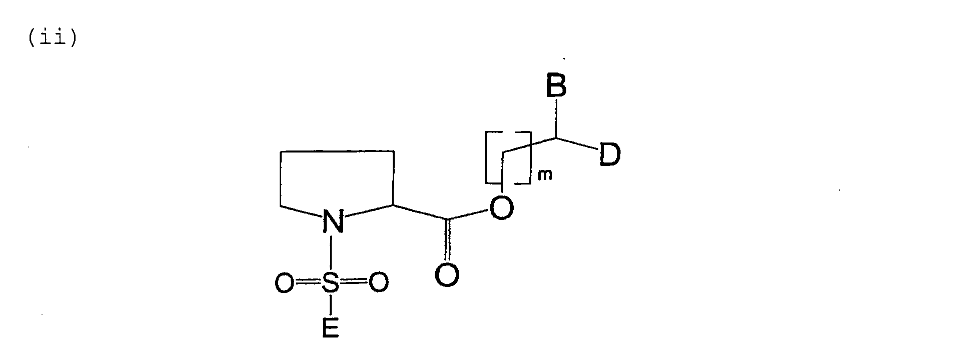 ou un sel pharmaceutiquement acceptable de celui-ci, où : B et D sont indépendamment Ar, hydrogène, alkyle en (C1-C6) linéaire ou ramifié, alcényle en (C1-C6) linéaire ou ramifié, alkyle ou alcényle en (C1-C6) linéaire ou ramifié qui est substitué par un cycloalkyle en (C5-C7), alkyle ou alcényle en (C1-C6) linéaire ou ramifié qui est substitué par un cycloalcényle en (C5-C7) ou alkyle ou alcényle en (C1-C6) linéaire ou ramifié substitué par Ar, où, dans chaque cas, un ou deux des groupements CH₂ des chaînes alkyle ou alcényle peut/peuvent contenir 1-2 hétéroatomes choisis parmi le groupe constitué d'oxygène, de soufre, de SO et de SO₂, selon des motifs de substitution chimiquement raisonnables, ou 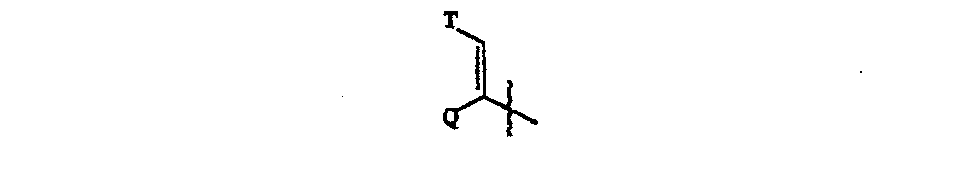 à condition que B et D ne soient ni l'un ni l'autre hydrogène ;
Q soit hydrogène, alkyle en (C1-C6) linéaire ou ramifié ou alcényle en (C1-C6) linéaire ou ramifié ;
T est Ar ou cycloalkyle à 5-7chaînons substitué par des substituants en positions 3 et 4 qui sont choisis indépendamment parmi le groupe constitué d'hydrogène, d'hydroxyle, de O--alkyle en (C1-C4), de O--alcényle en (C1-C4) et de carbonyle ;
Ar est choisi parmi le groupe constitué de phényle, 1-naphtyle, 2-naphtyle, 2-furyle, 3-furyle, 2-thiényle, 3-thiényle, 2-pyridyle, 3-pyridyle, 4-pyridyle, des systèmes de cycle hétérocyclique monocyclique et bicyclique avec des tailles de cycle individuelles de 5 ou 6 qui peuvent contenir dans l'un ou l'autre des cycles, ou les deux, un total de 1-4 hétéroatomes choisis indépendamment parmi 0, N et S ; où Ar peut contenir un à trois substituants choisis indépendamment parmi le groupe constitué d'hydrogène, halogéno, hydroxyle, nitro, trifluorométhyle, trifluorométhoxy, alkyle en (C1-C6) linéaire ou ramifié, alcényle en (C2-C6) linéaire ou ramifié, O--alkyle en (C1-C4) linéaire ou ramifié, 0-alcényle en (C2-C4) linéaire ou ramifié, O-benzyle, 0-phényle, 1,2-méthylènedioxy, amino, carboxyle et phényle ;
E est alkyle en (C1-C6) linéaire ou ramifié, alcényle en (C1-C6) linéaire ou ramifié, cycloalkyle en (C5-C7), cycloalcényle en (C5-C7) substitué par alkyle en (C1-C4) linéaire ou ramifié ou alcényle en (C1-C4) linéaire ou ramifié, [alkyle en (C2-C4) ou alcényle en (C2-C4)]-Ar ou Ar ; et m vaut de 0 à 3 ; ou 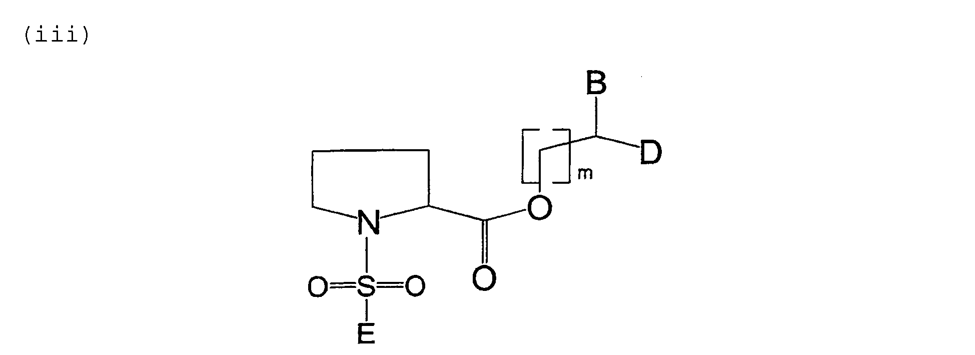 ou un sel pharmaceutiquement acceptable de celui-ci, où : B et D sont indépendamment Ar, hydrogène, alkyle en (C1-C6) linéaire ou ramifié, alcényle en (C1-C6) linéaire ou ramifié, alkyle ou alcényle en (C1-C6) linéaire ou ramifié qui est substitué par un cycloalkyle en (C5-C7), alkyle ou alcényle en (C1-C6) linéaire ou ramifié qui est substitué par un cycloalcényle en (C5-C7) ou alkyle ou alcényle en (C1-C6) linéaire ou ramifié substitué par Ar, où, dans chaque cas, un ou deux des groupements CH₂ des chaînes alkyle ou alcényle peut/peuvent contenir 1-2 hétéroatomes choisis parmi le groupe constitué d'oxygène, de soufre, de SO et de SO₂, selon des motifs de substitution chimiquement raisonnables, ou 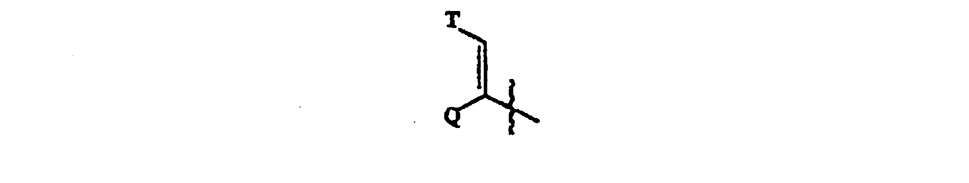 à condition que B et D ne soient ni l'un ni l'autre hydrogène ; Q soit hydrogène, alkyle en (C1-C6) linéaire ou ramifié ou alcényle en (C1-C6) linéaire ou ramifié ;
T est Ar ou cycloalkyle à 5-7 chaînons substitué par des substituants en positions 3 et 4 qui sont choisis indépendamment parmi le groupe constitué d'hydrogène, d'hydroxyle, de 0--alkyle en (C1-C4), de 0--alcényle en (C1-C4) et de carbonyle ;
Ar est choisi parmi le groupe constitué de phényle, 1-naphtyle, 2-naphtyle, 2-furyle, 3-furyle, 2-thiényle, 3-thiényle, 2-pyridyle, 3-pyridyle, 4-pyridyle, des systèmes de cycle hétérocyclique monocyclique et bicyclique avec des tailles de cycle individuelles de 5 ou 6 qui peuvent contenir dans l'un ou l'autre des cycles, ou les deux, un total de 1-4 hétéroatomes choisis indépendamment parmi 0, N et S ; où Ar peut contenir un à trois substituants choisis indépendamment parmi le groupe constitué d'hydrogène, halogéno, hydroxyle, nitro, trifluorométhyle, trifluorométhoxy, alkyle en (C1-C6) linéaire ou ramifié, alcényle en (C2-C6) linéaire ou ramifié, O--alkyle en (C1-C4) linéaire ou ramifié, 0--alcényle en (C2-C4) linéaire ou ramifié, O-benzyle, O-phényle, 1,2-méthylènedioxy, amino, carboxyle et phényle ;
E est alkyle en (C1-C6) linéaire ou ramifié, alcényle en (C1-C6) linéaire ou ramifié, cycloalkyle en (C5-C7), cycloalcényle en (C5-C7) substitué par alkyle en (C1-C4) linéaire ou ramifié ou alcényle en (C1-C4) linéaire ou ramifié, [alkyle en (C2-C4) ou alcényle en (C2-C4)]-Ar ou Ar ; et
m vaut de 0 à 3 ;
(d) un thioester ou une cétone hétérocyclique, qui est 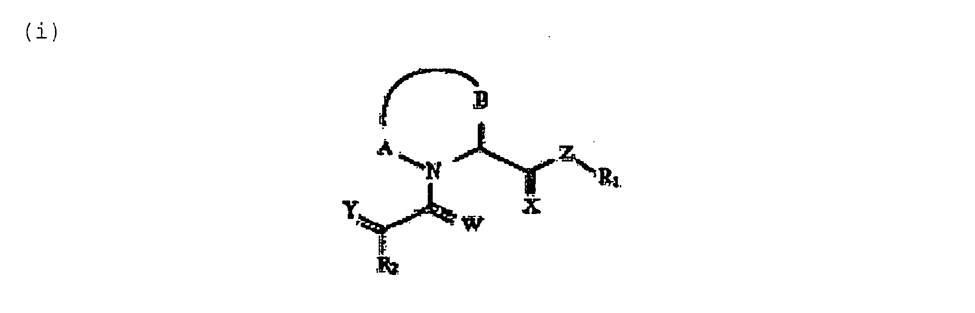 ou un sel pharmaceutiquement acceptable de celui-ci ; où : A et B, conjointement avec les atomes d'azote et de carbone auxquels ils sont respectivement liés, forment un cycle hétérocyclique saturé ou insaturé à 5-7 chaînons contenant une combinaison quelconque de CH₂, 0, S, SO, SO₂, NH ou NR₂ dans un état d'oxydation chimiquement stable quelconque ;
X est soit O, soit S ;
Z est S, CH₂, CHR₁ ou C(R₁)₂ ;
W et Y sont indépendamment 0, S, CH₂ ou H₂ ;
R₁ est alkyle ou alcényle en C₁-C₆ à chaîne droite ou ramifiée, qui est substitué en une ou plusieurs positions par (Ar₁)ₙ, (Ar₁)ₙ relié par un alkyle ou alcényle en C₁-C₆ à chaîne droite ou ramifiée, cycloalkyle en C₃-C₈, cycloalkyle en C₃-C₈ relié par un alkyle ou alcényle en C₁-C₆ à chaîne droite ou ramifiée, Ar₂, ou une combinaison de ceux-ci ;
n vaut 1 ou 2 ;
R₂ est alkyle ou alcényle en C₁-C₉ à chaîne droite ou ramifiée, cycloalkyle en C₃-C₈, cycloalcényle en C₅-C₇ ou
Ar₁, où ledit alkyle, alcényle, cycloalkyle ou cycloalcényle est non substitué ou substitué en une ou plusieurs positions par alkyle ou alcényle en C₁-C₄ à chaîne droite ou ramifiée, hydroxyle, ou une combinaison de ceux-ci ;
Ar₁ et Ar₂ sont indépendamment un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ linéaire ou ramifié, alcoxy en C₁-C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de O, N, S et d'une combinaison de ceux-ci ; ou 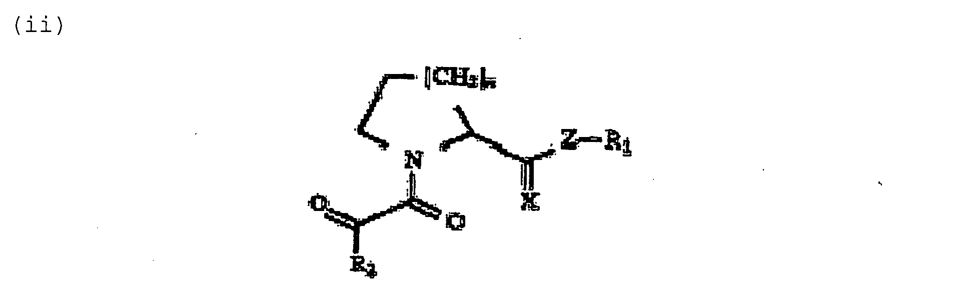
ou un sel pharmaceutiquement acceptable de celui-ci, où : n vaut 1 ou 2 ;
X est 0 ou S ;
Z est S, CH₂, CHR₁ ou C(R₁)₂ ;
R₁ est alkyle ou alcényle en C₁-C₅ à chaîne droite ou ramifiée, qui est substitué en une ou plusieurs positions par (Ar₁)ₙ, (Ar₁)ₙ relié par un alkyle ou alcényle en C₁-C₅ à chaîne droite ou ramifiée, ou une combinaison de ceux-ci ;
R₂ est alkyle ou alcényle en C₁-C₉ à chaîne droite ou ramifiée, cycloalkyle en C₃-C₈, cycloalcényle en C₅-C₇ ou
Ar₁, où ledit alkyle, alcényle, cycloalkyle ou cycloalcényle est non substitué ou substitué en une ou plusieurs positions par alkyle ou alcényle en C₁-C₄ à chaîne droite ou ramifiée, hydroxyle, ou une combinaison de ceux-ci ;
Ar₁ est un cycle carbocyclique ou hétérocyclique monocyclique, bicyclique ou tricyclique, qui est non substitué ou substitué en une à trois positions par halogéno, hydroxyle, nitro, trifluorométhyle, alkyle ou alcényle en C₁-C₆ à chaîne droite ou ramifiée, alcoxy en C₁₋C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de 0, N, S et d'une combinaison de ceux-ci ; ou 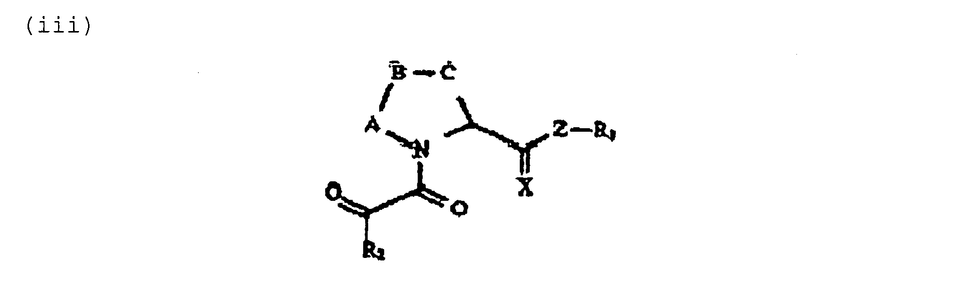 ou un sel pharmaceutiquement acceptable de celui-ci, où :
A, B, C et D sont indépendamment CH₂, 0, S, SO, SO₂, NH ou NR₂ ;
X est 0 ou S ;
Z est S, CH₂, CHR₁ ou C(R₁)₂ ;
R₁ est alkyle ou alcényle en C₁-C₆ à chaîne droite ou ramifiée, qui est substitué en une ou plusieurs positions par (Ar₁)ₙ, (Ar₁)ₙ relié par un alkyle ou alcényle en C₁-C₆ à chaîne droite ou ramifiée, cycloalkyle en C₃-C₈, cycloalkyle en C₃-C₈ relié par un alkyle ou alcényle en C₁-C₆ à chaîne droite ou ramifiée, Ar₂, ou une combinaison de ceux-ci ;
n vaut 1 ou 2 ;
R₂ est alkyle ou alcényle en C₁-C₉ à chaîne droite ou ramifiée, cycloalkyle en C₃-C₈, cycloalcényle en C₅-C₇ ou Ar₁, où ledit alkyle, alcényle, cycloalkyle ou cycloalcényle est non substitué ou substitué en une ou plusieurs positions par alkyle ou alcényle en C₁-C₄ à chaîne droite ou ramifiée, hydroxyle, ou une combinaison de ceux-ci ; et
Ar₁ et Ar₂ sont indépendamment un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁-C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de 0, N, S et d'une combinaison de ceux-ci ; ou 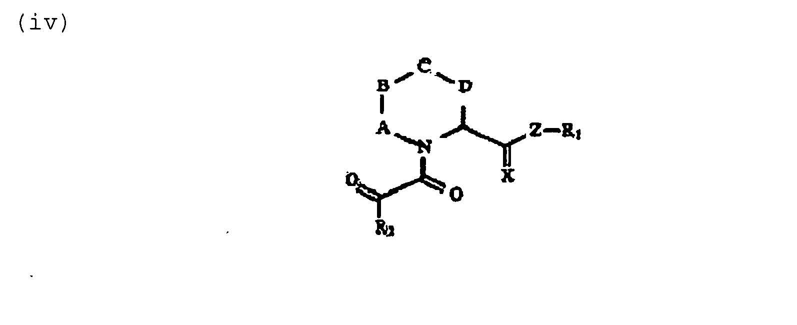 ou un sel pharmaceutiquement acceptable de celui-ci, où : A, B, C et D sont indépendamment CH₂, 0, S, SO, SO₂, NH ou NR₂ ;
X est O ou S ;
Z est S, CH₂, CHR₁ ou C(R₁)₂ ;
R₁ est alkyle ou alcényle en C₁-C₆ à chaîne droite ou ramifiée, qui est substitué en une ou plusieurs positions par (Ar₁)ₙ, (Ar₁)ₙ relié par un alkyle ou alcényle en C₁-C₆ à chaîne droite ou ramifiée, cycloalkyle en C₃-C₈, cycloalkyle en C₃-C₈ relié par un alkyle ou alcényle en C₁-C₆ à chaîne droite ou ramifiée, Ar₂, ou une combinaison de ceux-ci ;
n vaut 1 ou 2 ;
R₂ est alkyle ou alcényle en C₁-C₉ à chaîne droite ou ramifiée, cycloalkyle en C₃-C₈, cycloalcényle en C₅-C₇ ou Ar₁, où ledit alkyle, alcényle, cycloalkyle ou cycloalcényle est non substitué ou substitué en une ou plusieurs positions par alkyle ou alcényle en C₁-C₄ à chaîne droite ou ramifiée, hydroxyle, ou une combinaison de ceux-ci ; et
Ar₁ et Ar₂ sont indépendamment un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁-C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de 0, N, S et d'une combinaison de ceux-ci ; ou
(v)
(2S)-2-(1-oxo-4-phényl)butyl-1-(3,3-diméthyl-1,2-dioxopentyl)pyrrolidine ;
(2S)-2-(1-oxo-4-(3-pyridyl)butyl-1-(3,3-diméthyl-1,2-dioxopentyl)pyrrolidine ;
(2S)-2-(1-oxo-4-phényl)butyl-1-(3,3-diméthyl-1,2-dioxobutyl)pyrrolidine ;
(2S)-2-(1-oxo-4-(3-pyridyl)butyl-1-(3,3-diméthyl-1,2-dioxobutyl)pyrrolidine ;
(2S)-2-(1-oxo-4-(3-pyridyl)butyl-1-(2-cyclohexyl-1,2-dioxoéthyl)pyrrolidine ;
(2S)-2-(1-oxo-4-(3-pyridyl)butyl-1-(2-cyclopentyl-1,2-dioxoéthyl)pyrrolidine ;
(2S)-2-(1-oxo-4-(3-pyridyl)butyl-1-(2-cyclopentyl-1,2-dioxoéthyl)pyrrolidine ;
(2S)-2-(1-oxo-3-(9-fluorényl)propyl-1-(3,3-diméthyl-1,2-dioxopentyl)pyrrolidine ;
(2S)-1-(3,3-diméthyl-1,2-dioxopentyl)-2-pyrrolidinecarboxylate de 2-phényl-1-éthylsulfhydryle ;
(2S)-1-(3,3-diméthyl-1,2-dioxopentyl)-2-pyrrolidinecarboxylate de (3-thioindolyl)méthylsulfhydryle;
(2S)-1-(2-cyclohexyl-1,2-dioxopentyl)-2-pyrrolidinecarboxylate de 2-phényl-1-éthylsulfhydryle ;
(2S)-2-(1-oxo-4-(para-méthoxyphényl)butyl-1-(3,3-diméthyl-1,2-dioxopentyl)pyrrolidine ;
(2R,S)-1-(3,3-diméthyl-1,2-dioxopentyl)-2-pipéridinecarboxylate de 2-phényl-1-éthylsulfhydryle ;
(2R,S)-1-(3,3-diméthyl-1,2-dioxobutyl)-2-pipéridinecarboxylate de 2-phényl-1-éthylsulfhydryle ;
(2R,S)-1-(2-phényl-1,2-dioxoéthyl)-2-pipéridinecarboxylate de 2-phényl-1-éthylsulfhydryle ;
2-(1-oxo-5-(para-méthoxyphényl)pentyl-1-(3,3-diméthyl-1,2-dioxopentyl)pipéridine ;
2-(1-oxo-5-phényl)pentyl-1-(3,3-diméthyl-1,2-dioxopentyl)pipéridine ;
2-(1-oxo-5-phényl)pentyl-1-(2-phényl-1,2-dioxopentyl)pipéridine ; et
2-(1-oxo-5-phényl)pentyl-1-(2-cyclohexyl-1,2-dioxopentyl)pipéridine ; ou
(vi)
(2S)-1-(3,3-diméthyl-1,2-dioxopentyl)-2-pyrrolidinecarboxylate de 2-phényl-1-éthylsulfhydryle ;
(2S)-1-(3,3-diméthyl-1,2-dioxopentyl)-2-pyrrolidinecarboxylate de (3-thioindolyl)méthylsulfhydryle ; ou
(2S)-1-(2-cyclohexyl-1,2-dioxopentyl)-2-pyrrolidinecarboxylate de 2-phényl-1-éthylsulfhydryle ;
(e) un dérivé d'ester N-glyoxyl-propylique, qui est (i) un composé de formule : 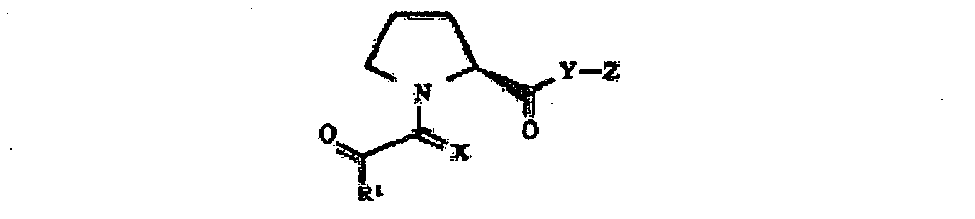 dans laquelle R₁ est un alkyle ou alcényle en C₁-C₉ à chaîne droite ou ramifiée éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 2-indolyle, 3-indolyle, 2-furyle, 3-furyle, 2-thiazolyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ droite ou ramifié, alcoxy en C₁-C₄ ou alcényloxy en C₁-C₄, phénoxy, benzyloxy et amino ;
X est oxygène, soufre, méthylène (CH₂) ou H₂ ;
Y est oxygène ou NR₂, où R₂ est hydrogène ou alkyle en C₁₋C₆ ; et
Z est un alkyle ou alcényle en C₂-C₆ à chaîne droite ou ramifiée, où la chaîne alkyle est substituée en une ou plusieurs positions par Ar₁ tel que défini ci-dessus, cycloalkyle en C₃-C₈, cycloalkyle relié par un alkyle ou alcényle en C₁-C₆ à chaîne droite ou non ramifié ou Ar₂, où Ar₂ est choisi parmi le groupe constitué de 2-indolyle, 3-indolyle, 2-furyle, 3-furyle, 2-thiazolyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcoxy en C₁-C₄ ou alcényloxy en C₁-C₄, phénoxy, benzyloxy et amino ;
Z peut également être le fragment : 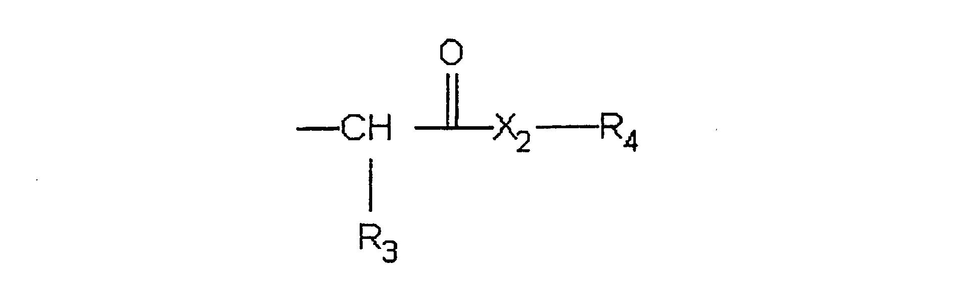 dans laquelle R₃ est choisi parmi le groupe constitué d'alkyle en C₁-C₈ linéaire ou ramifié, éventuellement substitué par cycloalkyle en C₃-C₈ ou Ar₁ tel que défini ci-dessus, et Ar₁ non substitué ;
X₂ est O ou NR₅, où NR₅ est choisi parmi le groupe constitué d'hydrogène, d'alkyle et alcényle en C₁-C₆ linéaire ou ramifié ;
R₄ est choisi parmi le groupe constitué de phényle, benzyle, alkyle ou alcényle en C₁-C₅ linéaire ou ramifié, et alkyle
ou alcényle en C₁-C₅ linéaire ou ramifié substitué par phényle ; ou des sels ou des hydrates pharmaceutiquement acceptables de celui-ci ;
(ii) un composé de formule : 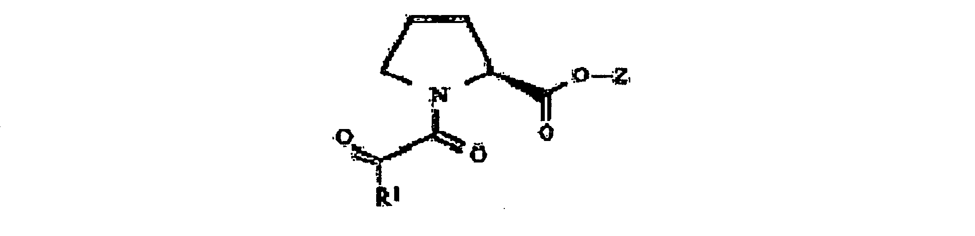 dans laquelle R₁ est un groupement alkyle ou alcényle en C₁-C₉ à chaîne droite ou ramifiée éventuellement substitué par cycloalkyle en C₃-C₆, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 2-indolyle, 3-indolyle, 2-furyle, 3-furyle, 2-thiazolyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcoxy en C₁-C₄ ou alcényloxy en C₁-C₄, phénoxy, benzyloxy et amino ;
Z est un alkyle ou alcényle en C₂-C₆ à chaîne droite ou ramifiée, où la chaîne alkyle est substituée en une ou plusieurs positions par Ar₁ tel que défini ci-dessus, cycloalkyle en C₃-C₈, cycloalkyle relié par une chaîne alkyle ou alcényle en C₁-C₆ linéaire ou non ramifiée ou Ar₂, où Ar₂ est choisi parmi le groupe constitué de 2-indolyle, 3-indolyle, 2-furyle, 3-furyle, 2-thiazolyle, 2-thiényle, 3-thiényle, 2-, 3- ou 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcoxy en C₅-C₄ ou alcényloxy en C₁-C₄, phénoxy, benzyloxy et amino ; ou des sels ou des hydrates pharmaceutiquement acceptables de celui-ci ; ou
(iii) un composé de formula : 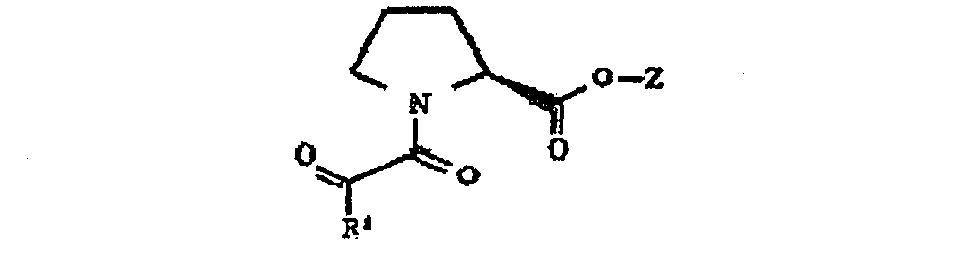 dans laquelle R₁ est un groupement alkyle ou alcényle en C₁₋C₅ à chaîne droite ou ramifiée éventuellement substitué par cycloalkyle en C₃ à C₆ ou Ar₁, où Ar₁ est choisi parmi le groupe constitué de 2-furyle, 2-thiényle ou phényle ;
X est choisi parmi le groupe constitué d'oxygène et de soufre ;
Y est oxygène ; et
Z est un alkyle ou alcényle à chaîne droite ou ramifiée, où la chaîne alkyle est substituée en une ou plusieurs positions par Ar₁ tel que défini ci-dessus, cycloalkyle en C₃-C₆, Ar₂, où Ar₂ est choisi parmi le groupe constitué de 2-, 3- ou 4-pyridyle, ou phényle, ayant un à trois substituants choisis indépendamment parmi le groupe constitué d'hydrogène et d'alcoxy en C₁-C₄; ou
(iv) (2S)-1-(3,3-diméthyl-1,2-dioxopentyl)-2-pyrrolidinecarboxylate de 3-phényl-1-propyle,
(2S)-1-(3,3-diméthyl-1,2-dioxopentyl)-2-pyrrolidinecarboxylate de 3-phényl-1-prop-2-(E)-ényle,
(2S)-1-(3,3-diméthyl-1,2-dioxopentyl)-2-pyrrolidinecarboxylate de 3-(3,4,5-triméthoxyphényl)-1-propyle,
(2S)-1-(3,3-diméthyl-1,2-dioxopentyl)-2-pyrrolidinecarboxylate de 3-(3,4,5-triméthoxyphényl)-1-prop-2-(E)-ényle,
(2S)-1-(3,3-diméthyl-1,2-dioxopentyl)-2-pyrrolidinecarboxylate de 3-(4,5-méthylènedioxyphényl)-1-propyle,
(2S)-1-(3,3-diméthyl-1,2-dioxopentyl)-2-pyrrolidinecarboxylate de 3-(4,5-méthylènedioxyphényl)-1-prop-2-(E)-ényle,
(2S)-1-(3,3-diméthyl-1,2-dioxopentyl)-2-pyrrolidinecarboxylate de 3-cyclohexyl-1-propyle,
(2S)-1-(3,3-diméthyl-1,2-dioxopentyl)-2-pyrrolidinecarboxylate de 3-cyclohexyl-1-prop-2-(E)-ényle,
(2S)-1-(3,3-diméthyl-1,2-dioxopentyl)-2-pyrrolidinecarboxylate de (1R)-1,3-diphényl-1-propyle,
(2S)-1-(1,2-dioxo-2-[2-furanyl])éthyl-2-pyrrolidinecarboxylate de 3-phényl-1-propyle,
(2S)-1-(1,2-dioxo-2-[2-thiényl])éthyl-2-pyrrolidinecarboxylate de 3-phényl-1-propyle,
(2S)-1-(1,2-dioxo-2-[2-thiazolyl])éthyl-2-pyrrolidinecarboxylate de 3-phényl-1-propyle,
(2S)-1-(1,2-dioxo-2-phényl)éthyl-2-pyrrolidinecarboxylate de 3-phényl-1-propyle,
(2S)-1-(3,3-diméthyl-1,2-dioxopentyl)-2-pyrrolidinecarboxylate de 3-(2,5-diméthoxyphényl)-1-propyle,
(2S)-1-(3,3-diméthyl-1,2-dioxopentyl)-2-pyrrolidinecarboxylate de 3-(2,5-diméthoxyphényl)-1-prop-2-(E)-ényle,
(2S)-1-(3,3-diméthyl-1,2-dioxopentyl)-2-pyrrolidinecarboxylate de 2-(3,4,5-triméthoxyphényl)-1-éthyle,
(2S)-1-(3,3-diméthyl-1,2-dioxopentyl)-2-pyrrolidinecarboxylate de 3-(3-pyridyl)-1-propyle,
(2S)-1-(3,3-diméthyl-1,2-dioxopentyl)-2-pyrrolidinecarboxylate de 3-(2-pyridyl)-1-propyle,
(2S)-1-(3,3-diméthyl-1,2-dioxopentyl)-2-pyrrolidinecarboxylate de 3-(4-pyridyl)-1-propyle,
(2S)-I-(2-cyclohexyl-1,2-dioxoéthyl)-2-pyrrolidinecarboxylate de 3-phényl-1-propyle,
(2S)-1-(2-tert-butyl-1,2-dioxoéthyl)-2-pyrrolidinecarboxylate de 3-phényl-1-propyle,
(2S)-1-(2-cyclohexyléthyl-1,2-dioxoéthyl)-2-pyrrolidinecarboxylate de 3-phényl-1-propyle,
(2S)-1-(2-cyclohexyl-1,2-dioxoéthyl)-2-pyrrolidinecarboxylate de 3-(3-pyridyl)-1-propyle,
(2S)-1-(2-tert-butyl-1,2-dioxoéthyl)-2-pyrrolidinecarboxylate de 3-phényl-1-propyle,
(2S)-1-(3,3-diméthyl-1,2-dioxopentyl)-2-pyrrolidinecarboxylate de 3,3-diphényl-1-propyle,
(2S)-I-(2-cyclohexyl-1,2-dioxoéthyl)-2-pyrrolidinecarboxylate de 3-(3-pyridyl)-1-propyle, (2S)-N-([2-thiényl]glyoxyl)pyrrolidinecarboxylate de 3-(3-pyridyl)-1-propyle,
(2S)-1-(3,3-diméthyl-1,2-dioxobutyl)-2-pyrrolidinecarboxylate de 3,3-diphényl-1-propyle, (2S)-1-cyclohexylglyoxyl-2-pyrrolidinecarboxylate de 3,3-diphényl-1-propyle,
(2S)-1-(2-thiényl)glyoxyl-2-pyrrolidinecarboxylate de 3,3-diphényl-1-propyle,
(2S)-1-(3,3-diméthyl-1,2-dioxopentyl)-2-pyrrolidinecarboxylate de 3-(2,5-diméthoxyphényl)-1-propyle,
(2S)-1-(3,3-diméthyl-1,2-dioxopentyl)-2-pyrrolidinecarboxylate de 3-(2,5-diméthoxyphényl)-1-prop-2-(E)-ényle,
(2S)-1-(3,3-diméthyl-1,2-dioxopentyl)-2-pyrrolidinecarboxylate de 2-(3,4,5-triméthoxyphényl)-1-éthyle,
(2S)-1-(3,3-diméthyl-1,2-dioxopentyl)-2-pyrrolidinecarboxylate de 3-(3-pyridyl)-1-propyle,
(2S)-1-(3,3-diméthyl-1,2-dioxopentyl)-2-pyrrolidinecarboxylate de 3-(2-pyridyl)-1-propyle,
(2S)-1-(3,3-diméthyl-1,2-dioxopentyl) -2-pyrrolidinecarboxylate de 3-(4-pyridyl)-1-propyle,
(2S)-1-(2-tert-butyl-1,2-dioxoéthyl)-2-pyrrolidinecarboxylate de 3-phényl-1-propyle,
(2S)-1-(2-cyclohexyléthyl-1,2-dioxoéthyl)-2-pyrrolidinecarboxylate de 3-phényl-1-propyle,
(2S)-1-(2-cyclohexyléthyl-1,2-dioxoéthyl)-2-pyrrolidinecarboxylate de 3-(3-pyridyl)-1-propyle,
(2S)-I-(2-tert-butyl-1,2-dioxoéthyl)-2-pyrrolidinecarboxylate de 3-(3-pyridyl)-1-propyle,
(2S)-1-(3,3-diméthyl-1,2-dioxopentyl)-2-pyrrolidinecarboxylate de 3,3-diphényl-1-propyle,
(2S)-I-(2-cyclohexyl-1,2-dioxoéthyl)-2-pyrrolidinecarboxylate de 3-(3-pyridyl)-1-propyle, (2S)-N-([2-thiényl]glyoxyl)pyrrolidinecarboxylate de 3-(3-pyridyl)-1-propyle,
(25)-1-(3,3-diméthyl-1,2-dioxobutyl)-2-pyrrolidinecarboxylate de 3,3-diphényl-i-propyle, (2S)-i-cyclohexylglyoxyl-2-pyrrolidinecarboxylate de 3,3-diphényl-1-propyle, et
(2S)-I-(2-thiényl)glyoxyl-2-pyrrolidinecarboxylate de 3,3-diphényl-1-propyle,
(f) un dérivé de l'acide pipécolique, qui est 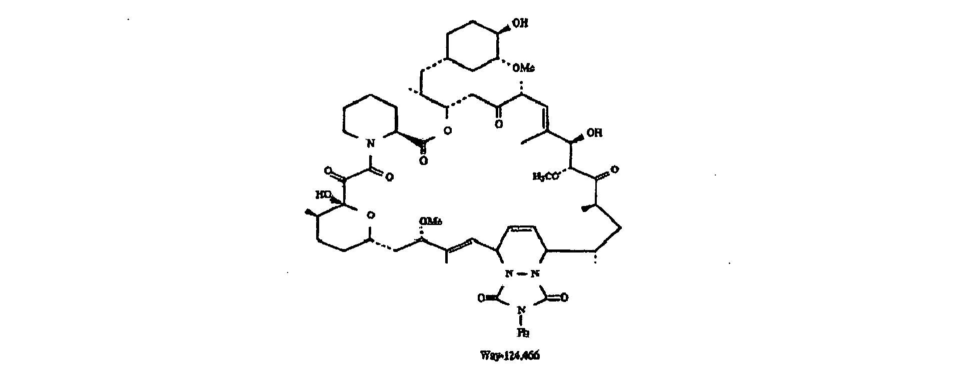 ou 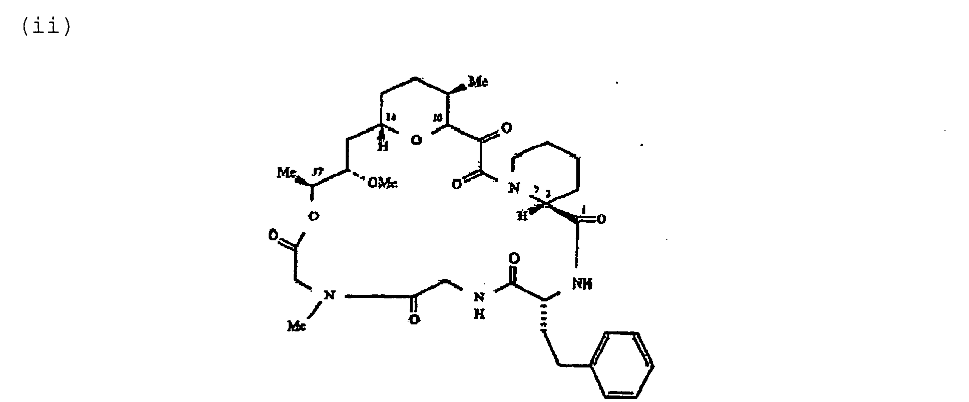 ou 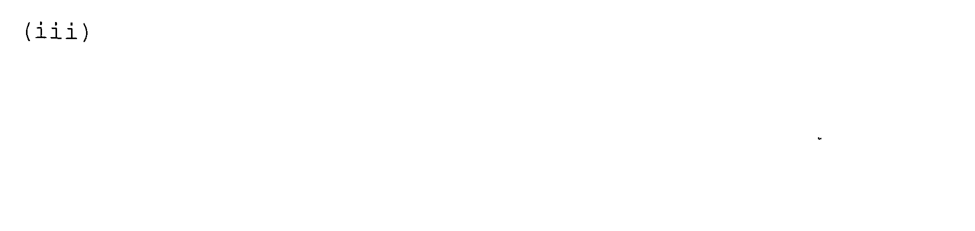 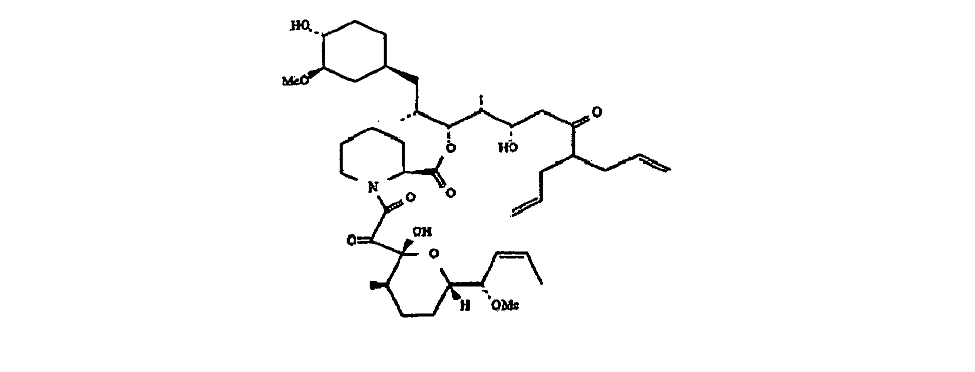 ou
(iv)
(2S)-1-[2-(3,4,5-triméthoxyphényl)acryloyl]hexahydro-2-pyridinecarboxylate de 4-(4-méthoxyphényl)butyle,
(2S)-1-[2-(3,4,5-triméthoxyphényl)propanoyl]hexahydro-2-pyridinecarboxylate de 4-(4-méthoxyphényl)butyle,
(2S)-1-[2-oxo-2-(3,4,5-triméthoxyphényl)acétyl]hexahydro-2-pyridinecarboxylate de 4-(4-méthoxyphényl)butyle,
(2S)-1-(3,3-diméthyl-2-oxopentanoyl)hexahydro-2-pyridinecarboxylate de 3-cyclohexylpropyle,
(2S)-1-(3,3-diméthyl-2-oxopentanoyl)hexahydro-2-pyridinecarboxylate de 3-phénylpropyle,
(2S)-1-(3,3-diméthyl-2-oxopentanoyl)hexahydro-2-pyridinecarboxylate de 3-(3,4,5-triméthoxyphényl)propyle,
(2S)-1-(3,3-diméthyl-2-oxopentanoyl)hexahydro-2-pyridinecarboxylate de (1R)-2,2-diméthyl-1-phénéthyl-3-butényle,
(2S)-1-(3,3-diméthyl-2-oxopentanoyl)hexahydro-2-pyridinecarboxylate de (1R)-1,3-diphénylpropyle,
(2S)-1-(3,3-diméthyl-2-oxopentanoyl)hexahydro-2-pyridinecarboxylate de (1R)-1-cyclohexyl-3-phénylpropyle,
(2S)-1-(3,3-diméthyl-2-oxopentanoyl)hexahydro-2-pyridinecarboxylate de (1S)-1,3-diphénylpropyle, (2S)-1-(3,3-diméthyl-2-oxopentanoyl)hexahydro-2-pyridinecarboxylate de (1S)-1-cyclohexyl-3-phénylpropyle,
(22aS)-15,15-diméthylperhydropyrido[2,1-c][1,9,4]dioxazacyclononadécine-1,12,16,17-tétraone,
(24aS)-17,17-diméthylperhydropyrido[2,1-c][1,9,4]dioxazacyclohénicosine-1,14,18,19-tétraone,
(3R,4R,23aS)-8-allyl-4,10-diméthyl-3-[2-(3-pyridyl)éthyl]-1, 3, 4, 5, 6, 7, 8, 11, 12, 15, 16, 17, 18, 20, 21, 22, 23, 23a-octadécahydro-14H-pyrido[2,1-c][1,10,4]dioxazacycloïcosine-1,7,14,17,18-pentaone,
(3R,4R,24aS)-8-allyl-4,10-diméthyl-3-[2-(3-pyridyl)éthyl]-1,3,4,5,6,7,8,11,12,14,15,16,17,18,19,21,22,23,24,24a-icosahydropyrido[2,1-c][1,11,4]dioxazacyclohénicosine-1,7,14,18,19-pentaone,
(3R,4R,25aS)-8-allyl-4,10-diméthyl-3-[2-(3-pyridyl)éthyl]-1,3,4,5,6,7,8,11,12,15,16,17,18,19,20,22,23,24,25,25a-icosahydro-14H-pyrido[2,1-c][1,12,4]dioxazacyclodocosine-1,7,14,19,20-pentaone,
(1R)-2-(3,3-diméthyl-2-oxopentanoyl)cyclohexane-1-carboxylate de (1R)-1-(3-benzoylphényl)-3-phénylpropyle,
(1R)-2-(3,3-diméthyl-2-oxopentanoyl)cyclohexane-1-carboxylate de (1R)-1-[3-(diallylcarbamoyl)phényl]-3-phénylpropyle,
1-(2-oxo-3-phénylpropanoyl)-2-pipéridinecarboxylate d'éthyle,
1-pyruvoyl-2-pipéridinecarboxylate d'éthyle, 1-(2-oxo-butanoyl)-2-pipéridinecarboxylate d'éthyle,
1-(3-méthyl-2-oxobutanoyl)-2-pipéridinecarboxylate d'éthyle,
1-(4-méthyl-2-oxopentanoyl)-2-pipéridinecarboxylate d'éthyle,
1-(3,3-diméthyl-2-oxobutanoyl)-2-pipéridinecarboxylate d'éthyle,
1-(3,3-diméthyl-2-oxopentanoyl)-2-pipéridinecarboxylate d'éthyle,
acétate de 4-[2-(éthyloxycarbonyl)pipéridino-2,2-diméthyl-3,4-dioxobutyle,
1-[2-(2-hydroxytétrahydro-2H-2-pyranyl)-2-oxoacétyl]-2-pipéridinecarboxylate d'éthyle,
1-[2-(2-méthoxytétrahydro-2H-2-pyranyl)-2-oxoacétyl]-2-pipéridinecarboxylate d'éthyle,
1-[2-(1-hydroxycyclohexyl)-2-oxoacétyl]-2-pipéridinecarboxylate d'éthyle,
1-[2-(1-méthoxycyclohexyl)-2-oxoérétyl]-2-pipéridinecarboxylate d'éthyle,
1-(2-cyclohexyl-2-oxoacétyl)-2-pipéridinecarboxylate d'éthyle,
1-(2-oxo-2-pipéridinoacétyl)-2-pipéridinecarboxylate d'éthyle,
1-[2-(3,4-dihydro-2H-6-pyranyl)-2-oxoacétyl]-2-pipéridinecarboxylate d'éthyle,
1-(2-oxo-2-phénylacétyl)-2-pipéridinecarboxylate d'éthyle,
1-(4-méthyl-2-oxo-1-thioxopentyl)-2-pipéridinecarboxylate d'éthyle,
1-(2-hydroxy-3,3-diméthylpentanoyl)-2-pipéridinecarboxylate de 3-phénylpropyle,
1-(3,3-diméthylbutanoyl)-2-pipéridinecarboxylate de (1R)-1-phényl-3-(3,4,5-triméthoxyphényl)propyle,
1-(benzylsulfonyl)-2-pipéridinecarboxylate de (1R)-1,3-diphénylpropyle,
1-(bénzylsulfonyl)-2-pipéridinecarboxylate de 3-(3,4,5-triméthoxyphényl)propyle,
acide 1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylique,
1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate de méthyle,
1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate d'isopropyle,
1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate de benzyle,
1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate de 1-phényléthyle,
1-(2-[(2,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate de (Z)-3-phényl-2-propényle,
1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate de 3-(3,4-diméthoxyphényl)propyle,
N2-benzyl-1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate, et
N2-(3-phénylpropyl)-1-(2-[(2R,3R,6S)-6-[(2S,3E,5E,7E,9S,11R)-2,13-diméthoxy-3,9,11-triméthyl-12-oxo-3,5,7-tridécatriényl]-2-hydroxy-3-méthyltétrahydro-2H-2-pyranyl)-2-oxoacétyl)-2-pipéridinecarboxylate ; ou
(v) 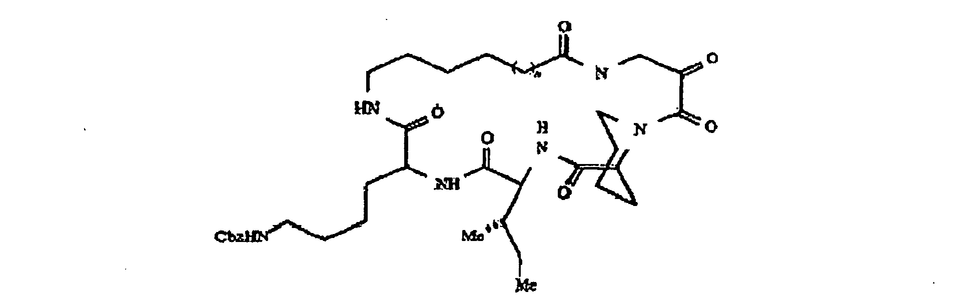 dans laquelle n vaut 1, 2 ou 3 ; 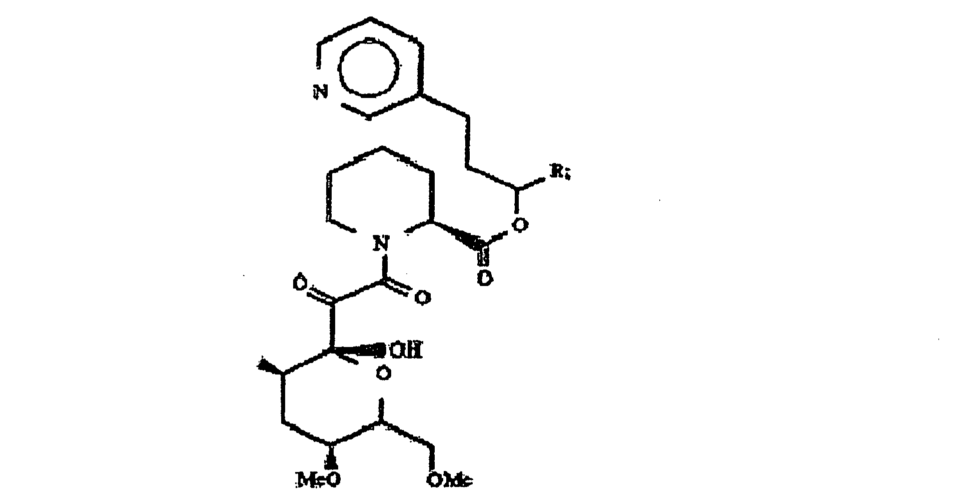 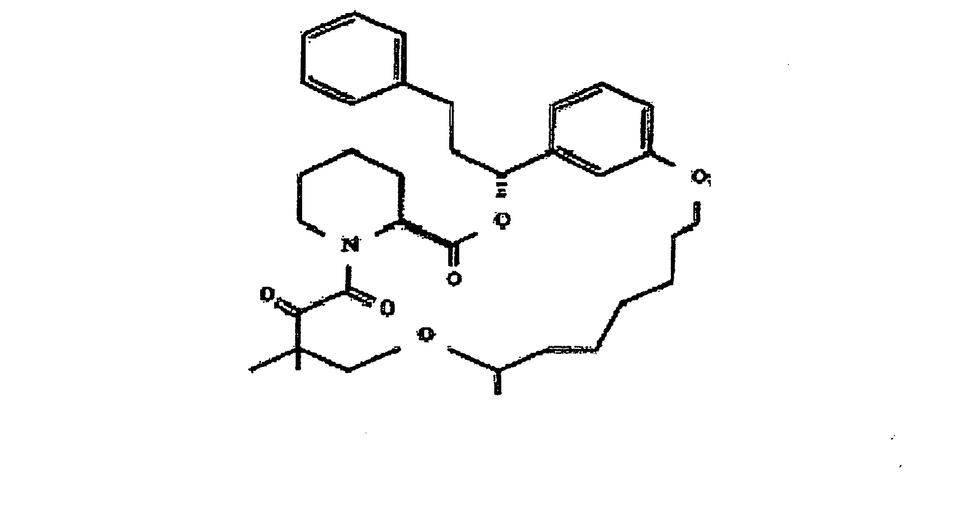 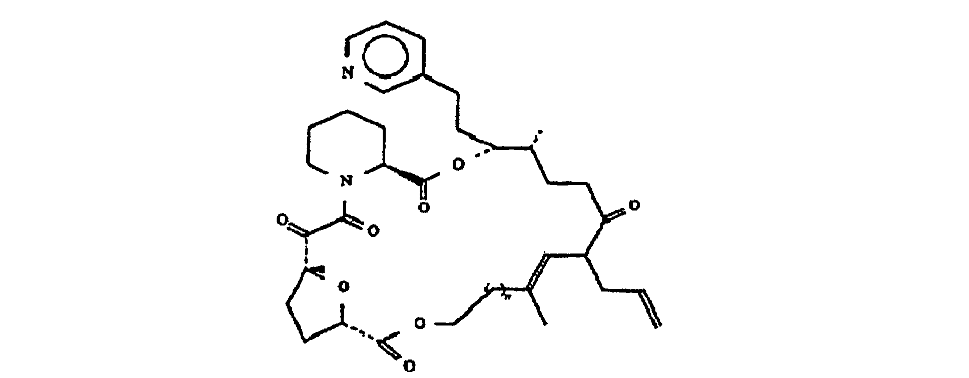 dans laquelle n vaut 1, 2 ou 3, 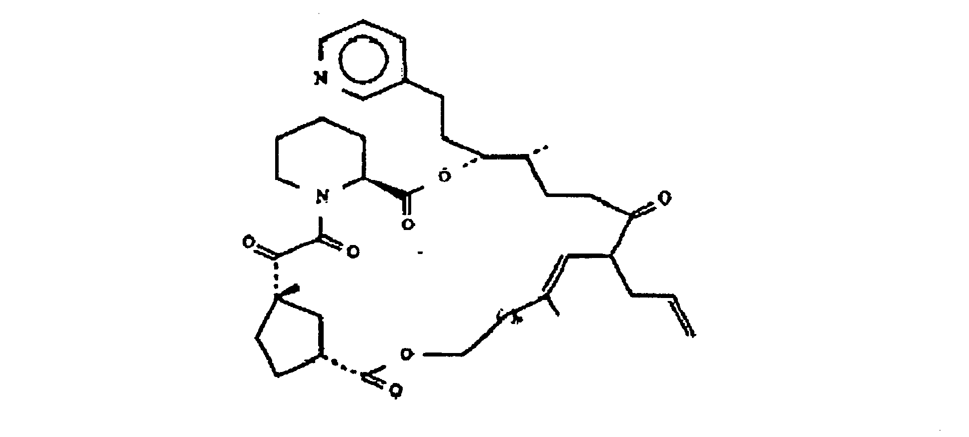 dans laquelle n vaut 1, 2 ou 3, 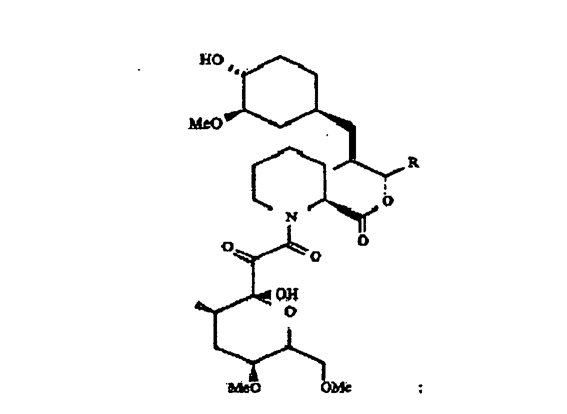 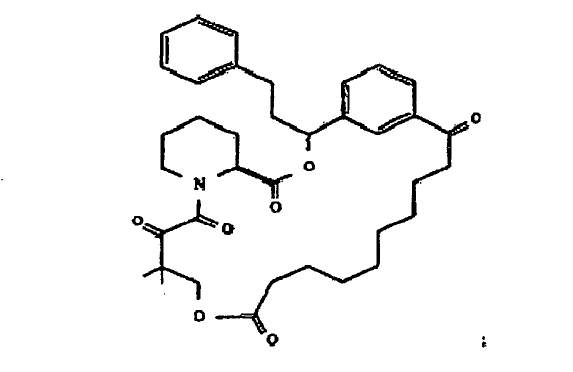 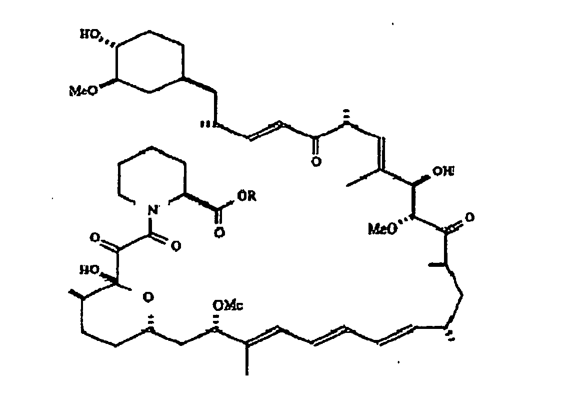 dans laquelle R est méthyle (Me) ou benzyle (Bn), 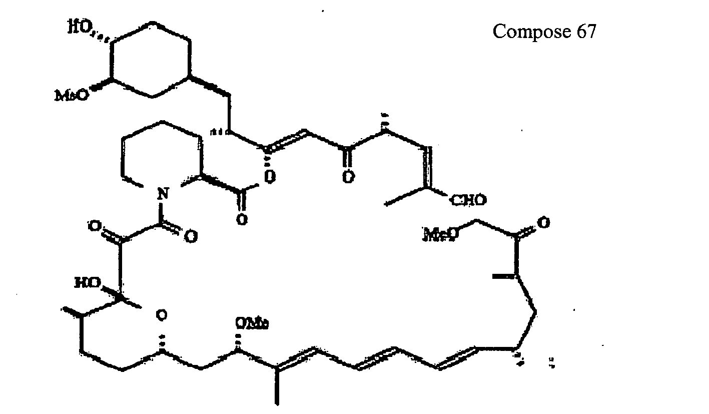 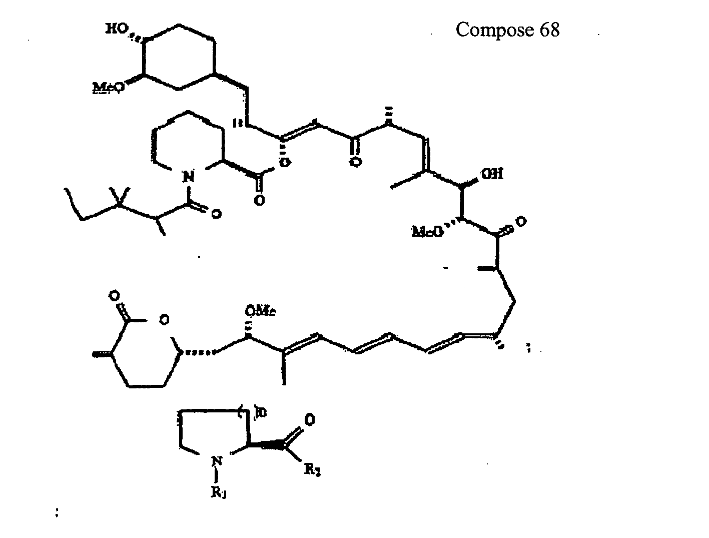 où n = 2
R₁ = 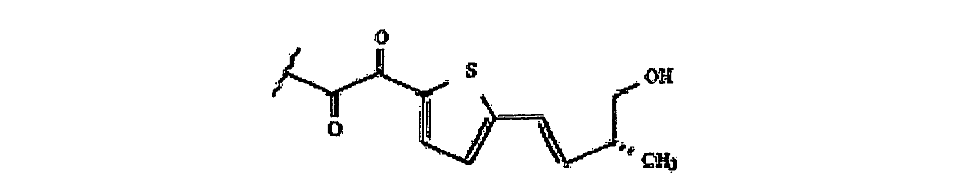 ou 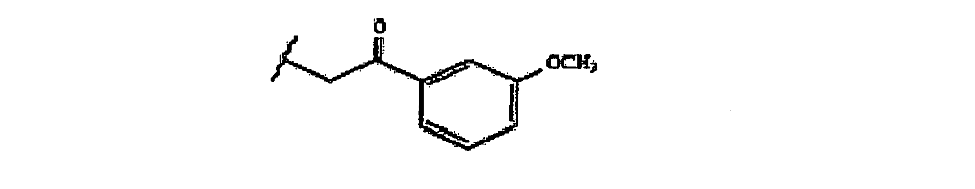 et R₂ = Phe-o-tert-butyle, 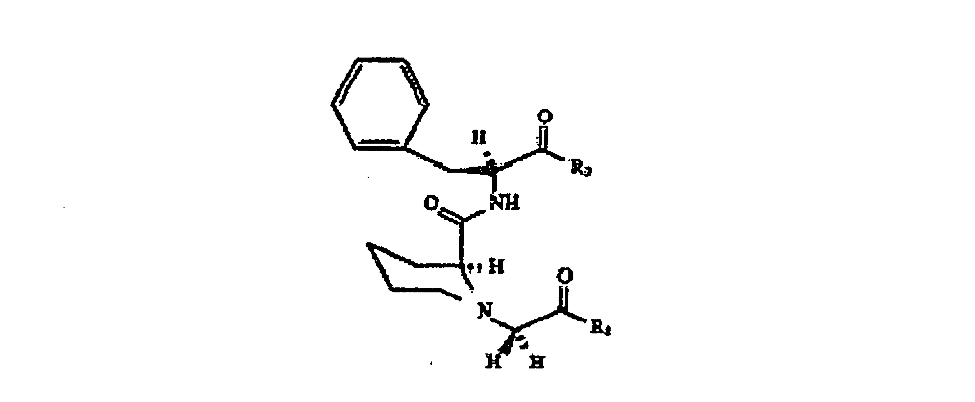 dans laquelle
R₁ = m-OCH₃ Ph, R₃ = Val-o-tert-butyle ;
R₁ = m-OCH₃ Ph, R₃ = Leu-o-tert-butyle ;
R₁ = m-OCH₃ Ph, R₃ = Ileu-o-tert-butyle ;
R₁ = m-OCH₃ Ph, R₃ = hexahydro-Phe-o-tert-butyle ;
R₁ = m-OCH₃ Ph, R₃ = allylalanine-o-tert-butyle ;
R₁ = B-naphtyle, R₃ = Val-o-tert-butyle ; 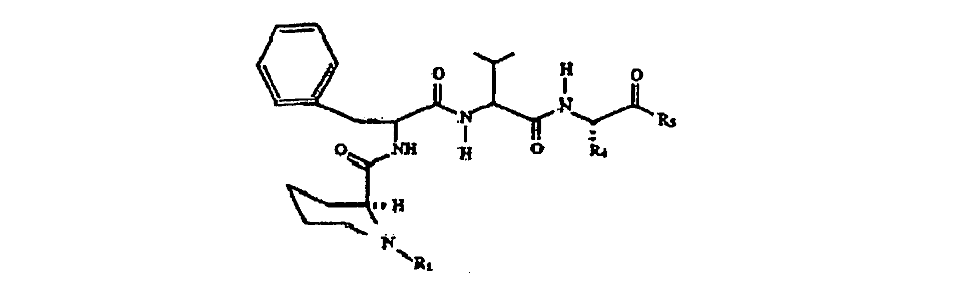 dans laquelle R₁ = CH2(CO)-m-OCH3 Ph
R₄ = CH₂ Ph
R₅ = OCH₃
ou
R₁ = CH₂(CO)-B-naphtyle
R₄ = CH₂ Ph
R₅ = OCH₃ 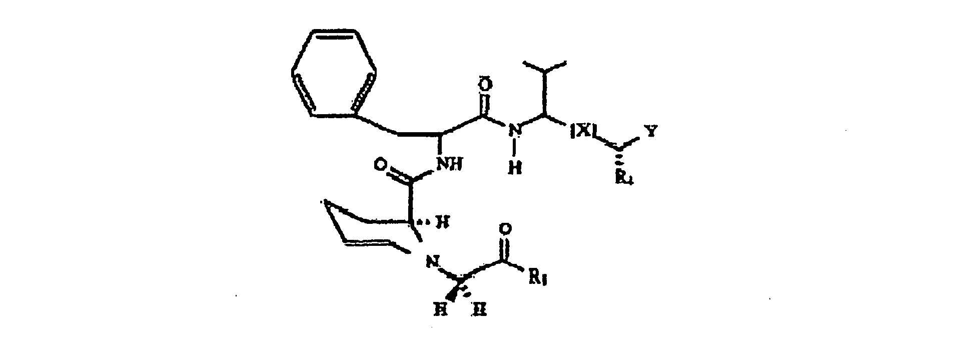 dans laquelle R₁ = m-OCH₃ Ph
X = trans-CH = CH
R₄ = H
Y = OC(o)Ph
R₁ = m-OCH₃ Ph
X = trans-CH = CH
R₄ = H
Y = OC(o)CF₃R₁ = m-OCH₃ Ph
X = trans-CH = CH
R₄ = - -
Y = - -
R₁ = m-OCH₃ Ph
X = trans-CH = CH
R₄ = H
Y = OCH₂ CH = CH₂
R₁ = m-OCH₃ Ph
X = C = O
R₄ = H Y = Ph 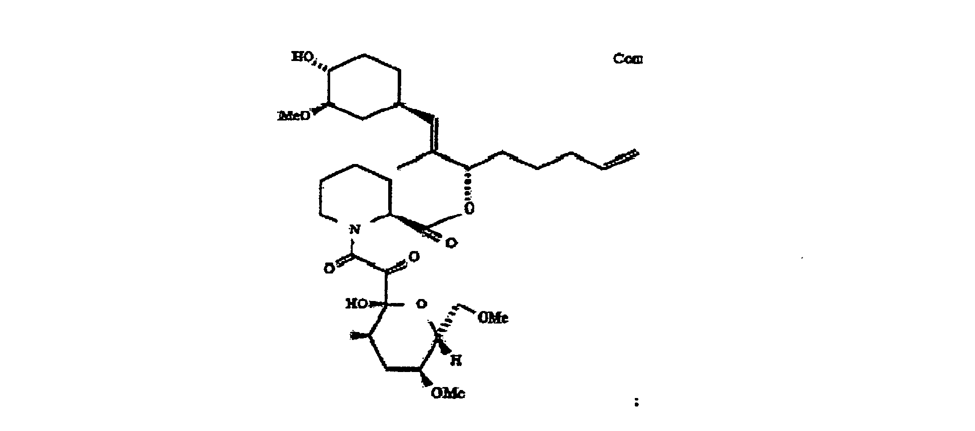 dans laquelle R₁ = H, R₂ = OMe et R₃ = CH₂ OMe ;
R₁ = H, R₂ = H et R₃ = H ; et
R₁ = Me, R₂ = H et R₃ = H,
(g) un dérivé d'amide ou d'ester hétérocyclique, qui est 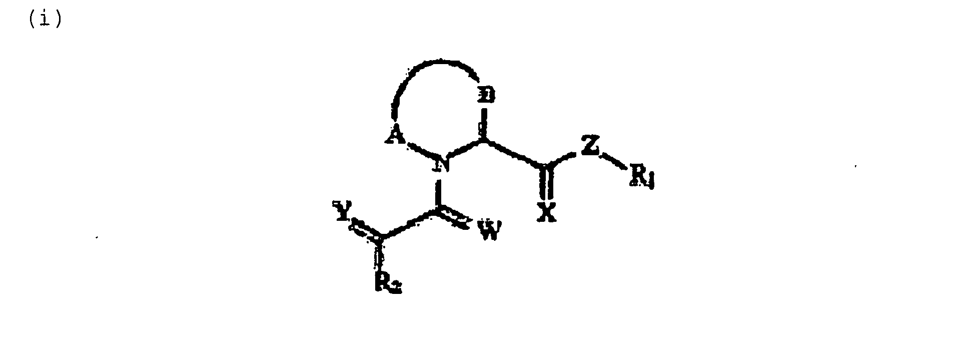 ou un sel pharmaceutiquement acceptable de celui-ci, dans laquelle :
A et B, conjointement avec les atomes d'azote et de carbone auxquels ils sont respectivement liés, forment un cycle hétérocyclique saturé ou insaturé à 5-7 chaînons contenant, en plus de l'atome d'azote, au moins un hétéroatome 0, S, SO, SO₂, NH ou NR₁ dans un état d'oxydation quelconque chimiquement stable ;
X est O ou S ;
Z est 0, NH ou NR₁ ;
W et Y sont indépendamment 0, S, CH₂ ou H₂ ;
R₁ est alkyle ou alcényle en C₁-C₆ à chaîne droite ou ramifiée, qui est substitué en une ou plusieurs positions par (Ar₁)ₙ, (Ar₁)ₙ relié par alkyle ou alcényle en C₁-C₆ à chaîne droite ou ramifiée, cycloalkyle en C₃-C₈, cycloalkyle en C₃-C₈ relié par alkyle ou alcényle en C₁-C₆ à chaîne droite ou ramifiée, Ar₂, ou une combinaison de ceux-ci ;
n vaut 1 ou 2 ;
R₂ est alkyle ou alcényle en C₁-C₉ à chaîne droite ou ramifiée, cycloalkyle en C₃-C₈, cycloalcényle en C₅-C₇ ou Ar₁, où ledit alkyle, alcényle, cycloalkyle ou cycloalcényle est non substitué ou substitué en une ou plusieurs positions par alkyle ou alcényle en C₁-C₄ à chaîne droite ou ramifiée, hydroxyle, ou une combinaison de ceux-ci ; et
Ar₁ et Ar₂ sont indépendamment un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁-C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de 0, N, S, et une combinaison de ceux-ci ; ou 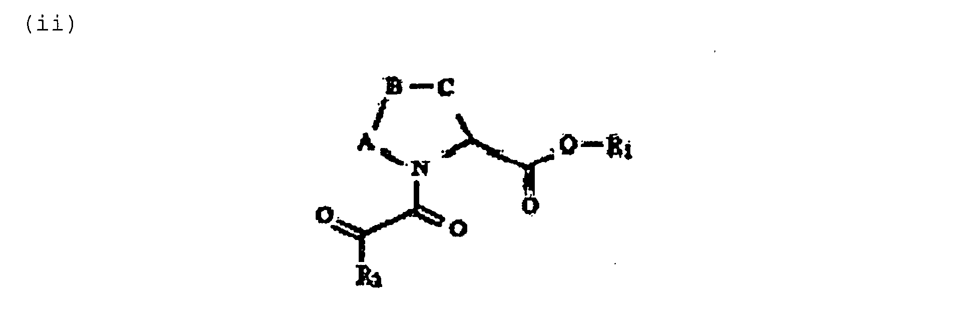 ou un sel pharmaceutiquement acceptable de celui-ci, où : A, B et C sont indépendamment CH₂, O, S, SO, SO₂, NH ou NR₁ ;
R₁ est alkyle ou alcényle en C₁-C₅ à chaîne droite ou ramifiée, qui est substitué en une ou plusieurs positions par (Ar₁)ₙ, (Ar₁)ₙ relié par un alkyle ou alcényle en C₁-C₆ à chaîne droite ou ramifiée, ou une combinaison de ceux-ci ;
n vaut 1 ou 2 ;
R₂ est alkyle ou alcényle en C₁-C₉ à chaîne droite ou ramifiée, cycloalkyle en C₃-C₈, cycloalcényle en C₅-C₇ ou Ar₁ ; et
Ar₁ est un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁₋C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de O, N, S, et une combinaison de ceux-ci ; ou 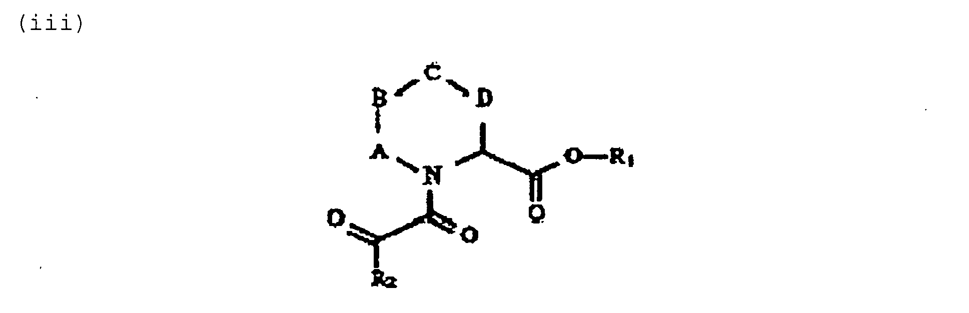 ou un sel pharmaceutiquement acceptable de celui-ci, où : A, B, C et D sont indépendamment CH₂, O, S, SO, SO₂, NH ou NR₁;
R₁ est alkyle ou alcényle en C₁-C₅ à chaîne droite ou ramifiée, qui est substitué en une ou plusieurs positions par (Ar₁)ₙ, (Ar₁)ₙ relié par un alkyle ou alcényle en C₁-C₆ à chaîne droite ou ramifiée, ou une combinaison de ceux-ci ;
n vaut 1 ou 2 ;
R₂ est alkyle ou alcényle en C₁-C₉ à chaîne droite ou ramifiée, cycloalkyle en C₃-C₈, cycloalcényle en C₅-C₇ ou Ar₁ ; et
Ar₁ est un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁₋C₄, alcényloxy en C₁-C₄, phénoxy, benzyloxy, amino, ou une combinaison de ceux-ci ; où les tailles de cycle individuelles sont de 5-6 chaînons ; et où le cycle hétérocyclique contient 1-6 hétéroatomes choisis parmi le groupe constitué de O, N, S, et une combinaison de ceux-ci ; ou
(iv) (2S)-1-(3,3-diméthyl-1,2-dioxopentyl)-2-(4-thiazolidine)carboxylate de 3-phényl-1-propyle ; et
(2S)-1-(3,3-diméthyl-1,2-dioxopentyl)-2-(4-thiazolidine)carboxylate de 3-(3-pyridyl)-1-propyle,
(h) un dérivé de pipéridine, qui est 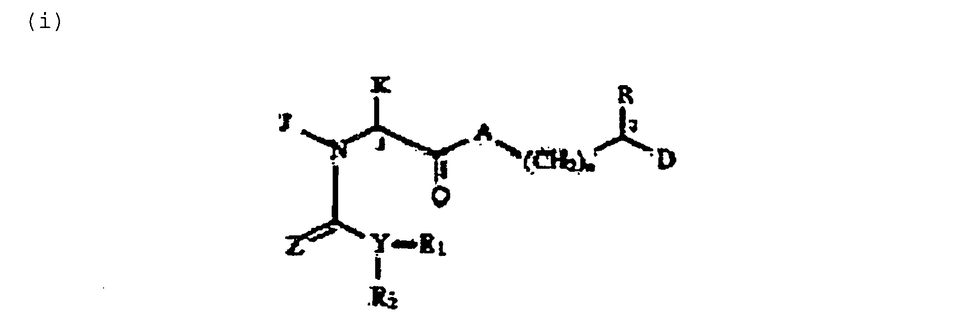 et des dérivés pharmaceutiquement acceptable de celui-ci,
dans laquelle A est CH₂, oxygène, NR₁ ; où R₁, B et D sont indépendamment :
Ar, alkyle en (C1-C6) linéaire ou ramifié, alcényle ou alcynyle en (C2-C6) linéaire ou ramifié, alkyle en (C1-C6) linéaire ou ramifié substitué par cycloalkyle en (C5-C7), alcényle ou alcynyle en (C3-C6) linéaire ou ramifié, alkyle en (C1-C6) linéaire ou ramifié substitué par cycloalcényle en (C5-C7), alcényle ou alcynyle en (C3-C6) linéaire ou ramifié, alkyle en (C1-C6) linéaire ou ramifié substitué par Ar, ou alcényle ou alcynyle en (C3-C6) linéaire ou ramifié substitué par Ar,
où l'un quelconque des groupements CH₂ desdites chaînes alkyle est éventuellement remplacé par un hétéroatome choisi parmi le groupe constitué de 0, S, SO, SO₂ et NR, où R est choisi parmi hydrogène, alkyle en (C1-C4) linéaire ou ramifié, alcényle ou alcynyle en (C3-C4) linéaire ou ramifié, ou alkyle de pontage en (C1-C4), où un pont est formé entre l'azote et un atome de carbone de ladite chaîne contenant un hétéroatome pour former un cycle, et où ledit cycle est éventuellement condensé en un groupement Ar ;
J est choisi parmi hydrogène, alkyle en (C1-C6) linéaire ou ramifié, alcényle en (C3-C6) linéaire ou ramifié, ou --CH₂ Ar ;
K est choisi parmi alkyle en (C1-C4) linéaire ou ramifié,--CH₂ Ar ou cyclohexylméthyle ; ou J et K sont pris ensemble pour former un cycle hétérocyclique à 5-7 chaînons contenant éventuellement un hétéroatome choisi parmi 0, S, SO ou SO₂ ; S
Z est O ou S ;
Y est O ou N, où,
lorsque Y est 0, R₁ est alors une paire célibataire (telle qu'utilisée dans la présente, l'expression « paire célibataire » désigne une paire d'électrons célibataires, telle que la paire d'électrons célibataires présente sur l'oxygène divalent) et R₂ est choisi parmi Ar, alkyle en (C1-C6) linéaire ou ramifié, alcényle ou alcynyle en (C3-C6) linéaire ou ramifié ; et
lorsque Y est N, R₁ et R₂ sont indépendamment choisis parmi Ar, alkyle en (C1-C6) linéaire ou ramifié, alcényle ou alcynyle en (C3-C6) linéaire ou ramifié ; ou R₁ et R₂ sont pris ensemble pour former un cycle hétérocyclique à 5-6 chaînons choisi parmi pyrrolidine, imidazolidine, pyrazolidine, pipéridine ou pipéraziné ;
où Ar est choisi parmi phényle, 1-naphtyle, 2-naphtyle, indényle, azulényle, fluorényle ou anthracényle, 2-furyle, 3-furyle, 2-thiényle, 3-thiényle, 2-pyridyle, 3-pyridyle, 4-pyridyle, pyrrolyle, oxazolyle, thiazolyle, imidazolyle, pyrazolyle, 2-pyrazolinyle, pyrazolidinyle, isoxazolyle, isotriazolyle, 1,2,3-oxadiazolyle, 1,2,3-triazolyle, 1,3,4-thiadiazolyle, pyridazinyle, pyrimidinyle, pyrazinyle, 1,3,5-triazinyle, 1,3,5-trithianyle, indolizinyle, indolyle, isoindolyle, 3H-indolyle, indolinyle, benzo[b]furanyle, benzo[b]thiophényle, 1H-indazolyle, benzimidazolyle, benzthiazolyle, purinyle, 4H-quinolizinyle, quinolinyle, 1,2,3,4-tétrahydroquinoléinyle, isoquinoléinyle, 1,2,3,4-tétrahydroisoquinoléinyle, cinnolinyle, phtalazinyle, quinazolinyle, quinoxalinyle, 1,8-naphtyridinyle, ptéridinyle, carbazolyle, acridinyle, phénazinyle, phénothiazinyle ou phénoxazinyle ;
où Ar contient éventuellement un à trois substituants choisis indépendamment parmi le groupe constitué d'hydrogène, halogène, hydroxyle, nitro, --SO₃ H, trifluorométhyle, trifluorométhoxy, alkyle en (C1-C6) linéaire ou ramifié, alcényle en (C2-C6) linéaire ou ramifié, O-[alkyle en (C1-C6) linéaire ou ramifié], O-[alcényle en (C3-C4) linéaire ou ramifié], O-benzyle, O-phényle, 1,2-méthylènedioxy, --NR₃R₄, carboxyle, N-(alkyle en C1-C5 linéaire ou ramifié ou alcényle en C3-C5 linéaire ou ramifié)carboxamides, N,N-di-(alkyle en C1-C5 linéaire ou ramifié ou alcényle en C3-C5 linéaire ou ramifié)carboxamides, morpholinyle, pipéridinyle, O--Z, CH₂--(CH₂)_{q}--Z, O--(CH₂)_{q}--Z, (CH₂)_{q}--Z--O--Z ou CH=CH--Z ;
où R₃ et R₄ sont choisis indépendamment parmi alkyle en (C1-C6) linéaire ou ramifié, alcényle en (C3-C6) linéaire ou ramifié, hydrogène ou benzyle ; ou où R₃ et R₄ sont pris ensemble pour former un cycle hétérocyclique à 5-6 chaînons ou 8-11 chaînons tel que, par exemple, pipéridinyle, morpholinyle ou pyrrolidinyle ;
où Z est choisi parmi 4-méthoxyphényle, 2-pyridyle, 3-pyridyle, 4-pyridyle, pyrazyle, quinolyle, 3,5-diméthylisoxazoyle, isoxazoyle, 2-méthylthiazoyle, thiazoyle, 2-thiényle, 3-thiényle ou pyrimidyle ;
où q est 0-2 ; et n vaut 0 ou 1 ; ou
(ii)
ester 4-pyridin-3-yl-1-(3-pyridin-3-ylpropyl)butylique d'acide (S)-1-((3,4,5-triméthoxyphényl)méthylcarbamoyl)pipéridine-2-carboxylique ;
ester 4-pyridin-3-yl-1-(3-pyridin-3-ylpropyl)butylique d'acide (S)-1-((3-trifluorométhylphényl)méthylcarbamoyl)pipéridine-2-carboxylique ;
ester 4-pyridin-3-yl-1-(3-pyridin-3-ylpropyl)butylique d'acide (S)-1-((4-tert-butylphényl)méthylcarbamoyl)pipéridine-2-carboxylique ;
ester 4-pyridin-3-yl-1-(3-pyridin-3-ylpropyl)butylique d'acide (S)-1-((4-isopropylphényl)méthylcarbamoyl)pipéridine-2-carboxylique ;
ester 4-pyridin-3-yl-1-(3-pyridin-3-ylpropyl)butylique d'acide (S)-1-(pipéridin-1-carbonyl)pipéridine-2-carboxylique ;
ester 4-pyridin-1-yl-1-(3-pyridin-1-ylpropyl)butylique d'acide (S)-1-((3,4,5-triméthoxyphényl)méthylcarbamoyl)pipéridine-2-carboxylique ;
ester 1-(3,4,5-triméthoxyphényl)ester-2-(4-pyridin-3-yl-1-(3-pyridin-3-ylpropyl)butylique) d'acide (S)-pipéridine-1,2-dicarboxylique ;
ester 1-(2-phényléthyl)-3-phénylpropylique d'acide (S)-1-((3,4,5-triméthoxyphényl)méthylcarbamoyl)pipéridine-2-carboxylique ;
acide 4-(méthyl-(2-(1-phénéthyl-3-phénylpropoxycarbonyl)pipéridine-1-carbonyl)amino)benzènesulfonique ;
ester 1-benzyloxyméthyl-2-benzyloxyéthylique d'acide (S)-pipéridine-2-carboxylique ;
ester 2-benzyloxy-1-(benzyloxyméthyl)éthylique d'acide (S)-1-(méthyl-(4-morpholin-1-ylphényl)carbamoyl)pipéridine-2-carboxylique ;
ester 2-benzyloxy-1-(benzyloxyméthyl)éthylique d'acide (S)-1-(méthyl-(4-pipéridin-1-ylphényl)carbamoyl)pipéridine-2-carboxylique ;
ester 2-(2-benzyloxy-1-(benzyloxyméthyl)éthyl)ester-1-quinoléin-5-ylique d'acide (S)-pipéridine-1,2-dicarboxylique ;
ester 2-(2-benzyloxy-1-(benzyloxyméthyl)éthyl)ester-1-pyridin-3-ylique d'acide (S)-pipéridine-1,2-dicarboxylique ;
(i) un N-oxyde d'un(e) ester, amide, thioester ou cétone hétérocyclique, qui est 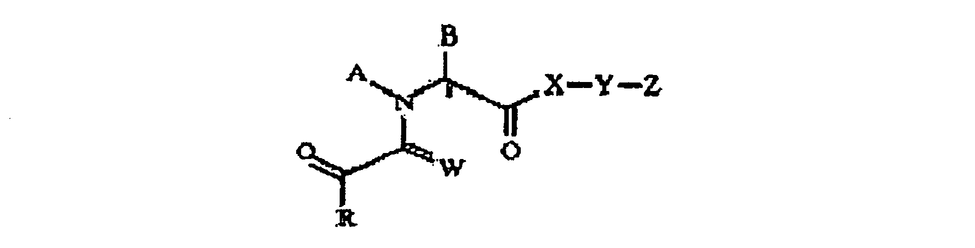 ou un sel pharmaceutiquement acceptable de celui-ci, dans laquelle :
A et B sont pris ensemble, avec les atomes d'azote et de carbone auxquels ils sont respectivement liés, pour former un cycle hétérocyclique saturé ou insaturé à 5-7 chaînons contenant une combinaison quelconque de CH₂, 0, S, SO, SO₂, NH ou NR₁ dans un état d'oxydation quelconque chimiquement stable ;
W est 0, S, CH₂ ou H₂ ;
R est un groupement alkyle ou alcényle en C₁-C₆ à chaîne droite ou ramifiée, éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 1-indolyle, 2-indolyle, 2-furyle, 3-furyle, 2-thiényle, 3-thiényle, 2-pyridyle, 3-pyridyle, 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcényloxy en C₁-C₄, phénoxy, benzyloxy et amino ;
X est 0, NH, NR₁, S, CH, CR₁ ou C(R₁)₂ :
Y est une liaison directe, ou un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy, ou oxygène du carbonyle, ou où l'un quelconque des atomes de carbone dudit groupement alkyle, alcényle, cycloalkyle, cycloalcényle ou Ar est éventuellement remplacé par O, NH, NR₂, S, SO ou SO₂, où R₂ est choisi parmi le groupe constitué d'hydrogène, alkyle en C₁-C₄ à chaîne droite ou ramifiée, alcényle ou alcynyle en C₃-C₈ à chaîne droite ou ramifiée, et alkyle de pontage en C₁-C₄, où un pont est formé entre l'azote et un atome de carbone de ladite chaîne alkyle ou alcényle contenant ledit hétéroatome pour former un cycle, où ledit cycle est éventuellement condensé en un groupement Ar ; et
Z est une alkylamine aromatique ou tertiaire oxydée en un N-oxyde correspondant, où l'amine aromatique est Ar oxydé en un N-oxyde correspondant où Ar est un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁-C₄, alcényloxy en C₁-C₄, phénoxy, benzyloxy, amino, ou une combinaison de ceux-ci ;
où les tailles de cycle individuelles sont de 5-6 chaînons ; et où le cycle hétérocyclique contient 1-6 hétéroatomes choisis parmi le groupe constitué de 0, N, S, et une combinaison de ceux-ci, où au moins l'un des hétéroatomes est N, et où l'alkylamine est oxydée en un
N-oxyde correspondant où alkyle est un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy, ou oxygène du carbonyle, ou où l'un quelconque des atomes de carbone dudit groupement alkyle, alcényle, cycloalkyle, cycloalcényle ou Ar est éventuellement remplacé par 0, NH, NR₁, S, SO ou SO₂; et, R₁ est hydrogène, alkyle en C₁-C₄ à chaîne droite ou ramifiée, alcényle ou alcynyle en C₃-C₄ à chaîne droite ou ramifiée, ou R₁ est Y--Z, tel que défini ci-dessus ; ou
(ii) 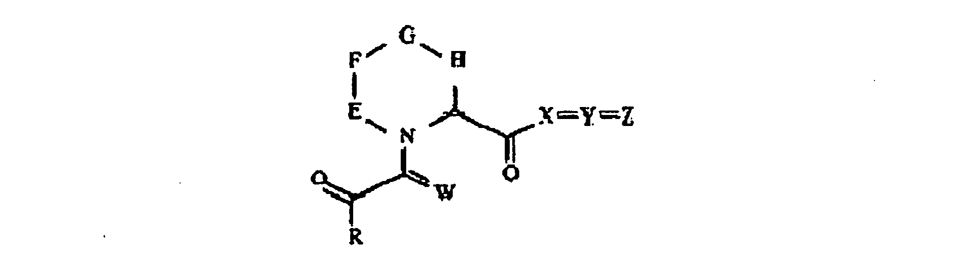 ou un sel pharmaceutiquement acceptable de celui-ci, dans laquelle : E, F, G et H sont indépendamment CH₂, 0, S, SO,
SO₂, NH ou NR₁ ;
W est O, S, CH₂ ou H₂ ;
R est un groupement alkyle ou alcényle en C₁-C₆ à chaîne droite ou ramifiée, éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 1-indolyle, 2-indolyle, 2-furyle, 3-furyle, 2-thiényle, 3-thiényle, 2-pyridyle, 3-pyridyle, 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcényloxy en C₁-C₄, phénoxy, benzyloxy et amino ;
X est O, NH, NR₁, S, CH, CR₁ ou C(R₁)₂ ;
Y est une liaison directe, ou un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy,
ou oxygène du carbonyle, ou où l'un quelconque des atomes de carbone dudit groupement alkyle, alcényle, cycloalkyle, cycloalcényle ou Ar est éventuellement remplacé par 0, NH, NR₂, S, SO ou SO₂, où R₂ est choisi parmi le groupe constitué d'hydrogène, alkyle en C₁-C₄ à chaîne droite ou ramifiée, alcényle ou alcynyle en C₃-C₄ à chaîne droite ou ramifiée, et alkyle de pontage en C₁-C₄, où un pont est formé entre l'azote et un atome de carbone de ladite chaîne alkyle ou alcényle contenant ledit hétéroatome pour former un cycle, où ledit cycle est éventuellement condensé en un groupement Ar ; et
Z est une alkylamine aromatique ou tertiaire oxydée en un N-oxyde correspondant, où l'amine aromatique est Ar oxydé en un N-oxyde correspondant où Ar est un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁-C₄, alcényloxy en C₁-C₄, phénoxy, benzyloxy, amino, ou une combinaison de ceux-ci ;
où les tailles de cycle individuelles sont de 5-6 chaînons ; et où le cycle hétérocyclique contient 1-6 hétéroatomes choisis parmi le groupe constitué de O, N, S, et une combinaison de ceux-ci, où au moins l'un des hétéroatomes est N, et où l'alkylamine est oxydée en un N-oxyde correspondant où alkyle est un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy, ou oxygène du carbonyle, ou où l'un quelconque des atomes de carbone dudit groupement alkyle, alcényle, cycloalkyle, cycloalcényle ou Ar est éventuellement remplacé par O, NH, NR₁, S, SO ou SO₂; et, R₁ est hydrogène, alkyle en C₁-C₄ à chaîne droite ou ramifiée, alcényle ou alcynyle en C₃-C₄ à chaîne droite ou ramifiée, ou R₁ est Y--Z, tel que défini ci-dessus ; ou 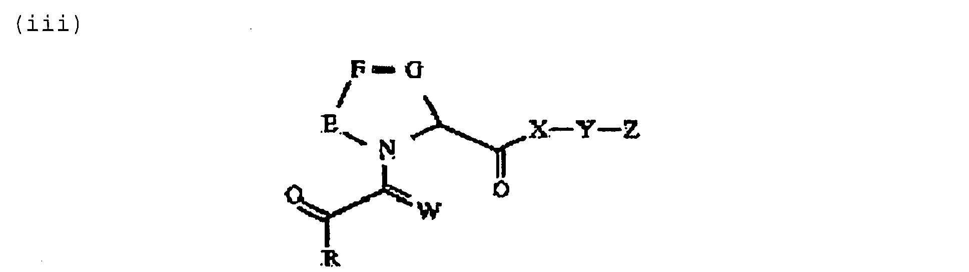 ou un sel pharmaceutiquement acceptable de celui-ci, dans laquelle : E, F et G sont indépendamment CH₂, 0, S, SO, SO₂,
NH ou NR₁ ;
W est O, S, CH₂ ou H₂ ;
R est un groupement alkyle ou alcényle en C₁-C₆ à chaîne droite ou ramifiée, éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 1-indolyle, 2-indolyle, 2-furyle, 3-furyle, 2-thiényle, 3-thiényle, 2-pyridyle, 3-pyridyle, 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcényloxy en C₁-C₄, phénoxy, benzyloxy et amino ;
X est O, NH, NR₁, S, CH, CR₁ ou C (R₁) ₂ ;
Y est une liaison directe, ou un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy, ou oxygène du carbonyle, ou où l'un quelconque des atomes de carbone dudit groupement alkyle, alcényle, cycloalkyle, cycloalcényle ou Ar est éventuellement remplacé par 0, NH, NR₂, S, SO ou SO₂, où R₂ est choisi parmi le groupe constitué d'hydrogène, alkyle en C₁-C₄ à chaîne droite ou ramifiée, alcényle ou alcynyle en C₃-C₄ à chaîne droite ou ramifiée, et alkyle de pontage en C₁-C₄, où un pont est formé entre l'azote et un atome de carbone de ladite chaîne alkyle ou alcényle contenant ledit hétéroatome pour former un cycle, où ledit cycle est éventuellement condensé en un groupement Ar ; et
Z est une alkylamine aromatique ou tertiaire oxydée en un N-oxyde correspondant, où l'amine aromatique est Ar oxydé en un N-oxyde correspondant où Ar est un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne droite ou ramifiée, alcoxy en C₁-C₄, alcényloxy en C₁-C₄, phénoxy, benzyloxy, amino, ou une combinaison de ceux-ci ;
où les tailles de cycle individuelles sont de 5-6 chaînons ; et où le cycle hétérocyclique contient 1-6 hétéroatomes choisis parmi le groupe constitué de 0, N, S, et une combinaison de ceux-ci, où au moins l'un des hétéroatomes est N, et où l'alkylamine est oxydée en un N-oxyde correspondant où alkyle est un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C-₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy, ou oxygène du carbonyle, ou où l'un quelconque des atomes de carbone dudit groupement alkyle, alcényle, cycloalkyle, cycloalcényle ou Ar est éventuellement remplacé par O, NH, NR₁, S, SO ou SO₂; et, R₁ est hydrogène, alkyle en C₁-C₄ à chaîne droite ou ramifiée, alcényle ou alcynyle en C₃-C₄ à chaîne droite ou ramifiée, ou R₁ est Y--Z, tel que défini ci-dessus ; ou 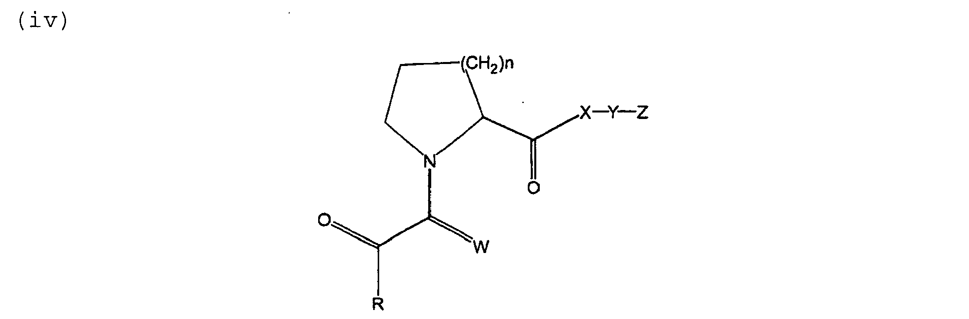 ou un sel pharmaceutiquement acceptable de celui-ci, dans laquelle : n vaut 1, 2 ou 3 formant un cycle hétérocyclique à 5-7 chaînons ;
W est 0, S, CH₂ ou H₂ ;
R est un groupement alkyle ou alcényle en C₁-C₆ à chaîne droite ou ramifiée, éventuellement substitué par cycloalkyle en C₃-C₈, cycloalkyle en C₃ ou C₅, cycloalcényle en C₅-C₇ ou Ar₁, où lesdits groupements alkyle, alcényle, cycloalkyle ou cycloalcényle peuvent être éventuellement substitués par alkyle en C₁-C₄, alcényle en C₁-C₄ ou hydroxy, et où Ar₁ est choisi parmi le groupe constitué de 1-naphtyle, 2-naphtyle, 1-indolyle, 2-indolyle, 2-furyle, 3-furyle, 2-thiényle, 3-thiényle, 2-pyridyle, 3-pyridyle, 4-pyridyle ou phényle, ayant un à trois substituants choisis indépendamment parmi le groupe constitué d'hydrogène, halogéno, hydroxyle, nitro, trifluorométhyle, alkyle ou alcényle en C₁-C₆ linéaire ou ramifié, alcényloxy en C₁-C₄, phénoxy, benzyloxy et amino ;
X est 0, NH, NR₁, S, CH, CR₁ ou C(R₁)₂ ;
Y est une liaison directe, ou un alkyle ou alcényle en C₁-C₆ à chaîne droite ou ramifiée, éventuellement substitué en une ou plusieurs positions par alkyle ou alcényle en C₁-C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy, ou oxygène du carbonyle, ou où l'un quelconque des atomes de carbone dudit groupement alkyle, alcényle, cycloalkyle, cycloalcényle ou Ar est éventuellement remplacé par 0, NH, NR₂, S, SO ou SO₂, où R₂ est choisi parmi le groupe constitué d'hydrogène, alkyle en C₁-C₄ à chaîne droite ou ramifiée, alcényle ou alcynyle en C₃-C₄ à chaîne droite ou ramifiée, et alkyle de pontage en C₁-C₄, où un pont est formé entre l'azote et un atome de carbone de ladite chaîne alkyle ou alcényle contenant ledit hétéroatome pour former un cycle, où ledit cycle est éventuellement condensé en un groupement Ar ; et
Z est une alkylamine aromatique ou tertiaire oxydée en un N-oxyde correspondant, où l'amine aromatique est Ar oxydé en un N-oxyde correspondant où Ar est un cycle carbocyclique ou hétérocyclique monocyclique, bicyclique ou tricyclique, où le cycle est non substitué ou substitué en une à trois positions par halogéno, hydroxyle, nitro, trifluorométhyle, alkyle ou alcényle en C₁-C₆ à chaîne linéaire ou ramifiée, alcoxy en C₁-C₄, alcényloxy en C₁-C₄, phénoxy, benzyloxy, amino, ou une combinaison de ceux-ci ;
où les tailles de cycle individuelles sont de 5-6 chaînons ; où le cycle hétérocyclique contient 1-6 hétéroatomes choisis parmi le groupe constitué de 0, N, S, et une combinaison de ceux-ci, où au moins l'un des hétéroatomes est N, et où l'alkylamine est oxydée en un N-oxyde correspondant où alkyle est un alkyle ou alcényle en C₁-C₆ à chaîne droite ou ramifiée, éventuellement substitué en une ou plusieurs positions par alkyle ou alcényle en C₁₋C₆ à chaîne droite ou ramifiée, ou cycloalkyle en C₃-C₈, ou cycloalcényle en C₅-C₇, ou hydroxyle, ou oxygène du carbonyle, ou par Ar, où ledit groupement alkyle, alcényle, cycloalkyle, cycloalcényle ou Ar est éventuellement substitué par alkyle en C₁-C₄, alcényle en C₁-C₄ ou hydroxy,
ou oxygène du carbonyle, ou où l'un quelconque des atomes de carbone dudit groupement alkyle, alcényle, cycloalkyle, cycloalcényle ou Ar est éventuellement remplacé par 0, NH, NR₁, S, SO ou SO₂ ; et,
R₁ est hydrogène, alkyle en C₁-C₄ à chaîne droite ou ramifiée, alcényle ou alcynyle en C₃-C₄ à chaîne droite ou ramifiée, ou R₁ est Y--Z, tel que défini ci-dessus ; ou
(v)
(2S)-1-(3,3-diméthyl-1,2-dioxopentyl)-2-pyrrolidinecarboxylate de 3-(2-pyridyl)-1-propyle, N-oxyde ;
(2S)-1-(3,3-diméthyl-1,2-dioxopentyl)-2-pyrrolidinecarboxylate de 3-(3-pyridyl)-1-propyle, N-oxyde ;
(2S)-1-(3,3-diméthyl-1,2-dioxopentyl)-2- - pyrrolidinecarboxylate de 3-(4-pyridyl)-1-propyle, N-oxyde ;
(2S)-1-(1,1-diméthyl-1,2-dioxopentyl)-2-pyrrolidinecarboxylate de 3-(2-quinolyl)-1-propyle, N-oxyde ;
(2S)-1-(1,1-diméthyl-1,2-dioxopentyl)-2-pyrrolidinecarboxylate de 3-(3-quinolyl)-1-propyle, N-oxyde ;
(2S)-1-(1,1-diméthyl-1,2-dioxopentyl)-2-pyrrolidinecarboxylate de 3-(4-quinolyl)-1-propyle, N-oxyde ; ou
(j) un dérivé de cycloheximide, qui est la 4-[2-(3,5-diméthylcyclohexyl-2-oxime)-2-hydroxyéthyl]-2,6-pipéridinedione ;
la 4-[2-(3,5-diméthyl-2-oxocyclohexyl)-2-acétoxyéthyl]-2,6-pipéridinedione ;
le 4-[2-(3,5-diméthyl-2-oxocyclohexyl)-2-(oxycarbonyl-1-adamantylamino)éthyl]-2,6-pipéridinedione-1-(éthyléthanoate) ;
la 4-[2-(3,5-diméthyl-2-oxocyclohexyl)-2-hydroxyéthyl]-1-méthyl-2,6-pipéridinedione ;
la 4-[2-(3,5-diméthyl-2-oxocyclohexyl)-2-hydroxyéthyl]-1-benzyl-2,6-pipéridinedione ;
la 4-[2-(3,5-diméthyl-2-oxocyclohexyl)-2-hydroxyéthyl]-1-(4-cyanobenzyl)-2,6-pipéridinedione ;
le 4-[2-(3,5-diméthyl-2-oxocyclohexyl)-2-hydroxyéthyl]-2,6-pipéridinedione-1-(amide d'acide acétique) ; le 4-[2-(3,5-diméthyl-2-oxocyclohexyl)-2-hydroxyéthyl]-2,6-pipéridinedione-1-(4-éthylbutanoate) ; ou
le 4-[2-(3,5-diméthyl-2-oxocyclohexyl)-2-hydroxyéthyl]-2,6-pipéridinedione-1-(éthyléthanoate) ; ou où
(k) le ligand de neurophiline est le (S)-N-benzyl-3-(4-chlorophényl)-2-(méthyl-(2-oxo-2-(3,4,5-triméthoxyphényl)acétyl)amino)-N-(3-pyridinyl-4-yl)-4-yl)-1-(2-pyrodinyl-4-yl)éthyl)propyl)propionamide ou l'ester 3-(3-pyridinyl)propylique de 1-(3,3-diméthyl-1,2-dioxopentyl)-(L)-proline.

2. Utilisation d'un ligand de neurophiline qui ne présente pas une activité immunosuppressive, pour la fabrication d'un médicament destiné à abaisser l'IOP chez un mammifère souffrant d'une PIO élevée, dans laquelle le ligand de neurophiline est un composé selon la revendication 1.

3. Utilisation d'un ligand de neurophiline qui ne présente pas une activité immunosuppressive, pour la fabrication d'un médicament destiné à la prévention de la perte de champ visuel associée à un glaucome, dans laquelle le ligand de neurophiline est un composé selon la revendication 1.

4. Utilisation d'un ligand de neurophiline qui ne r présente pas une activité immunosuppressive, pour la fabrication d'un médicament destiné à abaisser la PIO et à fournir une neuroprotection chez un mammifère souffrant d'une PIO élevée, dans laquelle le ligand de neurophiline est un composé selon la revendication 1.
